# EUROPEAN PATENT APPLICATION

(11) **EP 2 939 668 A1**
(43) Date of publication of application: **04.11.2015**
(21) Application number: 13869345.2
(22) Date of filing: 26.12.2013
(51) Int. Cl.: A61K 31/015, A61K 31/122, A61P 1/14, A61P 13/02, A61P 31/16, A61P 35/00, A61P 43/00

(54) **PDK4 INHIBITOR AND USE THEREOF**

(30) Priority: 26.12.2012 US 201261745819 P
(71) Applicant: The Kitasato Institute, Tokyo 108-8641 (JP); Tokushima University, Tokushima 770-8501 (JP); Nobelpharma Co., Ltd., Chuo-ku Tokyo 103-0024 (JP)
(72) Inventor: OMURA, Satoshi, Tokyo 108-8641 (JP); NAKANO, Hirofumi, Tokyo 108-8641 (JP); YAMAJI, Kenzaburo, Tokyo 108-8641 (JP); YAMAMOTO, Tsuyoshi, Tokyo 108-8641 (JP); KIDO, Hiroshi, Tokushima-shi Tokushima 770-8503 (JP); YAMANE, Kazuhiko, Tokushima-shi Tokushima 770-8503 (JP); SUNAZUKA, Toshiaki, Tokyo 108-8641 (JP); HIROSE, Tomoyasu, Tokyo 108-8641 (JP)
(74) Representative: Duckworth, Timothy John
(86) International application number: PCT/JP2013/007649
(87) International publication number: WO 2014/103321

(57) **Abstract**

The present invention to provide a novel pyruvate dehydrogenase kinase inhibitors. A pyruvate dehydrogenase kinase inhibitor comprising a compound represented by the general formula (I) as an active ingredient (wherein, ring A represents a 6-membered aromatic hydrocarbon ring optionally substituted with 2-4 substituents,
R¹ and R⁴, which are the same or different, represent a hydrogen atom, a hydroxyl group, an optionally substituted C1-6 alkyl group, R² and R³, which are the same or different, represent a hydrogen atom, a carboxyl group, an optionally substituted C1-6 alkyl group, an optionally substituted C6-10 aryl group, or a group represented by -C (=R⁹)-R¹⁰), a pharmaceutical composition for treatment or prophylaxis of diseases or disorders that pyruvate dehydrogenase kinase relates to its development or aggravation, and a cosmetic composition, and the like.

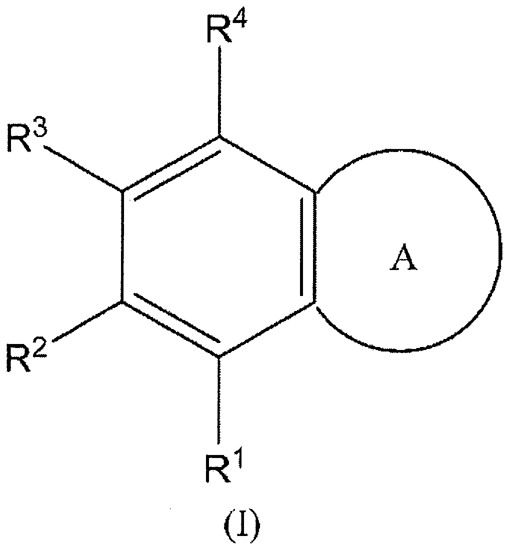

## Description

### CROSS-REFERENCE

This application claims priority of US provisional patent application 61/745,819 filed with the USPTO on December 26, 2012. The content of the US provisional application 61/745,819, which is claimed its priority by the present application, is herein incorporated by reference in its entirety. All the documents referred herein are incorporated by reference in its entirety.

### TECHNICAL FIELD

The present invention, which includes novel PDK4 inhibitors as an active ingredient, provides treatment or prophylactic agents for influenza aggravation by improving mitochondria function, improvement agents for anorexia or treatment agents for diseases such as cancer or diabetes, and cosmetics by improving metabolism.

### BACKGROUND ART

If a healthy adult is infected with an influenza virus, he will recover without aggravation and obtain immunity to the virus. In case elder people or children, it sometimes causes multiple organ failure (MOF: multiple organ failure) or influenza-associated encephalopathy (IAE: influenza-associated encephalopathy) and it is not uncommon to progress to death. Recently, it often happens that anti-virus drugs such as Neuraminidase inhibitors developed in the late 1990s are administered to patients infected with influenza virus. In a published report, it is confirmed from a large scale analysis of the clinical data in 2012 that an initial symptom can be improved (the effect that influenza symptom is improved one day earlier) by administration of Oseltamivir (Tamiflu) or Zanamivir (*Relenza*), however it is not confirmed that an effect to prevent aggravation after infection (Non-Patent Document 1).

Pyruvate dehydrogenase (hereinafter "PDH") localized in mitochondria is an important enzyme to control carbohydrate metabolism and is inactivated by phosphorylation by PDK. Human and mice have four kinds of PDK isozymes (PDK 1-4). It is known that PDK4 relates to development and aggravation of diabetes and cancer (see Non-Patent Documents 2 and 3). PDH reduction occurs due to PDK over expression in cancer and diabetes, so PDK inhibitors have received attention as target molecule of a drug for cancer and diabetes and researches have been conducted.

It has not been discovered, however, that a compound may inhibit PDK4 in 100µM or lower of IC50. For example, AZD7545, Compound K, and Novartis 3r etc. inhibit PDK isozymes 1, 2 and 3 in sub µM order of IC50, on the contrary it has been reported that PDK4 promotes the activity. Different from PDK 1-3, PDK4 exists in semi-activated condition. This might be one reason for difficulty of PDK4 inhibitors development. Dichloro acetic acid reported as PDK4 inhibitors has a weak inhibitory activity and has a serious side effect such as neurotoxicity, thus it could not be used as a medicament (see Non-patent literature 4).

### THE FIELD OF PRIOR ART

### NON-PATENT DOCUMENTS

Non-Patent Document 1: Published Online: 18 JAN 2012 DOI: 10.1002 / 14651858. CD008965. pub3
Non-Patent Document 2: Int. J. Cancer 2011: 128: 1001-1008
Non-Patent Document 3: Biochem. J. (2009) 423: 243-252
Non-Patent Document 4: J. Biol Chem. (2008) 283: 25305-25315

### SUMMARY OF THE INVENTION

The inventors, Kido et al. have analyzed an influenza aggravation developing mechanism, and reported that; in a patient complicating IAE or MOF, peripheral blood adenosine triphosphate (hereinafter "ATP") level is low due to virus infection, and there is a thermally sensitive gene polymorphism in mitochondria fatty acid metabolizing enzyme (carnitine palmitoyl transferase 2: CPT2) (Mol Cell Biochem (2007) 299: 85-92; Hum Mutat (2008) 29: 718-27) Further, as a result of an exhaustive analysis of energy producing system gene expression level after infecting 3 weeks old mice with influenza, the inventors found that PDK4 gene expression induction occurred with cytokine production increase and fever after influenza virus infection. Based on these studies, it is presumed that when influenza-infected patients get aggravated, systemic ATP exhaustion occurs due to an acute reduction in mitochondria function activity, and PDK4 inhibitors may prevent the acute aggravation.

The inventors, Nakano and Ohmura et al. have found protein phosphorylation enzyme inhibitor (Protein Kinase: PK), staurosporine and its related compounds since 1980's (J. Antibiotics (2009) 62: 17-26). The inventors performed a new PDK 4 research based on the presumption of Kido et al. to provide a new drug to prevent aggravation of influenza-infected patients. PDK is Ser/Thr kinase, but it is not inhibited totally by staurosporine known as a strong pan kinase inhibitor. As a result of PDK4 ATP binding site structure analysis, it became clear that staurosporine could not enter PDK4 ATP binding site. The compounds of the present invention was found as a result of the research compounds inhibiting PDK4 in nM order focusing on natural products having smaller molecules than staurosporine. It has been confirmed that the compounds of the present invention have an activity to prevent anorexia, weight loss and death from the influenza-infected mice test, then the present invention was completed.

As mentioned above, when influenza-infected patients get aggravated, PDK4 gene expression is induced, ATP level in peripheral blood by virus infection becomes low, and thermal sensitive gene polymorphism exits in mitochondria fatty acid metabolizing enzyme (CPT2). Thus PDK4 inhibitor of the present invention may probably be effective for treating a disease with a mutation in CPT or mitochondria ATP synthase family.

Further, in addition to the improvement of aggravation of influenza-infected patients and anorexia, it is known, as mentioned above, that PDK4 relates to development and aggravation of diabetes and cancer, thus the PDK4 inhibitor of the present invention may probably be effective for treating these diseases.

Thus, the present invention is to provide treatment or prophylaxis drugs for influenza aggravation. The present invention, in the other aspect, is to provide novel PDK4 inhibitors. Furthermore, the present invention, which includes novel PDK4 inhibitors as an active ingredient, is to provide treatment agents for mitochondrial function and anorexia improvement or treating diseases such as cancer or diabetes, and cosmetics by improving metabolism.

Accordingly, the present invention relates to the following:
(1) A pyruvate dehydrogenase kinase inhibitor comprising a compound represented by the following general formula (I) or ester derivatives thereof, or pharmacologically acceptable salts thereof as an active ingredient, 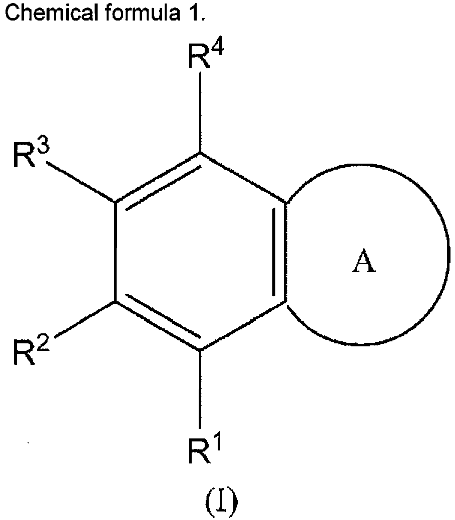 [wherein ring A represents a 6-membered aromatic hydrocarbon ring optionally substituted with 2-4 substituents,
   wherein the substituents of the ring A, which are the same or different, represent a group selected from a hydroxyl group, an oxo group, an optionally substituted amino group, a halogen atom, an optionally substituted C1-6 alkyl group, an optionally substituted C2-40 alkenyl group, an optionally substituted C1-6 alkoxy group, an optionally substituted C2-6 alkenyloxy group, an optionally substituted C1-6 alkoxycarbonyl group, and an optionally substituted C2-7 alkanoyloxy group, or two substituents of the ring A may be bonded to form an optionally substituted dihydropyran (the dihydropyran may be condensed with tetrahydrofuran optionally substituted with an oxo group),
   R¹ and R⁴, which are the same or different, represent a hydrogen atom, a hydroxyl group, an optionally substituted C1-6 alkyl group, an optionally substituted C2-6 alkenyl group, an optionally substituted C6-10 aryl group, an optionally substituted C1-6 alkoxy group, or an optionally substituted C2-7 alkanoyloxy group,
   R² and R³, which are the same or different, represent a hydrogen atom, a carboxyl group, an optionally substituted C1-6 alkyl group, an optionally substituted C6-10 aryl group, or a group represented by -C (=R⁹)-R¹⁰,
   wherein R⁹ represents an oxygen atom, a sulfur atom, a = N-R¹¹ group (wherein R¹¹ represents a hydroxyl group, an optionally substituted C1-6 alkyl group, an optionally substituted C6-10 aryl group, an optionally substituted C1-6 alkoxy group, an optionally substituted C2-6 alkenyloxy group, an optionally substituted C6-10 aryloxy group), or a = CH-R¹² group (wherein R¹² represents a formyl group, a carboxyl group, an optionally substituted C1-6 alkyl group, an optionally substituted C6-10 aryl group, C1-6 an optionally substituted alkoxycarbonyl group, an aminocarbonyl group, or an optionally substituted C1-6 alkylaminocarbonyl group),
   R¹⁰ represents a hydrogen atom, an amino group, an optionally substituted C1-6 alkyl group, an optionally substituted C6-10 aryl group, an optionally substituted C1-6 alkoxy group, an optionally substituted C6-C10 aryloxy group, an optionally substituted C1-6 alkylamino group, or an optionally substituted C1-6 alkoxycarbonyl C1-6 alkylamino group, or
   one of R² and R³ represents a group represented by the following general formula (wherein the definition of ring A, and groups represented by R¹, R² and R⁴ is, respectively, the same as the definition of ring A, R¹, R² and R⁴ in the general formula (I)): and 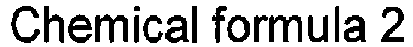 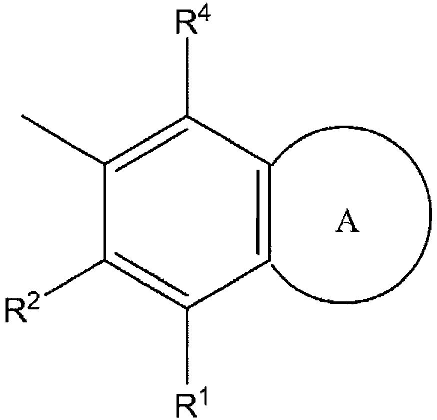 the other represents a hydrogen atom, a carboxyl group, an optionally substituted C1-6 alkyl group, a C6-10 aryl group, or a group represented by -C (=R⁹)-R¹⁰, or
   R² and R³ are taken together with the carbon atom to which they are attached to form a benzene ring or tetrahydrofuran, wherein the tetrahydrofuran may be spiro-linked with an optionally substituted tricyclic condensed heterocyclic ring],
(2) The pyruvate dehydrogenase kinase inhibitor according to (1) comprising a compound represented by the following general formula (II) or (III) or ester derivatives thereof, or pharmacologically acceptable salts thereof as an active ingredient, 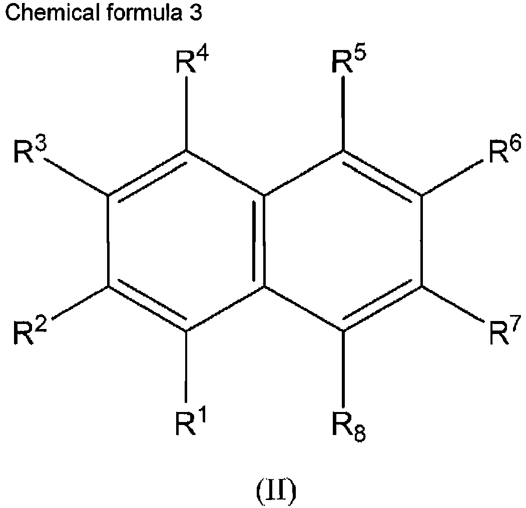 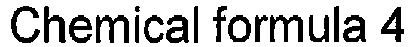 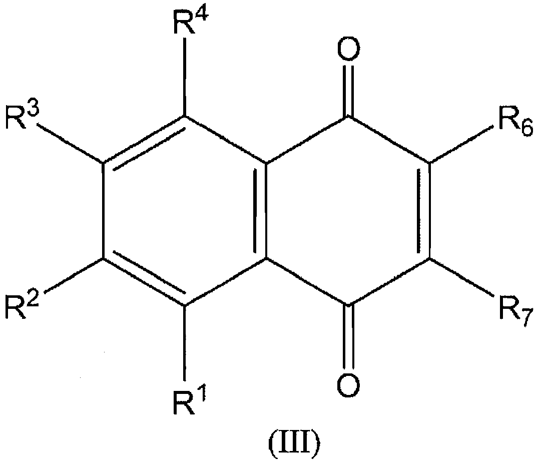 [wherein R¹ and R⁴, which are the same or different, represent a hydrogen atom, a hydroxyl group, an optionally substituted C1-6 alkyl group, an optionally substituted C2-6 alkenyl group, an optionally substituted C6-10 aryl group, an optionally substituted C1-6 alkoxy group, or an optionally substituted C2-7 alkanoyloxy group,
   R² and R³, which are the same or different, represent a hydrogen atom, a carboxyl group, an optionally substituted C1-6 alkyl, a C6-10 aryl group, or a group represented by -C (=R⁹)-R¹⁰,
   wherein R⁹ represents an oxygen atom, a sulfur atom, a = N-R¹¹ group (wherein R¹¹ represents a hydroxyl group, a C1-6 alkyl group, a C6-10 aryl group, a C1-6 alkoxy group, a C2-6 alkenyloxy group, a C6-10 aryloxy group), or a = CH-R¹² group (wherein R¹² represents a formyl group, a carboxyl group, a C1-6 alkyl group, a C6-10 aryl group, a C1-6 alkoxycarbonyl group, an aminocarbonyl group, or a C1-6 alkylaminocarbonyl group),
   R¹⁰ represents a hydrogen atom, an amino group, a C1-6 alkyl group, a C6-10 aryl group, a C1-6 alkoxy group, an optionally substituted C1-6 alkylamino group, or an optionally substituted C1-6 alkoxycarbonyl C1-6 alkylamino group, or one of R² and R³ represents a group represented by the following general formula (wherein the definition of ring A, and groups represented by R¹, R² and R⁴ is, respectively, the same as the definition of ring A, R¹, R² and R⁴ in the general formula (I)): or 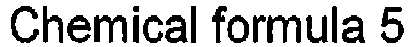 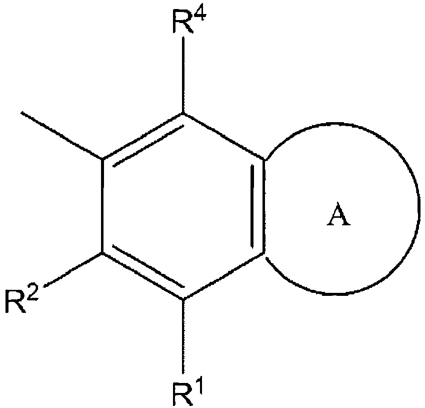 R² and R³ are taken together with the carbon atom to which they are attached to form a benzene ring or tetrahydrofuran, wherein the tetrahydrofuran may be spiro-linked with an optionally substituted tricyclic condensed heterocyclic ring,
   R⁵ and R⁸, which are the same or different, represent a hydroxyl group, an amino group, an optionally substituted C1-6 alkoxy group, an optionally substituted C2-40 alkenyloxy group, or a C2-7 alkanoyloxy group,
   R⁶ and R⁷ represent a hydrogen atom, a hydroxyl group, an optionally substituted amino group, a halogen atom, an optionally substituted C1-6 alkyl group, an optionally substituted C2-40 alkenyl, or an optionally substituted C1-6 alkoxy group,
   wherein, in the above, the substituent in case being optionally substituted is a group selected from the following: a hydroxyl group, a carboxyl group, an amino group, a halogen atom, a C1-6 alkyl group, a C2-6 alkenyl group, a C2-7 alkanoyl group, a C1-6 alkoxycarbonyl group,
   wherein, in the above, the substituent in case being optionally substituted is a group selected from the following: a hydroxyl group, a carboxyl group, an amino group, a halogen atom, a C1-6 alkyl group optionally substituted with a hydroxyl group, a C2-6 alkenyl group, a C2-7 alkanoyl group, a C1-6 alkoxy group, a C1-6 alkoxycarbonyl group, a C6-10 aryl group, a C6-10 aryl C1-6 alkyl group].
(3) A compound represented by the following general formula (II) or (III) or ester derivatives thereof, or pharmacologically acceptable salts thereof: 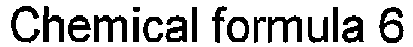 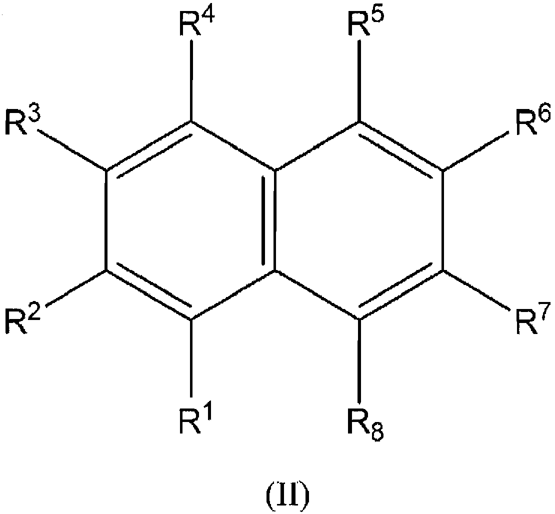 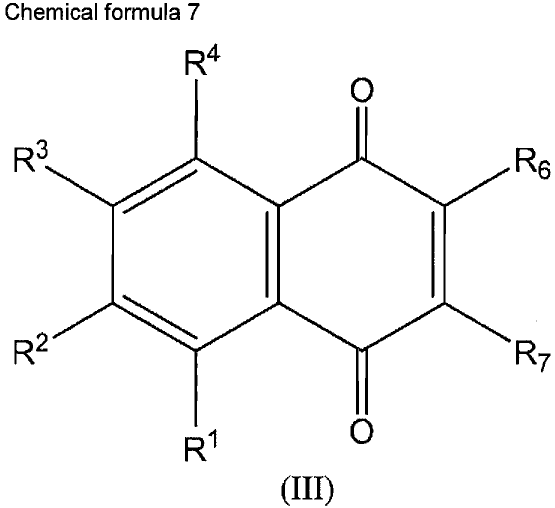 [wherein,
   R¹ and R⁴, which are the same or different, represent a hydrogen atom, an optionally substituted C1-6 alkyl group, or an optionally substituted C6-10 aryl group,
   one of R² and R³ represents a hydrogen atom, an optionally substituted C1-6 alkyl group, or an optionally substituted C6-10 aryl group,
   the other represents a group represented by -C (=R⁹)-R¹⁰,
   wherein, R⁹ represents an oxygen atom, a sulfur atom, a = N-R¹¹ group (wherein R¹¹ represents a C1-6 alkyl group, a C6-10 aryl group, a hydroxyl group, a C1-6 alkoxy group, a C2-20 alkenyloxy group, a C6-10 aryloxy group), or a = CH-R¹² group (wherein R¹² represents a C1-6 alkyl group, a C6-10 aryl group, a formyl group, a carboxyl group, a C1 - 6 alkoxycarbonyl group, an aminocarbonyl group, or a C1-6 alkylaminocarbonyl group),
   R¹⁰ represents a hydrogen atom, an amino group, an optionally substituted C1-6 alkyl group, an optionally substituted C6-10 aryl group, an optionally substituted C1-6 alkoxy group, an optionally substituted C6-10 aryloxy group, an optionally substituted C1-6 alkylamino group, or an optionally substituted C1-6 alkoxycarbonyl C1-6 alkylamino group,
   with the proviso that the group represented by -C (=R⁹)-R¹⁰ is not a carboxyl group, a methylcarbonyl group or a methoxycarbonyl group,
   both R⁵ and R⁸ represent a C1-6 alkoxy group, and
   R⁶ and R⁷ represent a hydrogen atom, an optionally substituted C1-6 alkyl group, or an optionally substituted amino group,
   wherein, in the above, the substituent in case being optionally substituted is a group selected from the following: a hydroxyl group, a carboxyl group, an amino group, a halogen atom, a C1-6 alkyl group optionally substituted with a hydroxyl group, a C2-6 alkenyl group, a C2-7 alkanoyl group, a C1-6 alkoxy group, a C1-6 alkoxycarbonyl group, a C6-10 aryl group, a C6-10 aryl C1-6 alkyl group].
(4) The compound according to (3) or ester derivatives thereof, or pharmacologically acceptable salts thereof,
   wherein R¹ and R⁴, which are the same or different, represent a hydrogen atom, or an optionally substituted C1-6 alkyl group,
   one of R² and R³ represents a hydrogen atom, or an optionally substituted C1-6 alkyl group,
   the other represents a group represented by -C (=R⁹)-R¹⁰,
   wherein R⁹ represents an oxygen atom, or a = N-R¹¹ group (wherein R¹¹ represents a C1-6 alkoxy group, a C2-6 alkenyloxy group, a C6-10 aryloxy group),
   R¹⁰ represents a hydrogen atom, an optionally substituted C1-6 alkyl group, an optionally substituted C6-10 aryl group, an optionally substituted C1-6 alkoxy group, an optionally substituted C1-6 aryloxy group, an optionally substituted C1-6 alkylamino group, or an optionally substituted C1-6 alkoxycarbonyl C1-6 alkylamino group,
   with the proviso that the group represented by -C (=R⁹)-R¹⁰ is not a carboxyl group, a methylcarbonyl group or a methoxycarbonyl group,
   both R⁵ and R⁸ represent a C1-6 alkoxy group,
   R⁶ and R⁷ represent a hydrogen atom, or an optionally substituted C1-6 alkyl group,
   wherein, in the above, the substituent in case being optionally substituted is a group selected from the following: a hydroxyl group, a carboxyl group, an amino group, a halogen atom, a C1-6 alkyl group optionally substituted with a hydroxyl group, a C2-6 alkenyl group, a C2-7 alkanoyl group, a C1-6 alkoxy group, a C1-6 alkoxycarbonyl group, a C6-10 aryl group].
(5) A pyruvate dehydrogenase kinase inhibitor comprising the compound according to (3) or (4) or ester derivatives thereof, or pharmacologically acceptable salts thereof as an active ingredient.
(6) The pyruvate dehydrogenase kinase inhibitor according to (1), (2) or (5), wherein the inhibitor is pyruvate dehydrogenase kinase inhibitor 4.
(7) A pharmaceutical composition for treatment or prophylaxis of diseases or disorders that pyruvate dehydrogenase kinase relates to its development or aggravation comprising the pyruvate dehydrogenase kinase inhibitor according to (1), (2) or (5) as an active ingredient.
(8) The pharmaceutical composition according to (7), wherein the diseases or disorders that pyruvate dehydrogenase kinase relates to its development or aggravation is influenza aggravation after infection.
(9) The pharmaceutical composition according to (7), wherein the diseases or disorders that pyruvate dehydrogenase kinase relates to its development or aggravation is anorexia.
(10) The pharmaceutical composition according to (7), wherein the diseases or disorders that pyruvate dehydrogenase kinase relates to its development or aggravation is mitochondrial diseases, or diseases or disorders accompanied by decreasing ATP production.
(11) The pharmaceutical composition according to (7), wherein the diseases or disorders that pyruvate dehydrogenase kinase relates to its development or aggravation is diabetes.
(12) The pharmaceutical composition according to (7), wherein the diseases or disorders that pyruvate dehydrogenase kinase relates to its development or aggravation is cancer.
(13) A cosmetic composition comprising the pyruvate dehydrogenase kinase inhibitor according to (1), (2) or (5).

As used herein, the term "6-membered aromatic hydrocarbon ring" refers to a benzene ring or a p-benzoquinone (2,5-cyclohexadiene substituted in position-1 and position-4 with an oxo group).

As used herein, the term "C1-6 alkyl group" refers to a straight or branched saturated hydrocarbon group having a carbon number of 1 to 6 such as, for example, a methyl group, an ethyl group, a n-propyl group, an i-propyl group, a n-butyl group, a sec-butyl group, a t-butyl group, an isobutyl group, a pentyl group, an isopentyl group, a 2,3-dimethyl propyl group, a hexyl group, and a cyclohexyl group, preferably a C1-5 alkyl group, more preferably a methyl group, an ethyl group, a n-propyl group, an i-propyl group, a n-butyl group, a sec-butyl group, a t-butyl group, an isobutyl group, a pentyl group, an isopentyl group, or a 2,3-dimethyl propyl group. More preferably, it is a C1-3 alkyl group, such as a methyl group, an ethyl group, a n-propyl group, and an i-propyl group, and most preferably a methyl group or an ethyl group.

As used herein, the term "C2-40 alkenyl group" refers to a monovalent group having a carbon number of 2 to 40 obtained by removing one hydrogen atom from an optional carbon atom of the straight or branched unsaturated hydrocarbon having one or more double bond between carbons. The term "C2-6 alkenyl group" refers to a monovalent group having a carbon number of 2 to 6 obtained by removing one hydrogen atom from an optional carbon atom of the straight or branched unsaturated hydrocarbon having one or more double bond between carbons. The C2-6 alkenyl group or C2-40 alkenyl group includes, for example, a vinyl group, a propenyl group, an isopropenyl, a 1-butenyl group, a 2-butenyl group, a 3-butenyl group, a 1-methyl-1-propenyl group, a 2-methyl-1-propenyl group, a 1-methyl-2-propenyl group, a 2-methyl-2-propenyl group, a 1-methylidene-1-propane group, a 1-pentenyl group, a 3-pentenyl group, a 4-pentenyl group, a 1-methyl-1-butenyl group, a 1-methyl-2-butenyl group, a 1-methyl-3-butenyl group, a 1-methylidenebutyl group, a 2-methyl-1-butenyl group, a 2-methyl-2-butenyl group, a 2-methyl-3-butenyl group, a 2-methylidenebutyl group, a 3-methyl-1-butenyl group, a 3-methyl-2-butenyl group, a 3-methyl-3-butenyl group, a 1-ethyl-1-propenyl group, a 1-ethyl-2-propenyl group, a 1-hexenyl group, a 2-hexenyl group, a 3-hexenyl group, a 4-hexenyl group, a 5-hexenyl group, a 1-methyl-1-pentenyl group, a 1-methyl-2-pentenyl group, a 1-methyl-3-pentenyl group, a 1-methyl-4-pentenyl group, a 1-methylidenepentyl group, a 2-methyl-1-pentenyl group, a 2-methyl-2-pentenyl group, a 2-methyl-3-pentenyl group, a 2-methyl-4-pentenyl group, a 2-methylidenepentyl group, a 3-methyl-1-pentenyl group, a 3-methyl-2-pentenyl group, a 3-methyl-3-pentenyl group, a 3-methyl-4-pentenyl group, a 3-methylidenepentyl group, a 4-methyl-1-pentenyl group, a 4-methyl-2-pentenyl group, a 4-methyl-3-pentenyl group, a 4-methyl -4-pentenyl group, 1-ethyl-1-butenyl group, a 1-ethyl-2-butenyl group, a 1-ethyl-3-butenyl group, a 2-ethyl-1-butenyl group, 2-ethyl-2-butenyl group, a 2-ethyl-3-butenyl group, a 1- (1-methylethyl) -1-propenyl group, a 1- (1-methylethyl) -2-propenyl group, a 1-ethyl-2-methyl-1-propenyl group, or 1-ethyl-2-methyl-2-propenyl group.

Further, the C2-40 alkenyl group includes a 1-heptenyl group, a 2-heptenyl group, a 3-heptenyl group, a 4-heptenyl group, a 5-heptenyl group, a 7-heptenyl group, a 1-methyl-1-hexenyl group, a 1-methyl-2-hexenyl group, a 1-methyl-3-hexenyl group, a 1-methyl-4-hexenyl group, a 1-methyl-5-hexenyl group, a 1-methylidenehexyl group, a 2-methyl-1-hexenyl group, a 2-methyl-2-hexenyl group, a 2-methyl-3-hexenyl group, a 2-methyl-4-hexenyl group, a 2-methyl-5-hexenyl group, a 2-methylidenehexyl group, a 3-methyl-1-hexenyl group, a 3-methyl-2-hexenyl group, a 3-methyl-3-hexenyl group, a 3-methyl-4-hexenyl group, a 3-methyl-5-hexenyl group, a 3-methylidenehexyl group, a 4 methyl-1-hexenyl group, a 4-methyl-2-hexenyl group, a 4-methyl-3-hexenyl group, a 4-methyl-4-hexenyl group, a 4-methyl-5-hexenyl group, a 4-methylidenehexyl group, a 5-methyl-1-hexenyl group, a 5-methyl-2-hexenyl group, a 5-methyl-3-hexenyl group, a 5-methyl-4-hexenyl group, a 5-methyl-5-hexenyl group, a 1-ethyl-1-pentenyl group, a 1-ethyl-2-pentenyl group, a 1-ethyl-3-pentenyl group, a 1-ethyl-4-pentenyl group, a 2-ethyl-1-pentenyl group, a 2-ethyl-2-pentenyl group, a 2-ethyl-3-pentenyl group, a 2-ethyl-4-pentenyl group, a 3-ethyl-1-pentenyl group, a 3-ethyl-2-pentenyl group, a 3-ethyl-3-pentenyl group, a 3-ethyl-4-pentenyl group, a 1,1-dimethyl-2-pentenyl group, a 1,1-dimethyl-3-pentenyl group, a 1,1-dimethyl-4-pentenyl group, a 2,2-dimethyl-3-pentenyl group, a 2,2-dimethyl-4-pentenyl group, a 3,3-dimethyl-1-pentenyl group, a 3,3-dimethyl-4-pentenyl group, a 4,4-dimethyl-1-pentenyl group, a 4,4-dimethyl-2-pentenyl group, a 1,2-dimethyl-1-pentenyl group, a 1,2-dimethyl-2-pentenyl group, a 1,2-dimethyl-3-pentenyl group, a 1,2-dimethyl-4-pentenyl group, a 1-methylidene-2-methylpentyl group, a 2-methylidene-1-methylpentyl group, a 1,3-dimethyl-1-pentenyl group, a 1,3-dimethyl-2-pentenyl group, a 1,3-dimethyl-3-pentenyl group, a 1,3-dimethyl-4-pentenyl group, a 1-methylidene-3-methylpentyl group, a 3-methylidene-1-methylpentyl group, a 1,4-dimethyl-1-pentenyl group, a 1,4-dimethyl-2-pentenyl group, a 1,4-dimethyl-3-pentenyl group, a 1,4-dimethyl-4-pentenyl group, a 1-methylidene-4-methylpentyl group, a 1,1,2-trimethyl-2-butenyl group, a 1,1,2-trimethyl-3-butenyl group, a 1,1-dimethyl-2-methylidenebutyl group, a 1,1,3-trimethyl-2-butenyl group, a 1,1,3-trimethyl-3-butenyl group, a 1,2,2-trimethyl-3-butenyl group, a 2,2-dimethyl-1-methylidenebutyl group, 1,2,3-trimethyl-1-butenyl group, a 1,2,3-trimethyl-2-butenyl group, a 1,2,3-trimethyl-3-butenyl group, a 2,3-dimethyl-1-methylidenebutyl group, a 1,3-dimethyl-2-methylidenebutyl group, a 2,2,3-trimethyl-3-butenyl group, a 2,3,3-trimethyl-1-butenyl group, a 3,3-dimethyl-2-methylidenebutyl group, a 1-ethyl-1-methyl-2-butenyl group, a 1-ethyl-1-methyl-3-butenyl group, a 1-methyl-1-propyl-2-propenyl group, a 1-ethyl-2-methyl-1-butenyl group, a 1-ethyl-2-methyl-2-butenyl group, a 1-ethyl-2-methyl-3-butenyl group, a 1-(1-methylpropyl)-2-propenyl group, a 1-ethylidene-2-methylbutyl group, a 1-ethyl-3-methyl-1-butenyl group, a 1-ethyl-3-methyl-2-butenyl group, a 1-ethyl-3-methyl-3-butenyl group, a 1-(2-methylpropyl)-2-propenyl group, a 1-ethylidene-3-methylbutyl group, a 1-ethyl-3-methyl-1-butenyl group, a 1-ethyl-3-methyl-2-butenyl group, a 1-ethyl-3-methyl-3-butenyl group, a 1-ethynyl-3-methylbutyl group, a 1-ethylidene-3-methylbutyl group, a 1-methyl-2-ethyl-1-butenyl group, a 1-methyl-2-ethyl-2-butenyl group, a 1-methyl-2-ethyl-3-butenyl group, a 2-methyl-2-ethyl-3-butenyl group, a 1-(1-methylethyl)-1-butenyl group, a 1-(1-methylethyl)-2-butenyl group, a 1-(1-methylethyl)-3-butenyl group, a 2-methyl-1-propyl-1-propenyl group, a 2-methyl-1-propyl-2-propenyl group, a 2-(1-methylethyl)-1-butenyl group, a 2-(1-methylethyl)-2-butenyl group, a 2-(1-methylethyl)-3-butenyl group, a 2-ethyl-3-methyl-2-butenyl group, a 2-ethyl-3-methyl-3-butenyl group, or -CH₂-CH=C (CH₃)-((CH₂)₃-C(CH₃))ₙ-CH₃ group (n is a natural number of 1-10, preferably is a natural number of 1-5, more preferably a natural number of 2-4), (-CH₂-CH=C(CH₃) -CH₂-)ₙ-H group (n is a natural number of 1-8, preferably a natural number of 2-6, more preferably a natural number of 3-5), or, -(CH₂-CH=CH)ₙ-H group (n is a natural number of 1-13 preferably is a natural number of 1-6, more preferably natural number of 1-3).

As used herein, the term "C1-6 alkoxy group" refers to a group ((C1-6 alkyl) -O-group) bonding to the C1-6 alkyl group via an oxygen atom, and the alkyl moiety may be straight or branched. The C1-6 alkoxy group means that a number of carbon atoms in the alkyl moiety is 1-6. The alkoxy group includes, for example, a methoxy group, an ethoxy group, a 1-propyloxy group, a 2-propyloxy group, a 2-methyl-1-propyloxy group, a 2-methyl-2-propyloxy group, a 2,2-dimethyl-1-propyloxy group, a 1-butyloxy group, a 2-butyloxy group, a 2-methyl-1-butyloxy group, a 3-methyl-1-butyloxy group, a 2-methyl-2-butyloxy group, a 3-methyl-2-butyloxy group, a 1-pentyloxy group, a 2-pentyloxy group, 3-pentyloxy group, 2-methyl-1-pentyloxy group, a 3-methyl-1-pentyloxy group, a 2-methyl-2-pentyloxy group, a 3-methyl-2-pentyloxy group, a 1-hexyloxy group, a 2-hexyloxy group, and a 3-hexyloxy group. The C1-6 alkoxy group may be preferably a C1-5 alkoxy group, more preferably a methoxy group, an ethoxy group, a n-propyloxy group, a i-propyloxy group, a n-butyloxy group, a sec-butyloxy group, a t-butyloxy group, an isobutyloxy group, a pentyloxy group, an isopentyloxy group, and a 2,3-dimethylpropyloxy group, more preferably a C1-3 alkoxy group (a methoxy group, an ethoxy group, and propyloxy group), yet more preferably, a methoxy group or an ethoxy group.

As used herein, the term "C2-6 alkenyloxy group" refers to a group ((C2-6 alkenyl)-O-group) bonding to the C2-6 alkenyl group via an oxygen atom, and the alkenyl moiety may be straight or branched. The C2-6 alkenyl group includes, for example, a vinyloxy group, a propenyloxy group, an isopropenyloxy group, a 1-butenyloxy group, a 2-butenyloxy group, a 3-butenyloxy group, a 1-methyl-1-propenyloxy group, a 2-methyl-1-propenyloxy group, a 1-methyl-2-propenyloxy group, a 2-methyl-2-propenyloxy group, a 1-methylidene-1-propaneoxy group, a 1-pentenyloxy group, a 3-pentenyloxy group, a 4-pentenyloxy group, a 1-methyl-1-butenyloxy group, a 1-methyl-2-butenyloxy group, a 1-methyl-3-butenyloxy group, a 1-methylidenebutyloxy group, a 2-methyl-1-butenyloxy group, a 2-methyl-2-butenyloxy group, a 2-methyl-3-butenyloxy group, a 2-methylidenebutyloxy group, 3-methyl-1-butenyloxy group, a 3-methyl-2-butenyloxy group, a 3-methyl-3-butenyloxy group, a 1-ethyl-1-propenyloxy group, a 1-ethyl-2-propenyloxy group, a 1-hexenyloxy group, a 2-hexenyloxy group, a 3-hexenyloxy group, a 4-hexenyloxy group, 5-hexenyloxy group, a 1-methyl-1-pentenyloxy group, a 1-methyl-2-pentenyloxy group, a 1-methyl-3-pentenyloxy group, a 1-methyl-4-pentenyloxy group, a 1-methylidenepentyloxy group, a 2-methyl-1-pentenyloxy group, a 2-methyl-2-pentenyloxy group, a 2-methyl-3-pentenyloxy group, a 2-methyl-4-pentenyloxy group, a 2-methylidenepentyloxy group, a 3-methyl-1-pentenyloxy group, a 3-methyl-2-pentenyloxy group, a 3-methyl-3-pentenyloxy group, a 3-methyl-4-pentenyloxy group, a 3-methylidenepentyloxy group, a 4-methyl-1-pentenyloxy group, a 4-methyl-2-pentenyloxy group, a 4-methyl-3-pentenyloxy group, a 4-methyl-4-pentenyloxy group, a 1-ethyl-1-butenyloxy group, a 1-ethyl-2-butenyloxy group, a 1-ethyl-3-butenyloxy group, a 2-ethyl-1-butenyloxy group, a 2-ethyl-2-butenyloxy group, a 2-ethyl-3-butenyloxy group, a 1-(1-methylethyl)-1-propenyloxy group, a 1-(1-methylethyl)-2-propenyloxy group, a 1-ethyl-2-methyl-1-propenyloxy group, a 1-ethyl-2-methyl-2-propenyloxy group, or a -O-(CH₂-CH=CH)ₙ-H group (n is a natural number of 1 or 2).

As used herein, the term "C1-6 alkoxycarbonyl group" refers to a group ((C1-6 alkyl)-O-C(=O)-group) bonding to the alkoxy group via a carboxyl group, and the alkyl moiety may be straight or branched. The C1-6 alkoxycarbonyl group means that a number of carbon atoms in the alkyl moiety is 1 to 6. The C1-6 alkoxycarbonyl group includes, for example, a methoxycarbonyl group, an ethoxycarbonyl group, a 1-propyloxycarbonyl group, a 2-propyloxycarbonyl group, a 2-methyl-1-propyloxycarbonyl group, a 2-methyl-2-propyloxycarbonyl group, a 2,2-dimethyl-1-propyloxycarbonyl group, a 1-butyloxycarbonyl group, a 2-butyloxycarbonyl group, a 2-methyl-1-butyloxycarbonyl group, a 3-methyl-1-butyloxycarbonyl group, a 2-methyl-2-butyloxycarbonyl group, a 3-methyl-2-butyloxycarbonyl group, a 1-pentyloxycarbonyl group, a 2-pentyloxycarbonyl group, a 3-pentyloxycarbonyl group, a 2-methyl-1-pentyloxycarbonyl group, a 3-methyl-1-pentyloxycarbonyl group, a 2-methyl-2-pentyloxycarbonyl group, a 3-methyl-2-pentyloxycarbonyl group, a 1-hexyloxycarbonyl group, a 2-hexyloxycarbonyl group, a 3-hexyloxycarbonyl group. The C1-6 alkoxy group is preferably a C1-5 alkoxy group, more preferably a methoxy group, an ethoxy group, a n-propyloxycarbonyl group, a i-propyloxycarbonyl group, a n-butyloxycarbonyl group, a sec-butyloxycarbonyl group, a t-butyloxycarbonyl group, a isobutyloxycarbonyl group, a pentyloxycarbonyl group, a isopentyloxycarbonyl group, and a 2,3-dimethylpropyloxycarbonyl group, more preferably a C1-3 alkoxycarbonyl group (a methoxycarbonyl group, an ethoxycarbonyl group and a propyloxycarbonyl group), and yet more preferably a methoxycarbonyl group or an ethoxycarbonyl group.

As used herein, the term "C2-7 alkanoyl group" refers to a group ((C1-6 alkyl group) -C(=O)- group) bonding to the C1-6 alkyl group via an oxo group, and the alkyl moiety may be straight or branched. The C2-7 alkanoyl group includes, for example, an acetyl group, a propionyl group, a butyryl group, an isobutyryl group, a valeryl group, an isovaleryl group, a pivaloyl group, a valeryl group, an isovaleryl group, a hexanoyl group, a heptanoyl group.

As used herein, the term "C2-7 alkanoyloxy group" refers to a group ((C1-6 alkyl group) -C(=O)-O- group) bonding to the C1-6 alkyl group via an oxo group and an oxygen atom, and the alkyl moiety may be straight or branched. The C2-7 alkanoyloxy group includes, for example, an acetyloxy group, a propionyloxy group, a butyryloxy group, an isobutyryloxy group, a valeryloxy group, an isovaleryloxy group, a pivaloyloxy group, a valeryloxy group, an isovaleryloxy group, a hexanoyloxy group, a heptanoyloxy group.

As used herein, the term "C6-10 aryl group" refers to an aromatic hydrocarbon group having 6-10 carbon atoms, including benzene and naphthalene. The term "C6-10 aryloxy group" refers to a group ((C6-10 aryl group) -O- group) bonding to the C6-10 aryl group via oxygen atom, including a benzyloxy group and a naphthyloxy group.

As used herein, the term "C1-6 alkylamino group" refers to a group ((C1-6 alkyl group)-NH- group) bonding to the C1-6 alkyl group via a nitrogen atom, and the C1-6 alkyl moiety included in the group is the same defined in the C1-6 alkyl above. Such group includes, for example, a methylamino group, an ethylamino group, a n-propylamino group, an i-propylamino group, a n-butylamino group, a sec-butylamino group, a t-butylamino group, an isobutylamino group, a pentylamino group, an isopentylamino group, a 2,3-dimethylpropylamino group, a hexylamino group, and a cyclohexylamino group, preferably a C1-5 alkylamino group, more preferably a methylamino group, an ethylamino group, a n-propylamino group, an i-propylamino group, a n-butylamino group, a sec-butylamino group, a t-butylamino group, an isobutylamino group, a pentylamino group, an isopentylamino group, or a 2,3-dimethylpropylamino group. More preferably, it is a C1-3 alkylamino group such as a methylamino group, an ethylamino group, a n-propylamino group, and an i-propylamino group, and most preferably a methylamino group or an ethylamino group.

As used herein, the term "C1-6 alkylaminocarbonyl group", refers to a group ((C1-6 alkyl group) -NH-C(=O)-group) bonding to the C1-6 alkyl group via a nitrogen atom and a carboxyl group, and the C1-6 alkyl moiety included in the group is the same defined in the C1-6 alkyl above. Such group includes, for example, a methylaminocarbonyl group, an ethylaminocarbonyl group, a n-propylaminocarbonyl group, an i-propylaminocarbonyl group, a n-butylaminocarbonyl group, a sec-butylaminocarbonyl group, a t-butyl aminocarbonyl group, an isobutylaminocarbonyl group, a pentylaminocarbonyl group, an isopentylaminocarbonyl group, a 2,3-dimethylpropylaminocarbonyl group, a hexylaminocarbonyl group, and a cyclohexylaminocarbonyl group, preferably a C1-5 alkyl aminocarbonyl group, more preferably a methylaminocarbonyl group, an ethylaminocarbonyl group, a n-propylaminocarbonyl group, an i-propylaminocarbonyl group, a n-butylaminocarbonyl group, a sec-butylaminocarbonyl group, a t-butylaminocarbonyl group, an isobutylaminocarbonyl group, a pentylaminocarbonyl group, an isopentylaminocarbonyl group, or a 2,3-dimethylpropylaminocarbonyl group. More preferably, it is a C1-3 alkylaminocarbonyl group such as a methylaminocarbonyl group, an ethylaminocarbonyl group, a n-propylaminocarbonyl group, and an i-propylaminocarbonyl group, and the most preferably a methylaminocarbonyl or an ethylaminocarbonyl group.

As used herein, the term "C1-6 alkoxycarbonyl C1-6 alkylamino group" refers to a group represented by (C1-6 alkyl group) -O-C(=O)-(C1-6 alkylene) -NH-, the C1-6 alkyl moiety included in the group is the same defined in the C1-6 alkyl above. The C1-6 alkylene moiety included in the group is a divalent group obtained by removing one hydrogen atom from the C1-6 alkyl group. Such group includes, for example, a methoxycarbonylmethyleneamino group, an ethoxycarbonylmethyleneamino group, a 1-propyloxycarbonylmethyleneamino group, a 2-propyloxycarbonylmethyleneamino group, a 2-methyl-1-propyloxycarbonylmethyleneamino group, a 2-methyl-2-propyloxycarbonylmethyleneamino group, a 2,2-dimethyl-1-propyloxycarbonylmethyleneamino group, a 1-butyloxycarbonylmethyleneamino group, a 2-butyloxycarbonylmethyleneamino group, a 2-methyl-1- butyloxycarbonylmethyleneamino group, a 3-methyl-1-butyloxycarbonylmethyleneamino group, a 2-methyl-2-butyloxycarbonylmethyleneamino group, a 3-methyl-2-butyloxycarbonylmethyleneamino group, a 1-pentyloxycarbonylmethyleneamino group, a 2-pentyloxycarbonylmethyleneamino group, a 3-pentyloxycarbonylmethyleneamino group, a 2-methyl-1-pentyloxycarbonylmethyleneamino group, a 3-methyl-1-pentyloxycarbonylmethyleneamino group, a 2-methyl-2-pentyloxycarbonylmethyleneamino group, a 3-methyl-2-pentyloxycarbonylmethyleneamino group, a 1-hexyloxycarbonylmethyleneamino group, a 2-hexyloxycarbonylmethyleneamino group, a 3-hexyloxycarbonylmethyleneamino group, a methoxycarbonylethyleneamino group, an ethoxycarbonylethyleneamino group, a 1-propyloxycarbonylethyleneamino group, a 2-propyloxycarbonylethyleneamino group, a 2-methyl-1-propyloxycarbonylethyleneamino group, a 2-methyl-2-propyloxycarbonylethyleneamino group, a 2,2-dimethyl-1-propyloxycarbonylethyleneamino group, a 1-butyloxycarbonylethyleneamino group, a 2-butyloxycarbonylethyleneamino group, a 2- methyl-1-butyloxycarbonylethyleneamino group, 3-methyl-1-butyloxycarbonylethyleneamino group, a 2-methyl-2-butyloxycarbonylethyleneamino group, a 3-methyl-2-butyloxycarbonylethyleneamino group, a 1-pentyloxycarbonylethyleneamino group, a 2-pentyloxycarbonylethyleneamino group, a 3-pentyloxycarbonylethyleneamino group, a 2-methyl-1-pentyloxycarbonylethyleneamino group, a 3-methyl-1-pentyloxycarbonylethyleneamino group, a 2-methyl-2-pentyloxycarbonylethyleneamino group, a 3-methyl-2-pentyloxycarbonylethyleneamino group, a 1-hexyloxycarbonylethyleneamino group, a 2-hexyloxycarbonylethyleneamino group, a 3-hexyloxycarbonylethyleneamino group, a methoxycarbonylpropyleneamino group, an ethoxycarbonylpropyleneamino group, a 1-propyloxycarbonylpropyleneamino group, a 2-propyloxycarbonylpropyleneamino group, a 2-methyl-1-propyloxycarbonylpropyleneamino group, a 2-methyl-2-propyloxycarbonylpropyleneamino group, a 2,2-dimethyl-1-propyloxycarbonylpropyleneamino group, a 1-butyloxycarbonylpropyleneamino group, a 2-butyloxycarbonylpropyleneamino group, a 2-methyl-1-butyloxycarbonylpropyleneamino group, a 3-methyl-1-butyloxycarbonylpropyleneamino group, a 2-methyl-2-butyloxycarbonylpropyleneamino group, a 3-methyl-2-butyloxycarbonylpropyleneamino group, a 1-pentyloxycarbonylpropyleneamino group, a 2-pentyloxycarbonylpropyleneamino group, a 3-pentyloxycarbonylpropyleneamino group, a 2-methyl-1-pentyloxycarbonylpropyleneamino group, a 3-methyl-1-pentyloxycarbonylpropyleneamino group, a 2-methyl-2-pentyloxycarbonylpropyleneamino group, a 3-methyl-2-pentyloxycarbonylpropyleneamino group, a 1-hexyloxycarbonylpropyleneamino group, a 2-hexyloxycarbonylpropyleneamino group, 3-hexyl-butyloxycarbonyl propylene. The C1-6 alkoxycarbonyl C1-6 alkylamino group is preferably a C1-5 alkoxycarbonyl C1-5 alkyl group, more preferably a C1-4 alkoxycarbonyl C1-3 alkylamino group.

As used herein, an oxo group refers to a group represented by =O. The term "halogen atom" refers to a fluorine atom, a chlorine atom, a bromine atom, and an iodine atom, preferably a fluorine atom, a chlorine atom, and a bromine atom, and more preferably a fluorine atom or a chlorine atom. As used herein, the term "formyl group" refers to a group represented by -C(=O)-H. As used herein, the term "carboxyl group" refers to a group represented by -C(=O)-OH. The term "aminocarbonyl group" refers to a NH₂-C(=O)- group.

The term "pharmacologically acceptable salt(s)" refers to a salt obtained by bonding the compound of the present invention to organic or inorganic base or acid, it is acceptable to be administered to a body as a medicine. Such salts are, for example, described in Berge et al., J. Pharm. Sci. 66:1-19 (1977) and the like. The salts include, for example, when acidic groups such as a carboxylic acid group exist, salts of alkali metal and alkaline earth metal such as lithium, sodium, potassium, magnesium, calcium; salts of amines such as ammonia, methylamine, dimethylamine, trimethylamine, dicyclohexylamine, tris (hydroxymethyl) aminomethane, N, N-bis (hydroxyethyl) piperazine, 2-amino-2-methyl-1-propanol, ethanolamine, N-methylglucamine, L-glucamine; or salts of basic amino acids such as lysine, δ-hydroxylysine, argiriine. When basic groups exist, the salts include, for example, salts of mineral acids such as hydrochloric acid, hydrobromic acid, sulfuric acid, nitric acid, phosphoric acid; salts of organic acids such as methanesulfonic acid, benzenesulfonic acid, p-toluenesulfonic acid, acetic acid, propionic acid salt, tartaric acid, fumaric acid, maleic acid, malic acid, oxalic acid, succinic acid, citric acid, benzoic acid, mandelic acid, cinnamic acid, lactic acid, glycolic acid, glucuronic acid, ascorbic acid, nicotinic acid, salicylic acid; or salts of acidic amino acids such as aspartic acid, glutamic acid. Hydrates or solvates of the compound represented by the general formula(1) and hydrates or solvates of the pharmaceutically acceptable salts of the compound represented by the general formula(1) are also included in the compound of the present invention. As used herein, the term "compound represented by the general formula (1)," except in the case it is clearly unsuitable, if it is not mentioned, it includes pharmaceutically acceptable salts of the compound represented by the general formula (1), hydrates or solvates thereof, and also hydrates or solvates of the pharmaceutically acceptable salts of the compound represented by the general formula (1).

Since the compound of the present invention has a chiral carbon, optical isomers thereof exist. The compound of the present invention may include either of dextrorotatory (+) or levorotatory (-) compounds, or mixture thereof such as racemates. The compound represented by the general formula (1) in the present invention includes, unless otherwise specified, any tautomers or geometric isomers (eg, E-form, Z-form).

The compound of the present invention includes, in addition to the compounds above, pharmacologically acceptable esters thereof. The term "pharmacologically acceptable esters" refers to compounds that have a group which is metabolized in vivo to give the compound of the present invention, and esters which may be administered to the body as a medicament. As used herein, esters include a compound having an ester bond, and also a compound having an amide bond. The esters may be degraded by an esterase in vivo to give an active compound. The esters include, for example, substituted or unsubstituted, ower alkyl esters, lower alkenyl esters, lower alkylamino-lower alkyl esters, acylamino lower alkyl esters, acyloxy lower alkyl esters, aryl esters, aryl-lower alkyl esters, amides, lower alkyl amide, amide hydroxide. The preferred esters is propionic acid esters or acyl esters.

The compound represented by the formula (I) of the present invention includes the compound represented by the following general formula:

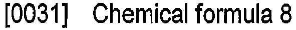

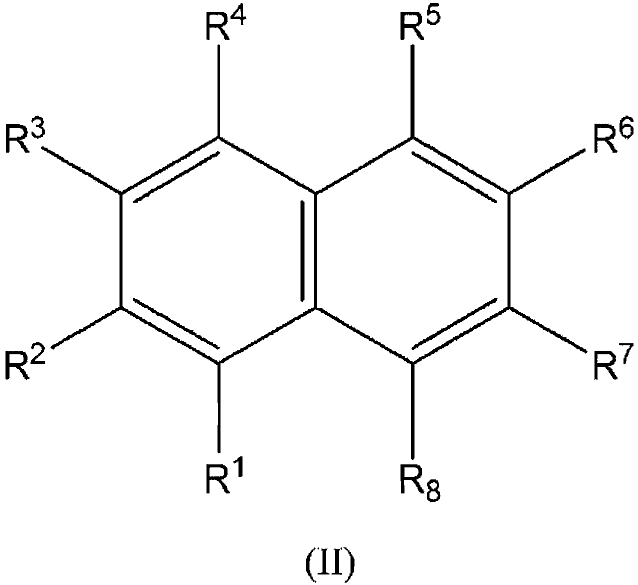

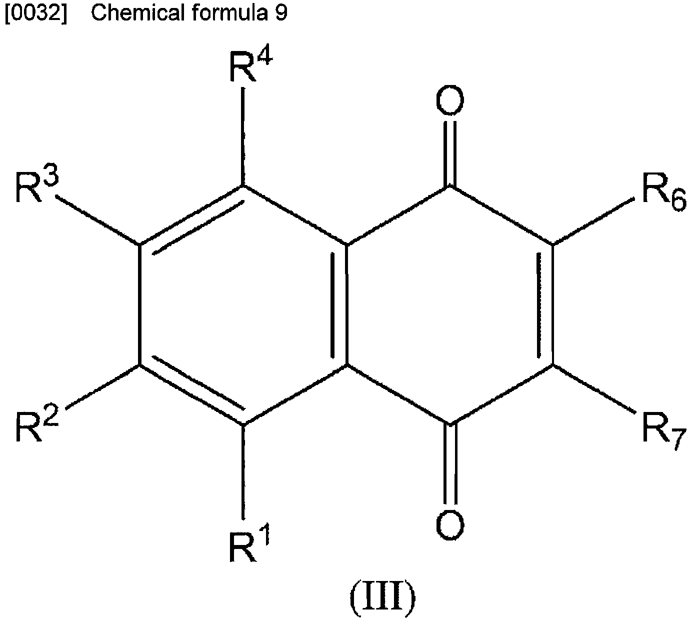

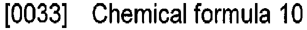

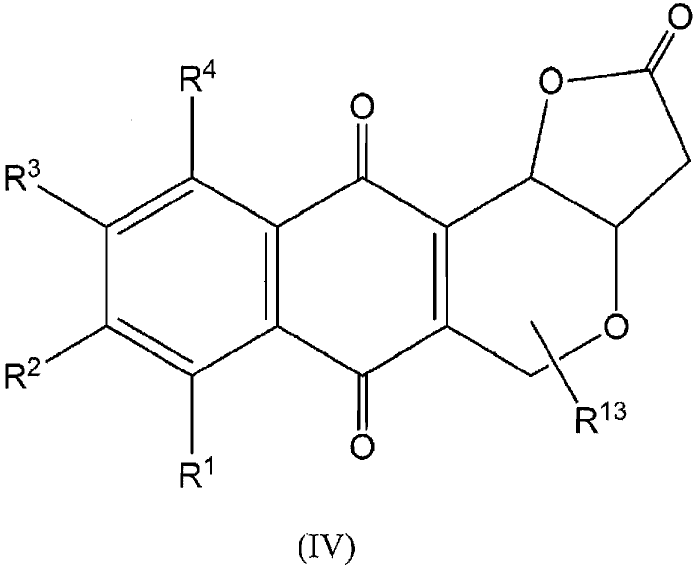

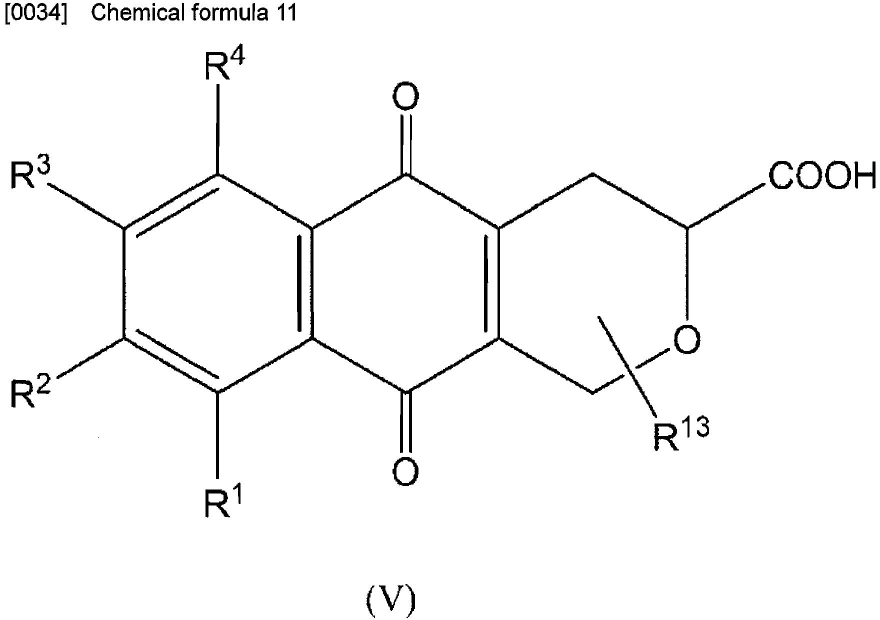

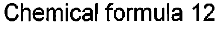

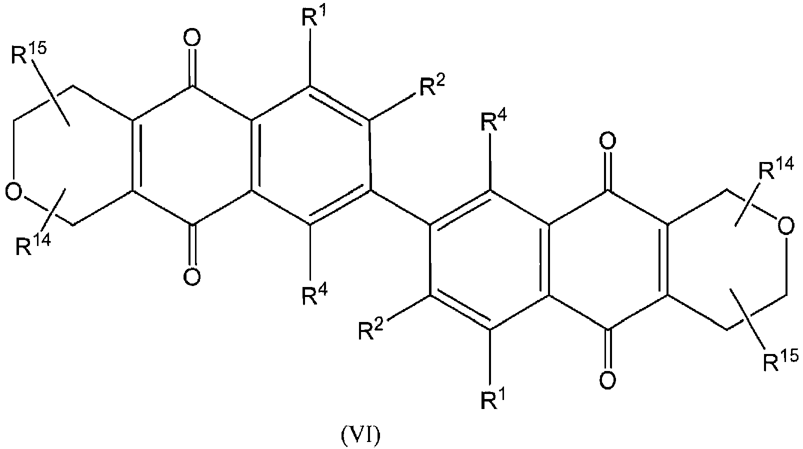

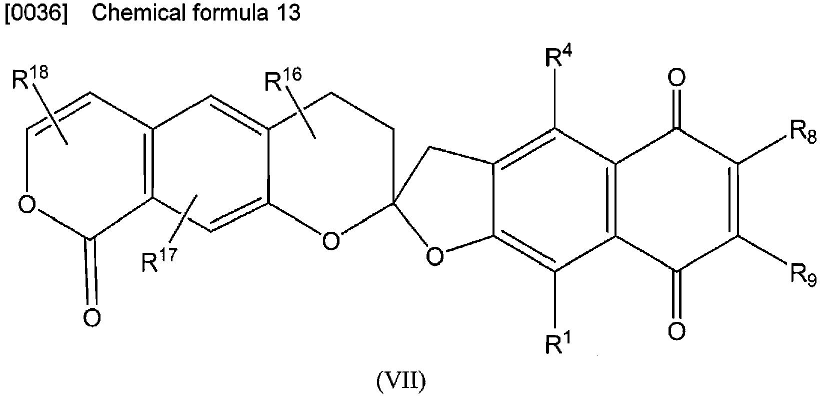

In the above, R¹³ represents an optionally substituted C1-6 alkyl group, R¹⁴ and R¹⁵, which are the same or different, represent an optionally substituted C1-6 alkyl group, R¹⁶, R¹⁷, and R¹⁸, which are the same or different, represent a hydroxyl group, or a C1-6 alkoxycarbonyl group.

In the compound represented by the general formulas (I) to (VII) (hereinafter "compound represented by the general formula (I), etc."), R¹ and R⁴, which are the same or different, represent a hydrogen atom, a hydroxyl group, an optionally substituted C1-6 alkyl, an optionally substituted C2-6 alkenyl, an optionally substituted C6-10 aryl group, an optionally substituted C1-6 alkoxy group, or an optionally substituted C2-7 alkanoyloxy group. Wherein, R¹ and R⁴ preferably include the following groups:
(1-1) R¹ and R⁴, which are the same or different, represent a hydrogen atom, a hydroxyl group, an optionally substituted C1-6 alkyl, an optionally substituted C2-6 alkenyl, or an optionally substituted C6-10 aryl group;
(1-2) R¹ and R⁴, which are the same or different, represent a hydrogen atom, a hydroxyl group, an optionally substituted C1-6 alkyl, or an optionally substituted C6-10 aryl group;
(1-3) R¹ and R⁴, which are the same or different, represent a hydrogen atom, a hydroxyl group, or an optionally substituted C1-6 alkyl;
(1-4) R¹ and R⁴, which are the same or different, represent a hydrogen atom, or a hydroxyl group;
(1-5) R¹ and R⁴, which are the same or different, represent a hydrogen atom, or an optionally substituted C1-6 alkyl;
(1-6) R¹ and R⁴, which are the same or different, represent a hydrogen atom, or an optionally substituted C1-6 alkyl; or
(1-7) R¹ and R⁴ are both hydrogen atom.

In the compound represented by the general formula (I) of the present specification, R² and R³, which are the same or different, represent a hydrogen atom, a carboxyl group, an optionally substituted C1-6 alkyl group, a C6-10 aryl group, or a group represented by -C (=R⁹)-R¹⁰, or one of R² and R³ represents a group represented by the following general formula (wherein the definition of ring A, and groups represented by R¹, R² and R⁴ is, respectively, the same as the definition of ring A, R¹, R² and R⁴ in the general formula (I)): and 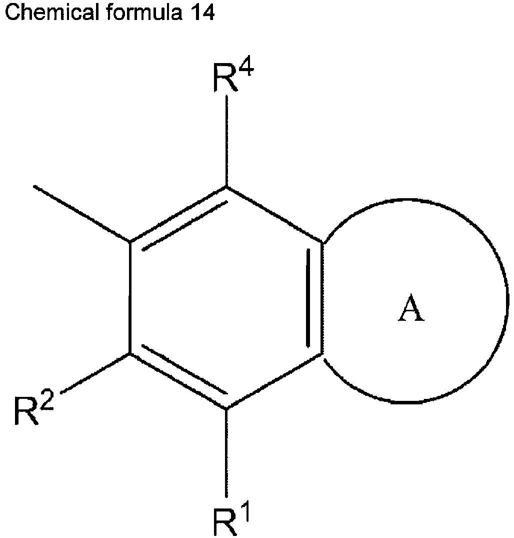 the other represents a hydrogen atom, a carboxyl group, an optionally substituted C1-6 alkyl group, a C6-10 aryl group, or a group represented by -C (=R⁹)-R¹⁰, or R² and R³ are taken together with the carbon atom to which they are attached to form a benzene ring or tetrahydrofuran (the tetrahydrofuran may be spiro-linked with an optionally substituted tricyclic condensed heterocyclic ring). Here, preferable R² and R³ may include:
(2-1) one of R² and R³ represents a hydrogen atom, a hydroxyl group, an optionally substituted C1-6 alkyl group, or an optionally substituted C6-10 aryl group, and the other represents a carboxyl group, a C1-6 alkyl group, or a group represented by -C (=R⁹)-R¹⁰;
(2-2) one of R² and R³ represents a hydrogen atom, a hydroxyl group, or an optionally substituted C1-6 alkyl group, and the other represents a carboxyl group, or a group represented by -C (=R⁹)-R¹⁰;
(2-3) R² represents a hydrogen atom, a hydroxyl group, or an optionally substituted C1-6 alkyl group, and R³ represents a carboxyl group, or a group represented by -C (=R⁹)-R¹⁰;
(2-4) R² represents a hydrogen atom, or a hydroxyl group, and R³ represents a carboxyl group, or a group represented by -C (=R⁹)-R¹⁰;
(2-5) one of R² and R³ represents a hydrogen atom, a hydroxyl group, an optionally substituted C1-6 alkyl group, or an optionally substituted C6-10 aryl group, and the other represents a group represented by -C (=R⁹)-R¹⁰ (with the proviso that the group represented by -C (=R⁹)-R¹⁰ is not a carboxyl group, a methylcarbonyl group or a methoxycarbonyl group);
(2-6) one of R² and R³ represents a hydrogen atom, a hydroxyl group, or an optionally substituted C1-6 alkyl group, and the other represents a group represented by -C (=R⁹)-R¹⁰ (with the proviso that the group represented by -C (=R⁹)-R¹⁰ is not a carboxyl group, a methylcarbonyl group or a methoxycarbonyl group);
(2-7) R² represents a hydrogen atom, or a C1-6 alkyl group, and R³ represents a group represented by -C (=R⁹)-R¹⁰ (with the proviso that the group represented by -C (=R⁹)-R¹⁰ is not a carboxyl group, a methylcarbonyl group or a methoxycarbonyl group);
(2-8) R² represents a hydrogen atom, and R³ represents a group represented by -C (=R⁹)-R¹⁰ (with the proviso that the group represented by -C (=R⁹)-R¹⁰ is not a carboxyl group, a methylcarbonyl group or a methoxycarbonyl group); or,
(2-9) R² represents a hydrogen atom, and R³ represents a formyl group, a 2-carboxyethyl aminocarbonyl group, a t- butoxycarbonylmethylaminocarbonyl group, a methoxycarbonyl group, a (2-propenyloxy) iminomethyl group, a 1-(t-butoxycarbonyl)-2-(t-butoxy) ethylaminocarbonyl group, a 1-carbonyl-2-hydroxyethylaminoarbonyl group, or a 1-(t-butoxycarbonyl)-2-phenylethyl aminocarbonyl group.

In the compound represented by the general formula (I), R⁹ represents an oxygen atom, a sulfur atom, a = N-R¹¹ group (wherein R¹¹ represents a hydroxyl group, a C1-6 alkyl group, a C6-10 aryl group, a C1-6 alkoxy group, a C2-6 alkenyloxy group, a C6-10 aryloxy group), or a = CH-R¹² group (wherein R¹² represents a formyl group, a carboxyl group, a C1-6 alkyl group, a C6-10 aryl group, a C1-6 alkyl group, a C1-6 alkoxycarbonyl group, an aminocarbonyl group, a C1-6 alkylaminocarbonyl group.). Here, preferable R⁹ includes the following groups:
(3-1) an oxygen atom, a = N-R¹¹ group (wherein R¹¹ represents a hydroxyl group, a C1-6 alkoxy group, a C2-6 alkenyloxy group, a C6-10 aryloxy group), or a = CH-R¹² group (wherein R¹² represents a formyl group, a carboxyl group, a C1-6 alkoxycarbonyl group, an aminocarbonyl group, a C1-6 alkylaminocarbonyl group.)
(3-2) an oxygen atom, or a = N-R¹¹ group (wherein R¹¹ represents a hydroxyl group, a C1-6 alkoxy group, a C2-6 alkenyloxy group, a C6-10 aryloxy group);
(3-3) an oxygen atom, or a = N-R¹¹ group (wherein R¹¹ represents a hydroxyl group, a C1-4 alkoxy group, or a C2-4 alkenyloxy group);
(3-4) an oxygen atom, or a = N-R¹¹ group (wherein R¹¹ represents a C2-4 alkenyloxy group); or
(3-5) an oxygen atom.

In the compound represented by the general formula (I), R¹⁰ represents a hydrogen atom, an amino group, an optionally substituted C1-6 alkyl group, an optionally substituted C6-10 aryl group, an optionally substituted C1-6 alkoxy group, an optionally substituted C6-10 aryloxy group, an optionally substituted C1-6 alkylamino group, or an optionally substituted C1-6 alkoxycarbonyl C1-6 alkylamino group. Here, preferable R¹⁰ includes the following groups:
(4-1) a hydrogen atom, an amino group, a C1-6 alkyl group, a C1-6 alkoxy group, a C1-6 alkylamino group, or a C1-6 alkoxycarbonyl C1-6 alkylamino group;
(4-2) a hydrogen atom, a C1-6 alkyl group, a C1-6 alkoxy group, a C1-6 alkylamino group, or a C1-6 alkoxycarbonyl C1-6 alkylamino group;
(4-3) a hydrogen atom, a C1-4 alkyl group, a C1-4 alkoxy group, a C1-4 alkylamino group, or a C1-4 alkoxycarbonyl C1-4 alkylamino group;
(4-4) a hydrogen atom, a C1-4 alkyl group, or a C1-4 alkoxy group;
(4-5) a hydrogen atom, an amino group, an optionally substituted C1-6 alkyl group, an optionally substituted C1-6 alkoxy group, an optionally substituted C1-6 alkylamino group, or an optionally substituted C1-6 alkoxycarbonyl C1-6 alkylamino group;
(4-6) a hydrogen atom, a C1-6 alkyl group optionally substituted with 1 to 3 substituent(s), a C1-6 alkoxy group optionally substituted with 1 to 3 substituent(s),a C1-6 alkylamino group optionally substituted with 1 to 3 substituent(s), or a C1-6 alkoxycarbonyl C1-6 alkylamino group optionally substituted with 1 to 3 substituent(s);
(4-7) a hydrogen atom, a C1-4 alkyl group optionally substituted with 1 to 3 substituent(s), a C1-4 alkoxy group optionally substituted with 1 to 3 substituent(s),a C1-4 alkylamino group optionally substituted with 1 to 3 substituent(s), or a C1-4 alkoxycarbonyl C1-4 alkylamino group optionally substituted with 1 to 3 substituent(s);
(4-8) a hydrogen atom, a C1-6 alkylamino group optionally substituted with 1 to 2 substituent(s), or a C1-6 alkoxycarbonyl C1-6 alkylamino group optionally substituted with 1 to 2 substituent(s)
(4-9) a hydrogen atom, a C1-4 alkylamino group optionally substituted with 1 to 2 substituent(s), or a C1-4 alkoxycarbonyl C1-4 alkylamino group optionally substituted with 1 to 2 substituent(s) (wherein, the substituents may be selected from a hydroxyl group, a carboxyl group, a C1-6 alkoxy group, and a C6-10 aryl group);
(4-10) a hydrogen atom, a C1-4 alkylamino group optionally substituted with 1 to 2 substituent(s), or a C1-4 alkoxycarbonyl C1-4 alkylamino group optionally substituted with 1 to 2 substituent(s) (wherein, the substituents may be selected from a carboxyl group, a C1-6 alkoxy group, and a C6 aryl group);

Thus, as the compound represented by the general formula (I) of the present invention, the groups represented by R may be preferably a combination of the groups selected from the abovementioned (1-1) to (1-7), (2-1) to (2-8), (3-1) to (3-5), and (4-1) to (4-10), respectively, or a combination of the groups selected from the (1-1) to (1-4), (2-1) to (2-4), (3-1) to (3-4), and (4-1) to (4-4). More preferably the combination of R includes the following: (1-2), (2-2), (3-2), and (4-2); (1-4), (2 -3), (3-3), and (4-3); (1-3), (2-4), (3-3), and (4-3); (1-3), (2-3), (3-4), and (4-3); (1-3), (2-3), (3-3), and (4-4); (1-3), (2 -3), (3-3), and (4-3); (1-4), (2-4), (3-4), and (4-4).

In one embodiment, the compound represented by the general formula (I) of the present invention is a compound represented by the following formula (II).

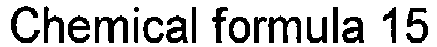

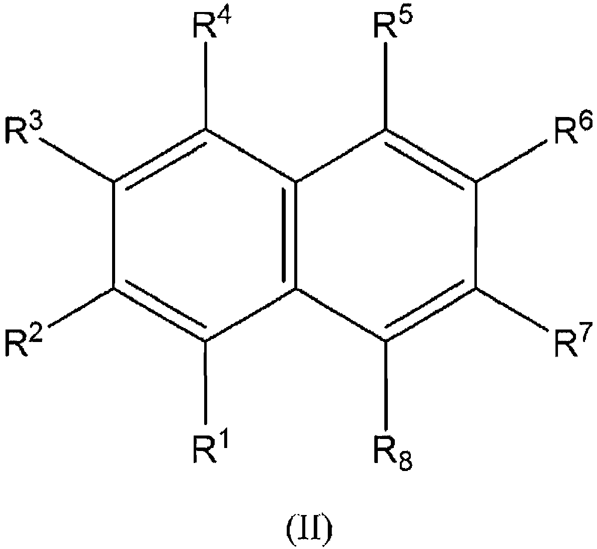

In the general formula (II), R¹-R⁴ are as defined in the general formula (I),
R⁵ and R⁸, which are the same or different, represent a hydroxyl group, an optionally substituted amino group, an optionally substituted C1-6 alkoxy group, an optionally substituted C2-40 alkenyl group, or a C2-7 alkanoyloxy group,
R⁶ and R⁷ represent a hydrogen atom, a hydroxyl group, an optionally substituted amino group, a halogen atom, an optionally substituted C1-6 alkyl group, an optionally substituted C2-40 alkenyl group, or a C1-6 alkoxy group,
wherein, in the above, the substituent in case being optionally substituted is a group selected from the following: a hydroxyl group, a carboxyl group, an amino group, a halogen atom, a C1-6 alkyl group, a C2-6 alkenyl group, a C2-7 alkanoyl group, a C1-6 alkoxycarbonyl group.

Wherein, R⁵ and R⁸, which are the same or different, preferably represent a hydroxyl group, an amino group, an optionally substituted C1-6 alkoxy group, or a C2-7 alkanoyloxy group, more preferably an optionally substituted C1-4 alkoxy group, yet more preferably a C1-3 alkoxy group (more preferably R⁵ and R⁸ both represent a C1-3 alkoxy group).

As preferable R⁶ and R⁷, R⁶ and R⁷, which are the same or different, represent a hydrogen atom, an optionally substituted amino group, or an optionally substituted C1-6 alkyl group, more preferably, a hydrogen atom, an optionally substituted amino group, or an optionally substituted C1-4 alkyl group, yet more preferably a hydrogen atom, an optionally substituted amino group, or an optionally substituted C1-3 alkyl group.

The compound represented by the general formula (II) preferably includes the following compounds:
(II-1) the compound, wherein R¹ and R⁴ represent the (1-1),
   R² and R³ represent the (2-1),
   R⁵ and R⁸, which are the same or different, represent a hydroxyl group, an amino group, an optionally substituted C1-6 alkoxy group, or a C2-7 alkanoyloxy group, and,
   R⁶ and R⁷, which are the same or different, represent a hydrogen atom, an optionally substituted amino group, or an optionally substituted C1-6 alkyl group;
(II-2) the compound, wherein R¹ and R⁴ represent the (1-2),
   R² and R³ represent the (2-2),
   R⁵ and R⁸, which are the same or different, represent a hydroxyl group, an amino group, an optionally substituted C1-6 alkoxy group, or a C2-7 alkanoyloxy group, and,
   R⁶ and R⁷, which are the same or different, represent a hydrogen atom, an optionally substituted amino group, or an optionally substituted C1-6 alkyl group;
(II-3) the compound, wherein R¹ and R⁴ represent the (1-3),
   R² and R³ represent the (2-3),
   R⁵ and R⁸, which are the same or different, represent and are optionally substituted C1-4 alkoxy group, and
   R⁶ and R⁷, which are the same or different, represent a hydrogen atom, an optionally substituted amino group, or a C1-3 alkyl group;
(II-4) the compound, wherein R¹ and R⁴ represent the (1-4),
   R² and R³ represent the (2-4),
   R⁵ and R⁸, which are the same or different, represent C1-3 alkoxy group and,
   R⁶ and R⁷, which are the same or different, represent a hydrogen atom, an optionally substituted amino group, or a C1-3 alkyl group;
(II-5) the compound, wherein R¹ and R⁴ represent the (1-5),
   R² and R³ represent the (2-5),
   R⁹ represents the (3-4), R¹⁰ represents the (4-5),
   R⁵ and R⁸, which are the same or different, represent a C1-6 alkoxy group, and
   R⁶ and R⁷, which are the same or different, represent a hydrogen atom, an optionally substituted amino group, or a C1-6 alkyl group;
(II-6) the compound, wherein R¹ and R⁴ represent the (1-6),
   R² and R³ represent the (2-8),
   R⁹ represents the (3-4), R¹⁰ represents the (4-6),
   R⁵ and R⁸, which are the same or different, represent a C1-3 alkoxy group and,
   R⁶ and R⁷, which are the same or different, represent a hydrogen atom, or a C1-6 alkyl group;
(II-7) the compound, wherein R¹ and R⁴ represent the (1-7),
   R² and R³ represent the (2-9),
   R⁹ represent the (3-5), R¹⁰ represent the (4-10),
   R⁵ and R⁸, which are the same or different, represent a C1-3 alkoxy group, and
   R⁶ and R⁷, which are the same or different, represent a hydrogen atom, or a C1-3 alkyl group.

In one embodiment, the compound represented by the general formula (I) of the present invention is a compound represented by the following general formula (III).

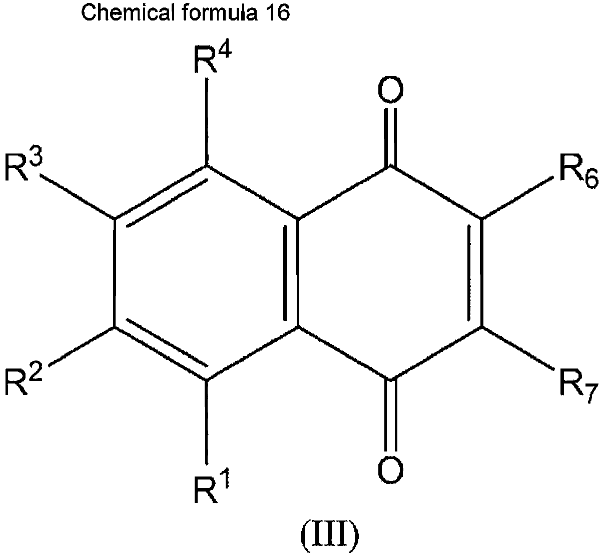

In the general formula (III), R¹-R⁴ are as defined in the general formula (I),
R⁶ and R⁷ represent a hydrogen atom, a hydroxyl group, an optionally substituted amino group, a halogen atom, an optionally substituted C1-6 alkyl group, an optionally substituted C2-40 alkenyl, an optionally substituted C1-6 alkoxy group,
here, in the above, the substituents in case being optionally substituted is a group selected from the following: a hydroxyl group, a carboxyl group, an amino group, a halogen atom, a C1-6 alkyl group, a C2-6 alkenyl group, a C2-7 alkanoyl group, a C1-6 alkoxycarbonyl group.

The compound represented by the general formula (III) preferably includes the following compounds:
(III-1) the compound, wherein R¹ and R⁴ represent the (1-1),
   R² and R³ represent the (2-1), and,
   R⁶ and R⁷, which are the same or different, represent a hydrogen atom, an optionally substituted amino group, or an optionally substituted C1-6 alkyl group;
(III-2) the compound, wherein R¹ and R⁴ represent the (1-2),
   R² and R³ represent the (2-2), and,
   R⁶ and R⁷, which are the same or different, represent a hydrogen atom, an optionally substituted amino group, or an optionally substituted C1-6 alkyl group;
(III-3) the compound, wherein R¹ and R⁴ represent the (1-3),
   R² and R³ represent the (2-3), and,
   R⁶ and R⁷, which are the same or different, represent a hydrogen atom, an optionally substituted amino group, or a C1-3 alkyl group;
(III-4) the compound, wherein R¹ and R⁴ represent the (1-4),
   R² and R³ represent the (2-4), and,
   R⁶ and R⁷, which are the same or different, represent a hydrogen atom, an optionally substituted amino group, or a C1-3 alkyl group;
(III-5) the compound, wherein R¹ and R⁴ represent the (1-5),
   R² and R³ represent the (2-5),
   R⁹ represents the (3-4), R¹⁰ represents the (4-5),
   R⁵ and R⁸, which are the same or different, represent a C1-6 alkoxy group, and
   R⁶ and R⁷, which are the same or different, represent a hydrogen atom, an optionally substituted amino group, or a C1-6 alkyl group;
(III-6) the compound, wherein R¹ and R⁴ represent the (1-6),
   R² and R³ represent the (2-8),
   R⁹ represents the (3-4), R¹⁰ represents the (4-6),
   R⁵ and R⁸, which are the same or different, represent a C1-3 alkoxy group, and
   R⁶ and R⁷, which are the same or different, represent a hydrogen atom, or a C1-6 alkyl group;
(III-7) the compound, wherein R¹ and R⁴ represent the (1-7),
   R² and R³ represent the (2-9),
   R⁹ represents the (3-5), R¹⁰ represents the (4-10),
   R⁵ and R⁸, which are the same or different, represent a C1-3 alkoxy group, and
   R⁶ and R⁷, which are the same or different, represent a hydrogen atom, or a C1-3 alkyl group;

In another embodiment, the compound represented by the general formula (I) of the present invention is a compound represented by the following general formula (IV) or (V).

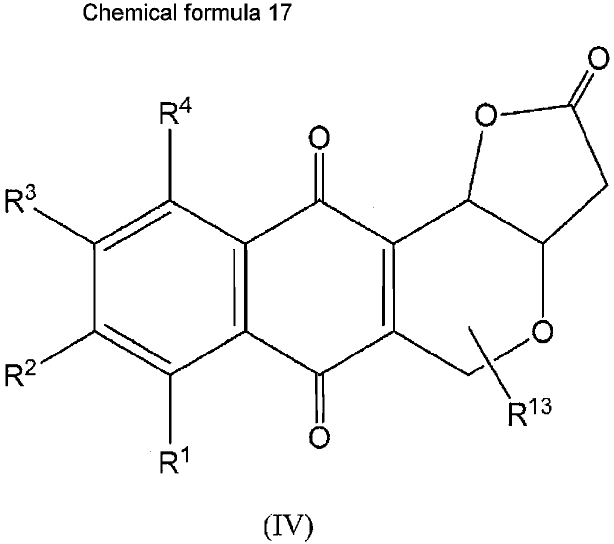

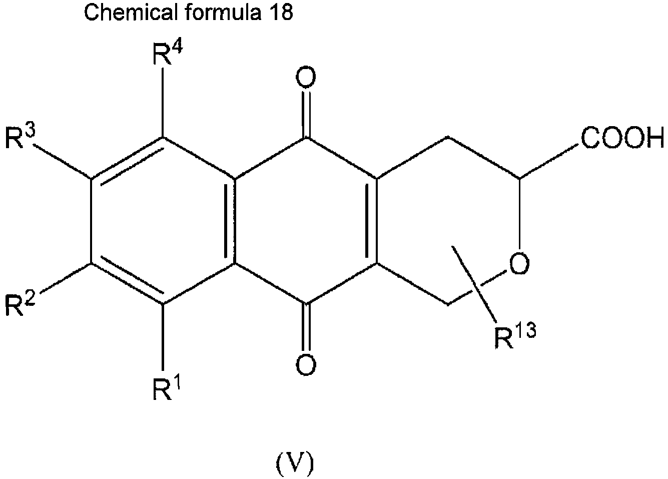

Among the compounds represented by the general formula (IV) or (V), particularly preferable compound is represented by the following general formula (IV') or (V').

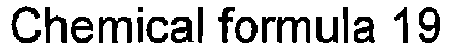

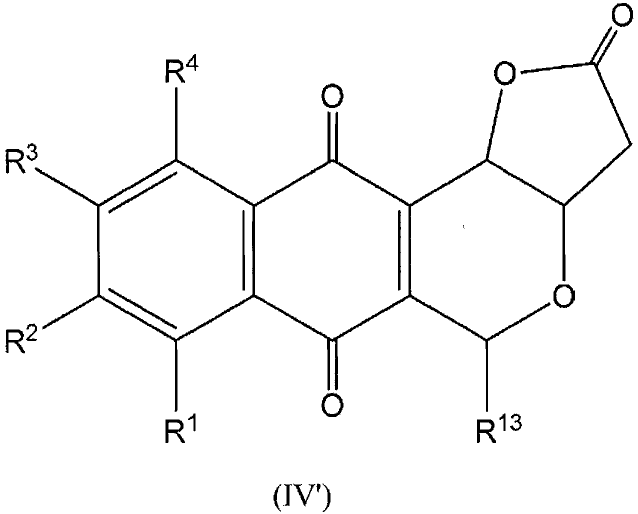

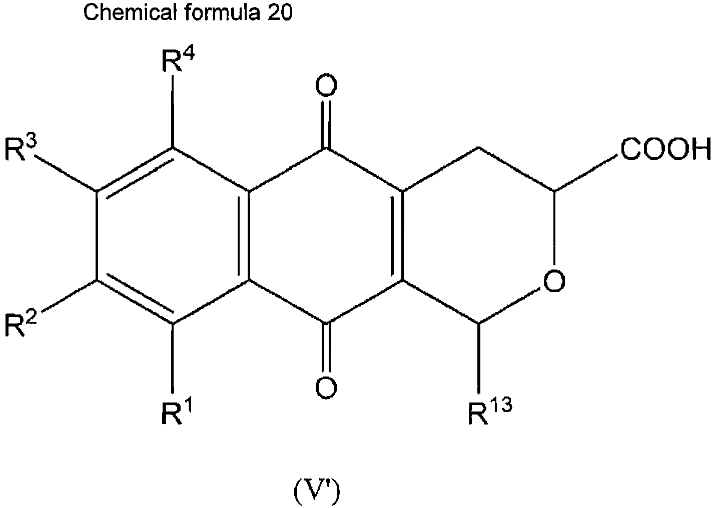

In the compound represented by the general formula (IV) or the general formula (V), (hereinafter, unless it is inconsistent, the compound represented by the general formula (IV') or the general formula (V') is included), R¹-R⁴ are as defined in the general formula (I), R¹³ represents an optionally substituted C1-6 alkyl group. R¹³ preferably includes a C1-6 alkyl group optionally substituted with a carboxyl group, more preferably a C1-4 alkyl group optionally substituted with a carboxyl group.

The compound represented by the general formula (IV) or (V) preferably includes the following compounds:
(IV-1) the compound, wherein R¹ and R⁴ represent the (1-1),
   R² and R³ represent the (2-1), and,
   R¹³ represents a C1-6 alkyl group optionally substituted with a carboxyl group;
(IV-2) the compound, wherein R¹ and R⁴ represent the (1-2),
   R² and R³ represent the (2-2), and,
   R¹³ represents a C1-6 alkyl group optionally substituted with a carboxyl group;
(IV-3) the compound, wherein R¹ and R⁴ represent the (1-3),
   R² and R³ represent the (2-3), and,
   R¹³ represents a C1-6 alkyl group optionally substituted with a carboxyl group;
(IV-4) the compound, wherein R¹ and R⁴ represent the (1-4),
   R² and R³ represent the (2-4), and,
   R¹³ represents a C1-4 alkyl group optionally substituted with a carboxyl group;

In yet another embodiment, the compound represented by the general formula (I) of the present invention is a compound represented by the following general formula (VI).

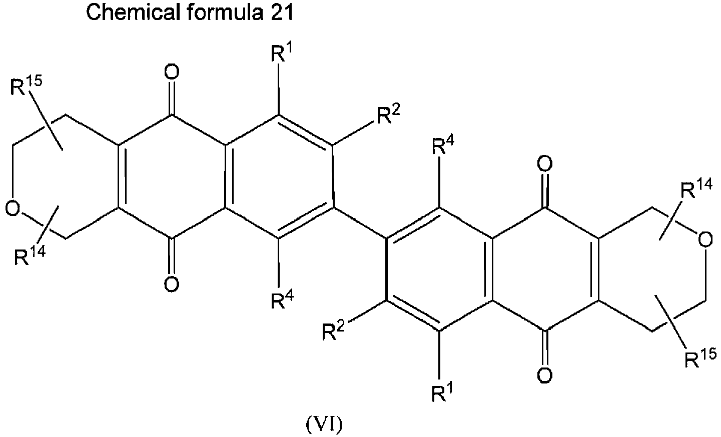

Among the compounds represented by the general formula (VI), particularly preferable compound is represented by the following general formula (VI').

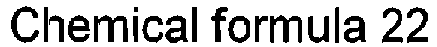

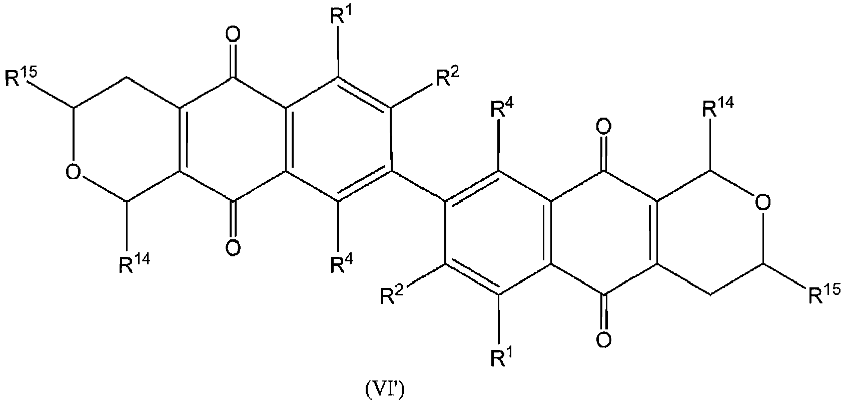

In the compound represented by the general formula (VI) (hereinafter, unless it is inconsistent, the compound represented by the general formula (VI') is included), R¹-R⁴ are as defined in the general formula (I), R¹⁴ and R¹⁵, which are the same or different, represent an optionally substituted C1-6 alkyl group. R¹⁴ preferably includes a C1-6 alkyl group, more preferably a C1-4 alkyl group, and yet more preferably a C1-3 alkyl group. R¹⁵ preferably includes a C1-6 alkyl group optionally substituted with a carboxyl group, more preferably a C1-4 alkyl group optionally substituted with a carboxyl group (eg. a C1-4 alkyl group substituted with a carboxyl group), and yet more preferably a C1-3 alkyl group optionally substituted with a carboxyl group (eg. a C1-3 alkyl group substituted with a carboxyl group).

The compound represented by the general formula (VI) preferably include the following compounds:
(IV-1) the compound, wherein R¹ and R⁴ represent the (1-1),
   R² and R³ represent the (2-1), and,
   R¹⁴ represents a C1-6 alkyl group, and
   R¹⁵ represents a C1-6 alkyl group optionally substituted with a carboxyl group;
(IV-2) the compound, wherein R¹ and R⁴ represent the (1-2),
   R² and R³ represent the (2-2), and,
   R¹⁴ represents a C1-6 alkyl group, and
   R¹⁵ represents a C1-6 alkyl group optionally substituted with a carboxyl group;
(IV-3) the compound, wherein R¹ and R⁴ represent the (1-3),
   R² and R³ represent the (2-3), and,
   R¹⁴ represents a C1-4 alkyl group, and
   R¹⁵ represents a C1-4 alkyl group optionally substituted with a carboxyl group;
(IV-4) the compound, wherein R¹ and R⁴ represent the (1-4),
   R² and R³ represent the (2-4), and,
   R¹⁴ represents a C1-3 alkyl group, and
   R¹⁵ represents a C1-3 alkyl group optionally substituted with a carboxyl group;

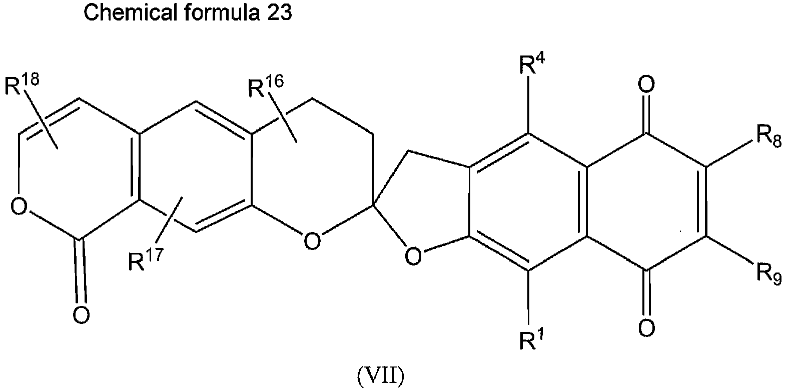

Among the compounds represented by the general formula (VII), particularly preferable compound is represented by the following general formula (VII').

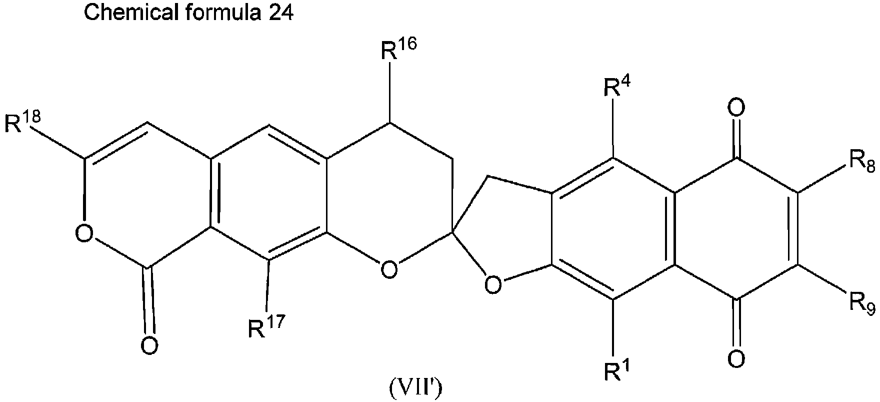

In the compound represented by the general formula (VII) (hereinafter, unless it is inconsistent, the compound represented by the general formula (VII') is included), R¹-R⁴ are as defined in the general formula (I), R¹⁶ , R¹⁷ and R¹⁸, which are the same or different, represent a hydroxyl group, or a C1-6 alkokycarbonyl group. R¹⁶, R¹⁷ and R¹⁸ preferably include a hydroxyl group as R¹⁶, a hydroxyl group as R¹⁷, and a C1-6 alkokycarbonyl group as R¹⁸. More preferably R¹⁶ represents a hydroxyl group, R¹⁷ represents a hydroxyl group, and R¹⁸ represents a C1-4 alkokycarbonyl group. Yet more preferably R¹⁶ represents a hydroxyl group, R¹⁷ represents a hydroxyl group, and R¹⁸ represents a C1-3 alkokycarbonyl group.

The compound represented by the general formula (VII) preferably include the following compounds:
(IV-1) the compound, wherein R¹ and R⁴ represent the (1-1),
   R² and R³ represent the (2-1), and,
   R¹⁶ represents a hydroxyl group,
   R¹⁷ represents a hydroxyl group, and
   R¹⁸ represents a C1-6 alkokycarbonyl group;
(IV-2) the compound, wherein R¹ and R⁴ represent the (1-2),
   R² and R³ represent the (2-2), and,
   R¹⁶ represents a hydroxyl group,
   R¹⁷ represents a hydroxyl group, and
   R¹⁸ represents a C1-6 alkokycarbonyl group;
(IV-3) the compound, wherein R¹ and R⁴ represent the (1-3),
   R² and R³ represent the (2-3), and,
   R¹⁶ represents a hydroxyl group,
   R¹⁷ represents a hydroxyl group, and
   R¹⁸ represents a C1-4 alkokycarbonyl group;
(IV-4) the compound, wherein R¹ and R⁴ represent the (1-4),
   R² and R³ represent the (2-4), and,
   R¹⁶ represents a hydroxyl group,
   R¹⁷ represents a hydroxyl group, and
   R¹⁸ represents a C1-3 alkokycarbonyl group.

Preferred embodiment of the compounds of the present invention in the general formula (II) is the compound, wherein R¹ to R⁸ represent the following groups.

**[Table 1]**

| Compou nd No. | R¹ | R² | R³ | R⁴ | R⁵ | R⁶ | R⁷ | R⁸ |
|---|---|---|---|---|---|---|---|---|
| II-1 | H | H | H | H | OH | H | H | OH |
| II-2 | H | COOH | H | H | OH | H | H | OH |
| II-3 | H | COOCH₃ | H | H | OH | H | H | OH |
| II-4 | H | H | H | H | NH₂ | H | H | OH |
| II-5 | H | COOH | H | H | NH₂ | H | H | OH |
| II-6 | H | COOCH₃ | H | H | NH₂ | H | H | OH |
| II-7 | H | H | H | H | OCH₃ | H | H | OH |
| II-8 | H | COOH | H | H | OCH₃ | H | H | OH |
| II-9 | H | COOCH₃ | H | H | OCH₃ | H | H | OH |
| II-10 | H | H | H | H | OCOCH₃ | H | H | OH |
| II-11 | H | COOH | H | H | OCOCH₃ | H | H | OH |
| II-12 | H | COOCH₃ | H | H | OCOCH₃ | H | H | OH |
| II-13 | H | H | H | H | OH | CH₃ | H | OH |
| II-14 | H | COOH | H | H | OH | CH₃ | H | OH |
| II-15 | H | COOCH₃ | H | H | OH | CH₃ | H | OH |
| II-16 | H | H | H | H | NH₂ | CH₃ | H | OH |
| II-17 | H | COOH | H | H | NH₂ | CH₃ | H | OH |
| II-18 | H | COOCH₃ | H | H | NH₂ | CH₃ | H | OH |
| II-19 | H | H | H | H | OCH₃ | CH₃ | H | OH |
| II-20 | H | COOH | H | H | OCH₃ | CH₃ | H | OH |
| II-21 | H | COOCH₃ | H | H | OCH₃ | CH₃ | H | OH |
| II-22 | H | H | H | H | OCOCH₃ | CH₃ | H | OH |
| II-23 | H | COOH | H | H | OCOCH₃ | CH₃ | H | OH |
| II-24 | H | COOCH₃ | H | H | OCOCH₃ | CH₃ | H | OH |
| II-25 | H | H | H | H | OH | H | H | NH₂ |
| II-26 | H | COOH | H | H | OH | H | H | NH₂ |
| II-27 | H | COOCH₃ | H | H | OH | H | H | NH₂ |
| II-28 | H | H | H | H | NH₂ | H | H | NH₂ |
| II-29 | H | COOH | H | H | NH₂ | H | H | NH₂ |
| II-30 | H | COOCH₃ | H | H | NH₂ | H | H | NH₂ |
| II-31 | H | H | H | H | OCH₃ | H | H | NH₂ |
| II-32 | H | COOH | H | H | OCH₃ | H | H | NH₂ |
| II-33 | H | COOCH₃ | H | H | OCH₃ | H | H | NH₂ |

**[Table 2]**

| Compou nd No. | R¹ | R² | R³ | R⁴ | R⁵ | R⁶ | R⁷ | R⁸ |
|---|---|---|---|---|---|---|---|---|
| II-34 | H | H | H | H | OCOCH₃ | H | H | NH₂ |
| II-35 | H | COOH | H | H | OCOCH₃ | H | H | NH₂ |
| II-36 | H | COOCH₃ | H | H | OCOCH₃ | H | H | NH₂ |
| II-37 | H | H | H | H | OH | CH₃ | H | NH₂ |
| II-38 | H | COOH | H | H | OH | CH₃ | H | NH₂ |
| II-39 | H | COOCH₃ | H | H | OH | CH₃ | H | NH₂ |
| II-40 | H | H | H | H | NH₂ | CH₃ | H | NH₂ |
| II-41 | H | COOH | H | H | NH₂ | CH₃ | H | NH₂ |
| II-42 | H | COOCH₃ | H | H | NH₂ | CH₃ | H | NH₂ |
| II-43 | H | H | H | H | OCH₃ | CH₃ | H | NH₂ |
| II-44 | H | COOH | H | H | OCH₃ | CH₃ | H | NH₂ |
| II-45 | H | COOCH₃ | H | H | OCH₃ | CH₃ | H | NH₂ |
| II-46 | H | H | H | H | OCOCH₃ | CH₃ | H | NH₂ |
| II-47 | H | COOH | H | H | OCOCH₃ | CH₃ | H | NH₂ |
| II-48 | H | COOCH₃ | H | H | OCOCH₃ | CH₃ | H | NH₂ |
| II-49 | H | H | H | H | OH | H | H | OCH₃ |
| II-50 | H | COOH | H | H | OH | H | H | OCH₃ |
| II-51 | H | COOCH₃ | H | H | OH | H | H | OCH₃ |
| II-52 | H | H | H | H | NH₂ | H | H | OCH₃ |
| II-53 | H | COOH | H | H | NH₂ | H | H | OCH₃ |
| II-54 | H | COOCH₃ | H | H | NH₂ | H | H | OCH₃ |
| II-55 | H | H | H | H | OCH₃ | H | H | OCH₃ |
| II-56 | H | COOH | H | H | OCH₃ | H | H | OCH₃ |
| II-57 | H | COOCH₃ | H | H | OCH₃ | H | H | OCH₃ |
| II-58 | H | H | H | H | OCOCH₃ | H | H | OCH₃ |
| II-59 | H | COOH | H | H | OCOCH₃ | H | H | OCH₃ |
| II-60 | H | COOCH₃ | H | H | OCOCH₃ | H | H | OCH₃ |
| II-61 | H | H | H | H | OH | CH₃ | H | OCH₃ |
| II-62 | H | COOH | H | H | OH | CH₃ | H | OCH₃ |
| II-63 | H | COOCH₃ | H | H | OH | CH₃ | H | OCH₃ |
| II-64 | H | H | H | H | NH₂ | CH₃ | H | OCH₃ |
| II-65 | H | COOH | H | H | NH₂ | CH₃ | H | OCH₃ |
| II-66 | H | COOCH₃ | H | H | NH₂ | CH₃ | H | OCH₃ |

**[Table 3]**

| Compound No. | R¹ | R² | R³ | R⁴ | R⁵ | R⁶ | R⁷ | R⁸ |
|---|---|---|---|---|---|---|---|---|
| II-67 | H | H | H | H | OCH₃ | CH₃ | H | OCH₃ |
| II-68 | H | COOH | H | H | OCH₃ | CH₃ | H | OCH₃ |
| II-69 | H | COOCH₃ | H | H | OCH₃ | CH₃ | H | OCH₃ |
| II-70 | H | H | H | H | OCOCH₃ | CH₃ | H | OCH₃ |
| II-71 | H | COOH | H | H | OCOCH₃ | CH₃ | H | OCH₃ |
| II-72 | H | COOCH₃ | H | H | OCOCH₃ | CH₃ | H | OCH₃ |
| II-73 | H | H | H | H | OH | H | H | OCOCH₃ |
| II-74 | H | COOH | H | H | OH | H | H | OCOCH₃ |
| II-75 | H | COOCH₃ | H | H | OH | H | H | OCOCH₃ |
| II-76 | H | H | H | H | NH₂ | H | H | OCOCH₃ |
| II-77 | H | COOH | H | H | NH₂ | H | H | OCOCH₃ |
| II-78 | H | COOCH₃ | H | H | NH₂ | H | H | OCOCH₃ |
| II-79 | H | H | H | H | OCH₃ | H | H | OCOCH₃ |
| II-80 | H | COOH | H | H | OCH₃ | H | H | OCOCH₃ |
| II-81 | H | COOCH₃ | H | H | OCH₃ | H | H | OCOCH₃ |
| II-82 | H | H | H | H | OCOCH₃ | H | H | OCOCH₃ |
| II-83 | H | COOH | H | H | OCOCH₃ | H | H | OCOCH₃ |
| II-84 | H | COOCH₃ | H | H | OCOCH₃ | H | H | OCOCH₃ |
| II-85 | H | H | H | H | OH | CH₃ | H | OCOCH₃ |
| II-86 | H | COOH | H | H | OH | CH₃ | H | OCOCH₃ |
| II-87 | H | COOCH₃ | H | H | OH | CH₃ | H | OCOCH₃ |
| II-88 | H | H | H | H | NH₂ | CH₃ | H | OCOCH₃ |
| II-89 | H | COOH | H | H | NH₂ | CH₃ | H | OCOCH₃ |
| II-90 | H | COOCH₃ | H | H | NH₂ | CH₃ | H | OCOCH₃ |
| II-91 | H | H | H | H | OCH₃ | CH₃ | H | OCOCH₃ |
| II-92 | H | COOH | H | H | OCH₃ | CH₃ | H | OCOCH₃ |
| II-93 | H | COOCH₃ | H | H | OCH₃ | CH₃ | H | OCOCH₃ |
| II-94 | H | H | H | H | OCOCH₃ | CH₃ | H | OCOCH₃ |
| II-95 | H | COOH | H | H | OCOCH₃ | CH₃ | H | OCOCH₃ |
| II-96 | H | COOCH₃ | H | H | OCOCH₃ | CH₃ | H | OCOCH₃ |

**[Table 4]**

| Compound No. | R¹ | R² | R³ | R⁴ | R⁵ | R⁶ | R⁷ | R⁸ |
|---|---|---|---|---|---|---|---|---|
| II-97 | H | C(O)NHCH(CH₂OH)COOH | CH₃ | CH₃ | OH | CH₃ | CH₃ | OH |
| II-98 | CH₃ | C(O)NHCH(CH₂OH)COOH | CH₂CH₃ | CH₃ | OH | CH₃ | CH₃ | OH |
| II-99 | H | C(O)NHCH(CH₂OH)COOH | H | CH₃ | OH | CH₃ | CH₃ | OH |
| II-100 | CH₃ | C(O)NHCH(CH₂OH)COOH | H | CH₃ | OH | CH₃ | CH₃ | OH |
| II-101 | H | C(O)NHCH(CH₂OH)COOH | CH₃ | H | OH | CH₃ | CH₃ | OH |
| II-102 | CH₃ | C(O)NHCH(CH₂OH)COOH | CH₂CH₃ | H | OH | CH₃ | CH₃ | OH |
| II-103 | H | C(O)NHCH(CH₂OH)COOH | H | H | OH | CH₃ | CH₃ | OH |
| II-104 | CH₃ | C(O)NHCH(CH₂OH)COOH | H | H | OH | CH₃ | CH₃ | OH |
| II-105 | H | C(O)NHCH(CH₂OH)COOH | CH₃ | CH₃ | OH | H | CH₃ | OH |
| II-106 | CH₃ | C(O)NHCH(CH₂OH)COOH | CH₂CH₃ | CH₃ | OH | H | CH₃ | OH |
| II-107 | H | C(O)NHCH(CH₂OH)COOH | H | CH₃ | OH | H | CH₃ | OH |
| II-108 | CH₃ | C(O)NHCH(CH₂OH)COOH | H | CH₃ | OH | H | CH₃ | OH |
| II-109 | H | C(O)NHCH(CH₂OH)COOH | CH₃ | H | OH | H | CH₃ | OH |
| II-110 | CH₃ | C(O)NHCH(CH₂OH)COOH | CH₂CH₃ | H | OH | H | CH₃ | OH |
| II-111 | H | C(O)NHCH(CH₂OH)COOH | H | H | OH | H | CH₃ | OH |
| II-112 | CH₃ | C(O)NHCH(CH₂OH)COOH | H | H | OH | H | CH₃ | OH |
| II-113 | H | C(O)NHCH(CH₂OH)COOH | CH₃ | CH₃ | OH | CH₃ | CH₃ | OH |
| II-114 | CH₃ | C(O)NHCH(CH₂OH)COOH | CH₂CH₃ | CH₃ | OH | CH₃ | CH₃ | OH |
| II-115 | H | C(O)NHCH(CH₂OH)COOH | H | CH₃ | OH | CH₃ | CH₃ | OH |
| II-116 | CH₃ | C(O)NHCH(CH₂OH)COOH | H | CH₃ | OH | CH₃ | CH₃ | OH |
| II-117 | H | C(O)NHCH(CH₂OH)COOH | CH₃ | H | OH | CH₃ | CH₃ | OH |
| II-118 | CH₃ | C(O)NHCH(CH₂OH)COOH | CH₂CH₃ | H | OH | CH₃ | CH₃ | OH |
| II-119 | H | C(O)NHCH(CH₂OH)COOH | H | H | OH | CH₃ | CH₃ | OH |
| II-120 | CH₃ | C(O)NHCH(CH₂OH)COOH | H | H | OH | CH₃ | CH₃ | OH |
| II-121 | H | C(O)NHCH(CH₂OH)COOH | CH₃ | CH₃ | OH | H | CH₃ | OH |
| II-122 | CH₃ | C(O)NHCH(CH₂OH)COOH | CH₂CH₃ | CH₃ | OH | H | CH₃ | OH |
| II-123 | H | C(O)NHCH(CH₂OH)COOH | H | CH₃ | OH | H | CH₃ | OH |
| II-124 | CH₃ | C(O)NHCH(CH₂OH)COOH | H | CH₃ | OH | H | CH₃ | OH |
| II-125 | H | C(O)NHCH(CH₂OH)COOH | CH₃ | H | OH | H | CH₃ | OH |
| II-126 | CH₃ | C(O)NHCH(CH₂OH)COOH | CH₂CH₃ | H | OH | H | CH₃ | OH |
| II-127 | H | C(O)NHCH(CH₂OH)COOH | H | H | OH | H | CH₃ | OH |
| II-128 | CH₃ | C(O)NHCH(CH₂OH)COOH | H | H | OH | H | CH₃ | OH |
| II-129 | H | CONHCH₂COOH | CH₃ | CH₃ | OH | CH₃ | CH₃ | OH |

**[Table 5]**

| Compound No. | R¹ | R² | R³ | R⁴ | R⁵ | R⁶ | R⁷ | R⁸ |
|---|---|---|---|---|---|---|---|---|
| II-130 | CH₃ | CONHCH₂COOH | CH₂CH₃ | CH₃ | OH | CH₃ | CH₃ | OH |
| II-131 | H | CONHCH₂COOH | H | CH₃ | OH | CH₃ | CH₃ | OH |
| II-132 | CH₃ | CONHCH₂COOH | H | CH₃ | OH | CH₃ | CH₃ | OH |
| II-133 | H | CONHCH₂COOH | CH₃ | H | OH | CH₃ | CH₃ | OH |
| II-134 | CH₃ | CONHCH₂COOH | CH₂CH₃ | H | OH | CH₃ | CH₃ | OH |
| II-135 | H | CONHCH₂COOH | H | H | OH | CH₃ | CH₃ | OH |
| II-136 | CH₃ | CONHCH₂COOH | H | H | OH | CH₃ | CH₃ | OH |
| II-137 | H | CONHCH₂COOH | CH₃ | CH₃ | OH | H | CH₃ | OH |
| II-138 | CH₃ | CONHCH₂COOH | CH₂CH₃ | CH₃ | OH | H | CH₃ | OH |
| II-139 | H | CONHCH₂COOH | H | CH₃ | OH | H | CH₃ | OH |
| II-140 | CH₃ | CONHCH₂COOH | H | CH₃ | OH | H | CH₃ | OH |
| II-141 | H | CONHCH₂COOH | CH₃ | H | OH | H | CH₃ | OH |
| II-142 | CH₃ | CONHCH₂COOH | CH₂CH₃ | H | OH | H | CH₃ | OH |
| II-143 | H | CONHCH₂COOH | H | H | OH | H | CH₃ | OH |
| II-144 | CH₃ | CONHCH₂COOH | H | H | OH | H | CH₃ | OH |
| II-145 | H | CONHCH₂COOH | CH₃ | CH₃ | OH | CH₃ | CH₃ | OH |
| II-146 | CH₃ | CONHCH₂COOH | CH₂CH₃ | CH₃ | OH | CH₃ | CH₃ | OH |
| II-147 | H | CONHCH₂COOH | H | CH₃ | OH | CH₃ | CH₃ | OH |
| II-148 | CH₃ | CONHCH₂COOH | H | CH₃ | OH | CH₃ | CH₃ | OH |
| II-149 | H | CONHCH₂COOH | CH₃ | H | OH | CH₃ | CH₃ | OH |
| II-150 | CH₃ | CONHCH₂COOH | CH₂CH₃ | H | OH | CH₃ | CH₃ | OH |
| II-151 | H | CONHCH₂COOH | H | H | OH | CH₃ | CH₃ | OH |
| II-152 | CH₃ | CONHCH₂COOH | H | H | OH | CH₃ | CH₃ | OH |
| II-153 | H | CONHCH₂COOH | CH₃ | CH₃ | OH | H | CH₃ | OH |
| II-154 | CH₃ | CONHCH₂COOH | CH₂CH₃ | CH₃ | OH | H | CH₃ | OH |
| II-155 | H | CONHCH₂COOH | H | CH₃ | OH | H | CH₃ | OH |
| II-156 | CH₃ | CONHCH₂COOH | H | CH₃ | OH | H | CH₃ | OH |
| II-157 | H | CONHCH₂COOH | CH₃ | H | OH | H | CH₃ | OH |
| II-158 | CH₃ | CONHCH₂COOH | CH₂CH₃ | H | OH | H | CH₃ | OH |
| II-159 | H | CONHCH₂COOH | H | H | OH | H | CH₃ | OH |
| II-160 | CH₃ | CONHCH₂COOH | H | H | OH | H | CH₃ | OH |
| II-161 | H | CHO | CH₃ | CH₃ | OH | CH₃ | CH₃ | OH |
| II-162 | CH₃ | CHO | CH₂CH₃ | CH₃ | OH | CH₃ | CH₃ | OH |

**[Table 6]**

| Compound No. | R¹ | R² | R³ | R⁴ | R⁵ | R⁶ | R⁷ | R⁸ |
|---|---|---|---|---|---|---|---|---|
| II-163 | H | CHO | H | CH₃ | OH | CH₃ | CH₃ | OH |
| II-164 | CH₃ | CHO | H | CH₃ | OH | CH₃ | CH₃ | OH |
| II-165 | H | CHO | CH₃ | H | OH | CH₃ | CH₃ | OH |
| II-166 | CH₃ | CHO | CH₂CH₃ | H | OH | CH₃ | CH₃ | OH |
| II-167 | H | CHO | H | H | OH | CH₃ | CH₃ | OH |
| II-168 | CH₃ | CHO | H | H | OH | CH₃ | CH₃ | OH |
| II-169 | H | CHO | CH₃ | CH₃ | OH | H | CH₃ | OH |
| II-170 | CH₃ | CHO | CH₂CH₃ | CH₃ | OH | H | CH₃ | OH |
| II-171 | H | CHO | H | CH₃ | OH | H | CH₃ | OH |
| II-172 | CH₃ | CHO | H | CH₃ | OH | H | CH₃ | OH |
| II-173 | H | CHO | CH₃ | H | OH | H | CH₃ | OH |
| II-174 | CH₃ | CHO | CH₂CH₃ | H | OH | H | CH₃ | OH |
| II-175 | H | CHO | H | H | OH | H | CH₃ | OH |
| II-176 | CH₃ | CHO | H | H | OH | H | CH₃ | OH |
| II-177 | H | CHO | CH₃ | CH₃ | OH | CH₃ | CH₃ | OH |
| II-178 | CH₃ | CHO | CH₂CH₃ | CH₃ | OH | CH₃ | CH₃ | OH |
| II-179 | H | CHO | H | CH₃ | OH | CH₃ | CH₃ | OH |
| II-180 | CH₃ | CHO | H | CH₃ | OH | CH₃ | CH₃ | OH |
| II-181 | H | CHO | CH₃ | H | OH | CH₃ | CH₃ | OH |
| II-182 | CH₃ | CHO | CH₂CH₃ | H | OH | CH₃ | CH₃ | OH |
| II-183 | H | CHO | H | H | OH | CH₃ | CH₃ | OH |
| II-184 | CH₃ | CHO | H | H | OH | CH₃ | CH₃ | OH |
| II-185 | H | CHO | CH₃ | CH₃ | OH | H | CH₃ | OH |
| II-186 | CH₃ | CHO | CH₂CH₃ | CH₃ | OH | H | CH₃ | OH |
| II-187 | H | CHO | H | CH₃ | OH | H | CH₃ | OH |
| II-188 | CH₃ | CHO | H | CH₃ | OH | H | CH₃ | OH |
| II-189 | H | CHO | CH₃ | H | OH | H | CH₃ | OH |
| II-190 | CH₃ | CHO | CH₂CH₃ | H | OH | H | CH₃ | OH |
| II-191 | H | CHO | H | H | OH | H | CH₃ | OH |
| II-192 | CH₃ | CHO | H | H | OH | H | CH₃ | OH |
| II-193 | H | CONHCH₂COOC(CH₃)₃ | CH₃ | CH₃ | OH | CH₃ | CH₃ | OH |
| II-194 | CH₃ | CONHCH₂COOC(CH₃)₃ | CH₂CH₃ | CH₃ | OH | CH₃ | CH₃ | OH |
| II-195 | H | CONHCH₂COOC(CH₃)₃ | H | CH₃ | OH | CH₃ | CH₃ | OH |

**[Table 7]**

| Compound No. | R¹ | R² | R³ | R⁴ | R⁵ | R⁶ | R⁷ | R⁸ |
|---|---|---|---|---|---|---|---|---|
| II-196 | CH₃ | CONHCH₂COOC(CH₃)₃ | H | CH₃ | OH | CH₃ | CH₃ | OH |
| II-197 | H | CONHCH₂COOC(CH₃)₃ | CH₃ | H | OH | CH₃ | CH₃ | OH |
| II-198 | CH₃ | CONHCH₂COOC(CH₃)₃ | CH₂CH₃ | H | OH | CH₃ | CH₃ | OH |
| II-199 | H | CONHCH₂COOC(CH₃)₃ | H | H | OH | CH₃ | CH₃ | OH |
| II-200 | CH₃ | CONHCH₂COOC(CH₃)₃ | H | H | OH | CH₃ | CH₃ | OH |
| II-201 | H | CONHCH₂COOC(CH₃)₃ | CH₃ | CH₃ | OH | H | CH₃ | OH |
| II-202 | CH₃ | CONHCH₂COOC(CH₃)₃ | CH₂CH₃ | CH₃ | OH | H | CH₃ | OH |
| II-203 | H | CONHCH₂COOC(CH₃)₃ | H | CH₃ | OH | H | CH₃ | OH |
| II-204 | CH₃ | CONHCH₂COOC(CH₃)₃ | H | CH₃ | OH | H | CH₃ | OH |
| II-205 | H | CONHCH₂COOC(CH₃)₃ | CH₃ | H | OH | H | CH₃ | OH |
| II-206 | CH₃ | CONHCH₂COOC(CH₃)₃ | CH₂CH₃ | H | OH | H | CH₃ | OH |
| II-207 | H | CONHCH₂COOC(CH₃)₃ | H | H | OH | H | CH₃ | OH |
| II-208 | CH₃ | CONHCH₂COOC(CH₃)₃ | H | H | OH | H | CH₃ | OH |
| II-209 | H | CONHCH₂COOC(CH₃)₃ | CH₃ | CH₃ | OH | CH₃ | CH₃ | OH |
| II-210 | CH₃ | CONHCH₂COOC(CH₃)₃ | CH₂CH₃ | CH₃ | OH | CH₃ | CH₃ | OH |
| II-211 | H | CONHCH₂COOC(CH₃)₃ | H | CH₃ | OH | CH₃ | CH₃ | OH |
| II-212 | CH₃ | CONHCH₂COOC(CH₃)₃ | H | CH₃ | OH | CH₃ | CH₃ | OH |
| II-213 | H | CONHCH₂COOC(CH₃)₃ | CH₃ | H | OH | CH₃ | CH₃ | OH |
| II-214 | CH₃ | CONHCH₂COOC(CH₃)₃ | CH₂CH₃ | H | OH | CH₃ | CH₃ | OH |
| II-215 | H | CONHCH₂COOC(CH₃)₃ | H | H | OH | CH₃ | CH₃ | OH |
| II-216 | CH₃ | CONHCH₂COOC(CH₃)₃ | H | H | OH | CH₃ | CH₃ | OH |
| II-217 | H | CONHCH₂COOC(CH₃)₃ | CH₃ | CH₃ | OH | H | CH₃ | OH |
| II-218 | CH₃ | CONHCH₂COOC(CH₃)₃ | CH₂CH₃ | CH₃ | OH | H | CH₃ | OH |
| II-219 | H | CONHCH₂COOC(CH₃)₃ | H | CH₃ | OH | H | CH₃ | OH |
| II-220 | CH₃ | CONHCH₂COOC(CH₃)₃ | H | CH₃ | OH | H | CH₃ | OH |
| II-221 | H | CONHCH₂COOC(CH₃)₃ | CH₃ | H | OH | H | CH₃ | OH |
| II-222 | CH₃ | CONHCH₂COOC(CH₃)₃ | CH₂CH₃ | H | OH | H | CH₃ | OH |
| II-223 | H | CONHCH₂COOC(CH₃)₃ | H | H | OH | H | CH₃ | OH |
| II-224 | CH₃ | CONHCH₂COOC(CH₃)₃ | H | H | OH | H | CH₃ | OH |
| II-225 | H | C(O)NHCH(CO₂tBu)CH₂OtBu | CH₃ | CH₃ | OH | CH₃ | CH₃ | OH |
| II-226 | CH₃ | C(O)NHCH(CO₂tBu)CH₂OtBu | CH₂CH₃ | CH₃ | OH | CH₃ | CH₃ | OH |
| II-227 | H | C(O)NHCH(CO₂tBu)CH₂OtBu | H | CH₃ | OH | CH₃ | CH₃ | OH |
| II-228 | CH₃ | C(O)NHCH(CO₂tBu)CH₂OtBu | H | CH₃ | OH | CH₃ | CH₃ | OH |

**[Table 8]**

| Compound No. | R¹ | R² | R³ | R⁴ | R⁵ | R⁶ | R⁷ | R⁸ |
|---|---|---|---|---|---|---|---|---|
| II-229 | H | C(O)NHCH(CO₂tBu)CH₂OtBu | CH₃ | H | OH | CH₃ | CH₃ | OH |
| II-230 | CH₃ | C(O)NHCH(CO₂tBu)CH₂OtBu | CH₂CH₃ | H | OH | CH₃ | CH₃ | OH |
| II-231 | H | C(O)NHCH(CO₂tBu)CH₂OtBu | H | H | OH | CH₃ | CH₃ | OH |
| II-232 | CH₃ | C(O)NHCH(CO₂tBu)CH₂OtBu | H | H | OH | CH₃ | CH₃ | OH |
| II-233 | H | C(O)NHCH(CO₂tBu)CH₂OtBu | CH₃ | CH₃ | OH | H | CH₃ | OH |
| II-234 | CH₃ | C(O)NHCH(CO₂tBu)CH₂OtBu | CH₂CH₃ | CH₃ | OH | H | CH₃ | OH |
| II-235 | H | C(O)NHCH(CO₂tBu)CH₂OtBu | H | CH₃ | OH | H | CH₃ | OH |
| II-236 | CH₃ | C(O)NHCH(CO₂tBu)CH₂OtBu | H | CH₃ | OH | H | CH₃ | OH |
| II-237 | H | C(O)NHCH(CO₂tBu)CH₂OtBu | CH₃ | H | OH | H | CH₃ | OH |
| II-238 | CH₃ | C(O)NHCH(CO₂tBu)CH₂OtBu | CH₂CH₃ | H | OH | H | CH₃ | OH |
| II-239 | H | C(O)NHCH(CO₂tBu)CH₂OtBu | H | H | OH | H | CH₃ | OH |
| II-240 | CH₃ | C(O)NHCH(CO₂tBu)CH₂OtBu | H | H | OH | H | CH₃ | OH |
| II-241 | H | C(O)NHCH(CO₂tBu)CH₂OtBu | CH₃ | CH₃ | OH | CH₃ | CH₃ | OH |
| II-242 | CH₃ | C(O)NHCH(CO₂tBu)CH₂OtBu | CH₂CH₃ | CH₃ | OH | CH₃ | CH₃ | OH |
| II-243 | H | C(O)NHCH(CO₂tBu)CH₂OtBu | H | CH₃ | OH | CH₃ | CH₃ | OH |
| II-244 | CH₃ | C(O)NHCH(CO₂tBu)CH₂OtBu | H | CH₃ | OH | CH₃ | CH₃ | OH |
| II-245 | H | C(O)NHCH(CO₂tBu)CH₂OtBu | CH₃ | H | OH | CH₃ | CH₃ | OH |
| II-246 | CH₃ | C(O)NHCH(CO₂tBu)CH₂OtBu | CH₂CH₃ | H | OH | CH₃ | CH₃ | OH |
| II-247 | H | C(O)NHCH(CO₂tBu)CH₂OtBu | H | H | OH | CH₃ | CH₃ | OH |
| II-248 | CH₃ | C(O)NHCH(CO₂tBu)CH₂OtBu | H | H | OH | CH₃ | CH₃ | OH |
| II-249 | H | C(O)NHCH(CO₂tBu)CH₂OtBu | CH₃ | CH₃ | OH | H | CH₃ | OH |
| II-250 | CH₃ | C(O)NHCH(CO₂tBu)CH₂OtBu | CH₂CH₃ | CH₃ | OH | H | CH₃ | OH |
| II-251 | H | C(O)NHCH(CO₂tBu)CH₂OtBu | H | CH₃ | OH | H | CH₃ | OH |
| II-252 | CH₃ | C(O)NHCH(CO₂tBu)CH₂OtBu | H | CH₃ | OH | H | CH₃ | OH |
| II-253 | H | C(O)NHCH(CO₂tBu)CH₂OtBu | CH₃ | H | OH | H | CH₃ | OH |
| II-254 | CH₃ | C(O)NHCH(CO₂tBu)CH₂OtBu | CH₂CH₃ | H | OH | H | CH₃ | OH |
| II-255 | H | C(O)NHCH(CO₂tBu)CH₂OtBu | H | H | OH | H | CH₃ | OH |
| II-256 | CH₃ | C(O)NHCH(CO₂tBu)CH₂OtBu | H | H | OH | H | CH₃ | OH |
| II-257 | H | C(O)NHCH(CO₂tBu)CH₂Ph | CH₃ | CH₃ | OH | CH₃ | CH₃ | OH |
| II-258 | CH₃ | C(O)NHCH(CO₂tBu)CH₂Ph | CH₂CH₃ | CH₃ | OH | CH₃ | CH₃ | OH |
| II-259 | H | C(O)NHCH(CO₂tBu)CH₂Ph | H | CH₃ | OH | CH₃ | CH₃ | OH |
| II-260 | CH₃ | C(O)NHCH(CO₂tBu)CH₂Ph | H | CH₃ | OH | CH₃ | CH₃ | OH |
| II-261 | H | C(O)NHCH(CO₂tBu)CH₂Ph | CH₃ | H | OH | CH₃ | CH₃ | OH |

**[Table 9]**

| Compound No. | R¹ | R² | R³ | R⁴ | R⁵ | R⁶ | R⁷ | R⁸ |
|---|---|---|---|---|---|---|---|---|
| II-262 | CH₃ | C(O)NHCH(CO₂tBu)CH₂Ph | CH₂CH₃ | H | OH | CH₃ | CH₃ | OH |
| II-263 | H | C(O)NHCH(CO₂tBu)CH₂Ph | H | H | OH | CH₃ | CH₃ | OH |
| II-264 | CH₃ | C(O)NHCH(CO₂tBu)CH₂Ph | H | H | OH | CH₃ | CH₃ | OH |
| II-265 | H | C(O)NHCH(CO₂tBu)CH₂Ph | CH₃ | CH₃ | OH | H | CH₃ | OH |
| II-266 | CH₃ | C(O)NHCH(CO₂tBu)CH₂Ph | CH₂CH₃ | CH₃ | OH | H | CH₃ | OH |
| II-267 | H | C(O)NHCH(CO₂tBu)CH₂Ph | H | CH₃ | OH | H | CH₃ | OH |
| II-268 | CH₃ | C(O)NHCH(CO₂tBu)CH₂Ph | H | CH₃ | OH | H | CH₃ | OH |
| II-269 | H | C(O)NHCH(CO₂tBu)CH₂Ph | CH₃ | H | OH | H | CH₃ | OH |
| II-270 | CH₃ | C(O)NHCH(CO₂tBu)CH₂Ph | CH₂CH₃ | H | OH | H | CH₃ | OH |
| II-271 | H | C(O)NHCH(CO₂tBu)CH₂Ph | H | H | OH | H | CH₃ | OH |
| II-272 | CH₃ | C(O)NHCH(CO₂tBu)CH₂Ph | H | H | OH | H | CH₃ | OH |
| II-273 | H | C(O)NHCH(CO₂tBu)CH₂Ph | CH₃ | CH₃ | OH | CH₃ | CH₃ | OH |
| II-274 | CH₃ | C(O)NHCH(CO₂tBu)CH₂Ph | CH₂CH₃ | CH₃ | OH | CH₃ | CH₃ | OH |
| II-275 | H | C(O)NHCH(CO₂tBu)CH₂Ph | H | CH₃ | OH | CH₃ | CH₃ | OH |
| II-276 | CH₃ | C(O)NHCH(CO₂tBu)CH₂Ph | H | CH₃ | OH | CH₃ | CH₃ | OH |
| II-277 | H | C(O)NHCH(CO₂tBu)CH₂Ph | CH₃ | H | OH | CH₃ | CH₃ | OH |
| II-278 | CH₃ | C(O)NHCH(CO₂tBu)CH₂Ph | CH₂CH₃ | H | OH | CH₃ | CH₃ | OH |
| II-279 | H | C(O)NHCH(CO₂tBu)CH₂Ph | H | H | OH | CH₃ | CH₃ | OH |
| II-280 | CH₃ | C(O)NHCH(CO₂tBu)CH₂Ph | H | H | OH | CH₃ | CH₃ | OH |
| II-281 | H | C(O)NHCH(CO₂tBu)CH₂Ph | CH₃ | CH₃ | OH | H | CH₃ | OH |
| II-282 | CH₃ | C(O)NHCH(CO₂tBu)CH₂Ph | CH₂CH₃ | CH₃ | OH | H | CH₃ | OH |
| II-283 | H | C(O)NHCH(CO₂tBu)CH₂Ph | H | CH₃ | OH | H | CH₃ | OH |
| II-284 | CH₃ | C(O)NHCH(CO₂tBu)CH₂Ph | H | CH₃ | OH | H | CH₃ | OH |
| II-285 | H | C(O)NHCH(CO₂tBu)CH₂Ph | CH₃ | H | OH | H | CH₃ | OH |
| II-286 | CH₃ | C(O)NHCH(CO₂tBu)CH₂Ph | CH₂CH₃ | H | OH | H | CH₃ | OH |
| II-287 | H | C(O)NHCH(CO₂tBu)CH₂Ph | H | H | OH | H | CH₃ | OH |
| II-288 | CH₃ | C(O)NHCH(CO₂tBu)CH₂Ph | H | H | OH | H | CH₃ | OH |

Preferred embodiment of the compounds of the present invention in the general formula (III) is the compound, wherein R¹ to R⁴, R⁶, and R⁷ represent the following groups.

**[Table 10]**

| Compound No. | R¹ | R² | R³ | R⁴ | R⁶ | R⁷ |
|---|---|---|---|---|---|---|
| III-1 | H | H | H | H | H | H |
| III-2 | OH | H | H | H | H | H |
| III-3 | H | CHO | H | H | H | H |
| III-4 | OH | CHO | H | H | H | H |
| III-5 | H | COOH | H | H | H | H |
| III-6 | OH | COOH | H | H | H | H |
| III-7 | H | CONHCH₂COOH | H | H | H | H |
| III-8 | OH | CONHCH₂COOH | H | H | H | H |
| III-9 | H | H | COOCH₃ | H | H | H |
| III-10 | OH | H | COOCH₃ | H | H | H |
| III-11 | H | CHO | COOCH₃ | H | H | H |
| III-12 | OH | CHO | COOCH₃ | H | H | H |
| III-13 | H | COOH | COOCH₃ | H | H | H |
| III-14 | OH | COOH | COOCH₃ | H | H | H |
| III-15 | H | CONHCH₂COOH | COOCH₃ | H | H | H |
| III-16 | OH | CONHCH₂COOH | COOCH₃ | H | H | H |
| III-17 | H | H | COOH | H | H | H |
| III-18 | OH | H | COOH | H | H | H |
| III-19 | H | CHO | COOH | H | H | H |
| III-20 | OH | CHO | COOH | H | H | H |
| III-21 | H | COOH | COOH | H | H | H |
| III-22 | OH | COOH | COOH | H | H | H |
| III-23 | H | CONHCH₂COOH | COOH | H | H | H |
| III-24 | OH | CONHCH₂COOH | COOH | H | H | H |
| III-25 | H | H | H | OH | H | H |
| III-26 | OH | H | H | OH | H | H |
| III-27 | H | CHO | H | OH | H | H |
| III-28 | OH | CHO | H | OH | H | H |
| III-29 | H | COOH | H | OH | H | H |
| III-30 | OH | COOH | H | OH | H | H |
| III-31 | H | CONHCH₂COOH | H | OH | H | H |
| III-32 | OH | CONHCH₂COOH | H | OH | H | H |
| III-33 | H | H | COOCH₃ | OH | H | H |

**[Table 11]**

| Compound No. | R¹ | R² | R³ | R⁴ | R⁶ | R⁷ |
|---|---|---|---|---|---|---|
| III-34 | OH | H | COOCH₃ | OH | H | H |
| III-35 | H | CHO | COOCH₃ | OH | H | H |
| III-36 | OH | CHO | COOCH₃ | OH | H | H |
| III-37 | H | COOH | COOCH₃ | OH | H | H |
| III-38 | OH | COOH | COOCH₃ | OH | H | H |
| III-39 | H | CONHCH₂COOH | COOCH₃ | OH | H | H |
| III-40 | OH | CONHCH₂COOH | COOCH₃ | OH | H | H |
| III-41 | H | H | COOH | OH | H | H |
| III-42 | OH | H | COOH | OH | H | H |
| III-43 | H | CHO | COOH | OH | H | H |
| III-44 | OH | CHO | COOH | OH | H | H |
| III-45 | H | COOH | COOH | OH | H | H |
| III-46 | OH | COOH | COOH | OH | H | H |
| III-47 | H | CONHCH₂COOH | COOH | OH | H | H |
| III-48 | OH | CONHCH₂COOH | COOH | OH | H | H |
| III-49 | H | H | H | H | CH₃ | H |
| III-50 | OH | H | H | H | CH₃ | H |
| III-51 | H | CHO | H | H | CH₃ | H |
| III-52 | OH | CHO | H | H | CH₃ | H |
| III-53 | H | COOH | H | H | CH₃ | H |
| III-54 | OH | COOH | H | H | CH₃ | H |
| III-55 | H | CONHCH₂COOH | H | H | CH₃ | H |
| III-56 | OH | CONHCH₂COOH | H | H | CH₃ | H |
| III-57 | H | H | COOCH₃ | H | CH₃ | H |
| III-58 | OH | H | COOCH₃ | H | CH₃ | H |
| III-59 | H | CHO | COOCH₃ | H | CH₃ | H |
| III-60 | OH | CHO | COOCH₃ | H | CH₃ | H |
| III-61 | H | COOH | COOCH₃ | H | CH₃ | H |
| III-62 | OH | COOH | COOCH₃ | H | CH₃ | H |
| III-63 | H | CONHCH₂COOH | COOCH₃ | H | CH₃ | H |
| III-64 | OH | CONHCH₂COOH | COOCH₃ | H | CH₃ | H |
| III-65 | H | H | COOH | H | CH₃ | H |
| III-66 | OH | H | COOH | H | CH₃ | H |
| III-67 | H | CHO | COOH | H | CH₃ | H |

**[Table 12]**

| Compound No. | R¹ | R² | R³ | R⁴ | R⁶ | R⁷ |
|---|---|---|---|---|---|---|
| III-68 | OH | CHO | COOH | H | CH₃ | H |
| III-69 | H | COOH | COOH | H | CH₃ | H |
| III-70 | OH | COOH | COOH | H | CH₃ | H |
| III-71 | H | CONHCH₂COOH | COOH | H | CH₃ | H |
| III-72 | OH | CONHCH₂COOH | COOH | H | CH₃ | H |
| III-73 | H | H | H | OH | CH₃ | H |
| III-74 | OH | H | H | OH | CH₃ | H |
| III-75 | H | CHO | H | OH | CH₃ | H |
| III-76 | OH | CHO | H | OH | CH₃ | H |
| III-77 | H | COOH | H | OH | CH₃ | H |
| III-78 | OH | COOH | H | OH | CH₃ | H |
| III-79 | H | CONHCH₂COOH | H | OH | CH₃ | H |
| III-80 | OH | CONHCH₂COOH | H | OH | CH₃ | H |
| III-81 | H | H | COOCH₃ | OH | CH₃ | H |
| III-82 | OH | H | COOCH₃ | OH | CH₃ | H |
| III-83 | H | CHO | COOCH₃ | OH | CH₃ | H |
| III-84 | OH | CHO | COOCH₃ | OH | CH₃ | H |
| III-85 | H | COOH | COOCH₃ | OH | CH₃ | H |
| III-86 | OH | COOH | COOCH₃ | OH | CH₃ | H |
| III-87 | H | CONHCH₂COOH | COOCH₃ | OH | CH₃ | H |
| III-88 | OH | CONHCH₂COOH | COOCH₃ | OH | CH₃ | H |
| III-89 | H | H | COOH | OH | CH₃ | H |
| III-90 | OH | H | COOH | OH | CH₃ | H |
| III-91 | H | CHO | COOH | OH | CH₃ | H |
| III-92 | OH | CHO | COOH | OH | CH₃ | H |
| III-93 | H | COOH | COOH | OH | CH₃ | H |
| III-94 | OH | COOH | COOH | OH | CH₃ | H |
| III-95 | H | CONHCH₂COOH | COOH | OH | CH₃ | H |
| III-96 | OH | CONHCH₂COOH | COOH | OH | CH₃ | H |
| III-97 | H | H | H | H | Cl | H |
| III-98 | OH | H | H | H | Cl | H |
| III-99 | H | CHO | H | H | Cl | H |
| III-100 | OH | CHO | H | H | Cl | H |
| III-101 | H | COOH | H | H | Cl | H |

**[Table 13]**

| Compound No. | R¹ | R² | R³ | R⁴ | R⁶ | R⁷ |
|---|---|---|---|---|---|---|
| III-102 | OH | COOH | H | H | Cl | H |
| III-103 | H | CONHCH₂COOH | H | H | Cl | H |
| III-104 | OH | CONHCH₂COOH | H | H | Cl | H |
| III-105 | H | H | COOCH₃ | H | Cl | H |
| III-106 | OH | H | COOCH₃ | H | Cl | H |
| III-107 | H | CHO | COOCH₃ | H | Cl | H |
| III-108 | OH | CHO | COOCH₃ | H | Cl | H |
| III-109 | H | COOH | COOCH₃ | H | Cl | H |
| III-110 | OH | COOH | COOCH₃ | H | Cl | H |
| III-111 | H | CONHCH₂COOH | COOCH₃ | H | Cl | H |
| III-112 | OH | CONHCH₂COOH | COOCH₃ | H | Cl | H |
| III-113 | H | H | COOH | H | Cl | H |
| III-114 | OH | H | COOH | H | Cl | H |
| III-115 | H | CHO | COOH | H | Cl | H |
| III-116 | OH | CHO | COOH | H | Cl | H |
| III-117 | H | COOH | COOH | H | Cl | H |
| III-118 | OH | COOH | COOH | H | Cl | H |
| III-119 | H | CONHCH₂COOH | COOH | H | Cl | H |
| III-120 | OH | CONHCH₂COOH | COOH | H | Cl | H |
| III-121 | H | H | H | OH | Cl | H |
| III-122 | OH | H | H | OH | Cl | H |
| III-123 | H | CHO | H | OH | Cl | H |
| III-124 | OH | CHO | H | OH | Cl | H |
| III-125 | H | COOH | H | OH | Cl | H |
| III-126 | OH | COOH | H | OH | Cl | H |
| III-127 | H | CONHCH₂COOH | H | OH | Cl | H |
| III-128 | OH | CONHCH₂COOH | H | OH | Cl | H |
| III-129 | H | H | COOCH₃ | OH | Cl | H |
| III-130 | OH | H | COOCH₃ | OH | Cl | H |
| III-131 | H | CHO | COOCH₃ | OH | Cl | H |
| III-132 | OH | CHO | COOCH₃ | OH | Cl | H |
| III-133 | H | COOH | COOCH₃ | OH | Cl | H |
| III-134 | OH | COOH | COOCH₃ | OH | Cl | H |
| III-135 | H | CONHCH₂COOH | COOCH₃ | OH | Cl | H |

**[Table 14]**

| Compound No. | R¹ | R² | R³ | R⁴ | R⁶ | R⁷ |
|---|---|---|---|---|---|---|
| III-136 | OH | CONHCH₂COOH | COOCH₃ | OH | Cl | H |
| III-137 | H | H | COOH | OH | Cl | H |
| III-138 | OH | H | COOH | OH | Cl | H |
| III-139 | H | CHO | COOH | OH | Cl | H |
| III-140 | OH | CHO | COOH | OH | Cl | H |
| III-141 | H | COOH | COOH | OH | Cl | H |
| III-142 | OH | COOH | COOH | OH | Cl | H |
| III-143 | H | CONHCH₂COOH | COOH | OH | Cl | H |
| III-144 | OH | CONHCH₂COOH | COOH | OH | Cl | H |
| III-145 | H | H | H | H | OCH₃ | H |
| III-146 | OH | H | H | H | OCH₃ | H |
| III-147 | H | CHO | H | H | OCH₃ | H |
| III-148 | OH | CHO | H | H | OCH₃ | H |
| III-149 | H | COOH | H | H | OCH₃ | H |
| III-150 | OH | COOH | H | H | OCH₃ | H |
| III-151 | H | CONHCH₂COOH | H | H | OCH₃ | H |
| III-152 | OH | CONHCH₂COOH | H | H | OCH₃ | H |
| III-153 | H | H | COOCH₃ | H | OCH₃ | H |
| III-154 | OH | H | COOCH₃ | H | OCH₃ | H |
| III-155 | H | CHO | COOCH₃ | H | OCH₃ | H |
| III-156 | OH | CHO | COOCH₃ | H | OCH₃ | H |
| III-157 | H | COOH | COOCH₃ | H | OCH₃ | H |
| III-158 | OH | COOH | COOCH₃ | H | OCH₃ | H |
| III-159 | H | CONHCH₂COOH | COOCH₃ | H | OCH₃ | H |
| III-160 | OH | CONHCH₂COOH | COOCH₃ | H | OCH₃ | H |
| III-161 | H | H | COOH | H | OCH₃ | H |
| III-162 | OH | H | COOH | H | OCH₃ | H |
| III-163 | H | CHO | COOH | H | OCH₃ | H |
| III-164 | OH | CHO | COOH | H | OCH₃ | H |
| III-165 | H | COOH | COOH | H | OCH₃ | H |
| III-166 | OH | COOH | COOH | H | OCH₃ | H |
| III-167 | H | CONHCH₂COOH | COOH | H | OCH₃ | H |
| III-168 | OH | CONHCH₂COOH | COOH | H | OCH₃ | H |
| III-169 | H | H | H | OH | OCH₃ | H |

**[Table 15]**

| Compound No. | R¹ | R² | R³ | R⁴ | R⁶ | R⁷ |
|---|---|---|---|---|---|---|
| III-170 | OH | H | H | OH | OCH₃ | H |
| III-171 | H | CHO | H | OH | OCH₃ | H |
| III-172 | OH | CHO | H | OH | OCH₃ | H |
| III-173 | H | COOH | H | OH | OCH₃ | H |
| III-174 | OH | COOH | H | OH | OCH₃ | H |
| III-175 | H | CONHCH₂COOH | H | OH | OCH₃ | H |
| III-176 | OH | CONHCH₂COOH | H | OH | OCH₃ | H |
| III-177 | H | H | COOCH₃ | OH | OCH₃ | H |
| 111-178 | OH | H | COOCH₃ | OH | OCH₃ | H |
| III-179 | H | CHO | COOCH₃ | OH | OCH₃ | H |
| III-180 | OH | CHO | COOCH₃ | OH | OCH₃ | H |
| III-181 | H | COOH | COOCH₃ | OH | OCH₃ | H |
| III-182 | OH | COOH | COOCH₃ | OH | OCH₃ | H |
| III-183 | H | CONHCH₂COOH | COOCH₃ | OH | OCH₃ | H |
| III-184 | OH | CONHCH₂COOH | COOCH₃ | OH | OCH₃ | H |
| III-185 | H | H | COOH | OH | OCH₃ | H |
| III-186 | OH | H | COOH | OH | OCH₃ | H |
| III-187 | H | CHO | COOH | OH | OCH₃ | H |
| III-188 | OH | CHO | COOH | OH | OCH₃ | H |
| III-189 | H | COOH | COOH | OH | OCH₃ | H |
| III-190 | OH | COOH | COOH | OH | OCH₃ | H |
| III-191 | H | CONHCH₂COOH | COOH | OH | OCH₃ | H |
| III-192 | OH | CONHCH₂COOH | COOH | OH | OCH₃ | H |
| III-193 | H | H | H | H | H | Cl |
| III-194 | OH | H | H | H | H | Cl |
| III-195 | H | CHO | H | H | H | Cl |
| III-196 | OH | CHO | H | H | H | Cl |
| III-197 | H | COOH | H | H | H | Cl |
| III-198 | OH | COOH | H | H | H | Cl |
| III-199 | H | CONHCH₂COOH | H | H | H | Cl |
| III-200 | OH | CONHCH₂COOH | H | H | H | Cl |
| III-201 | H | H | COOCH₃ | H | H | Cl |
| III-202 | OH | H | COOCH₃ | H | H | Cl |
| III-203 | H | CHO | COOCH₃ | H | H | Cl |

**[Table 16]**

| Compound No. | R¹ | R² | R³ | R⁴ | R⁶ | R⁷ |
|---|---|---|---|---|---|---|
| III-204 | OH | CHO | COOCH₃ | H | H | Cl |
| III-205 | H | COOH | COOCH₃ | H | H | Cl |
| III-206 | OH | COOH | COOCH₃ | H | H | Cl |
| III-207 | H | CONHCH₂COOH | COOCH₃ | H | H | Cl |
| III-208 | OH | CONHCH₂COOH | COOCH₃ | H | H | Cl |
| III-209 | H | H | COOH | H | H | Cl |
| III-210 | OH | H | COOH | H | H | Cl |
| III-211 | H | CHO | COOH | H | H | Cl |
| III-212 | OH | CHO | COOH | H | H | Cl |
| III-213 | H | COOH | COOH | H | H | Cl |
| III-214 | OH | COOH | COOH | H | H | Cl |
| III-215 | H | CONHCH₂COOH | COOH | H | H | Cl |
| III-216 | OH | CONHCH₂COOH | COOH | H | H | Cl |
| III-217 | H | H | H | OH | H | Cl |
| III-218 | OH | H | H | OH | H | Cl |
| III-219 | H | CHO | H | OH | H | Cl |
| III-220 | OH | CHO | H | OH | H | Cl |
| III-221 | H | COOH | H | OH | H | Cl |
| III-222 | OH | COOH | H | OH | H | Cl |
| III-223 | H | CONHCH₂COOH | H | OH | H | Cl |
| III-224 | OH | CONHCH₂COOH | H | OH | H | Cl |
| III-225 | H | H | COOCH₃ | OH | H | Cl |
| III-226 | OH | H | COOCH₃ | OH | H | Cl |
| III-227 | H | CHO | COOCH₃ | OH | H | Cl |
| III-228 | OH | CHO | COOCH₃ | OH | H | Cl |
| III-229 | H | COOH | COOCH₃ | OH | H | Cl |
| III-230 | OH | COOH | COOCH₃ | OH | H | Cl |
| III-231 | H | CONHCH₂COOH | COOCH₃ | OH | H | Cl |
| III-232 | OH | CONHCH₂COOH | COOCH₃ | OH | H | Cl |
| III-233 | H | H | COOH | OH | H | Cl |
| III-234 | OH | H | COOH | OH | H | Cl |
| III-235 | H | CHO | COOH | OH | H | Cl |
| III-236 | OH | CHO | COOH | OH | H | Cl |
| III-237 | H | COOH | COOH | OH | H | Cl |

**[Table 17]**

| Compound No. | R¹ | R² | R³ | R⁴ | R⁶ | R⁷ |
|---|---|---|---|---|---|---|
| III-238 | OH | COOH | COOH | OH | H | Cl |
| III-239 | H | CONHCH₂COOH | COOH | OH | H | Cl |
| III-240 | OH | CONHCH₂COOH | COOH | OH | H | Cl |
| III-241 | H | H | H | H | CH₃ | Cl |
| III-242 | OH | H | H | H | CH₃ | Cl |
| III-243 | H | CHO | H | H | CH₃ | Cl |
| III-244 | OH | CHO | H | H | CH₃ | Cl |
| III-245 | H | COOH | H | H | CH₃ | Cl |
| III-246 | OH | COOH | H | H | CH₃ | Cl |
| III-247 | H | CONHCH₂COOH | H | H | CH₃ | Cl |
| III-248 | OH | CONHCH₂COOH | H | H | CH₃ | Cl |
| III-249 | H | H | COOCH₃ | H | CH₃ | Cl |
| III-250 | OH | H | COOCH₃ | H | CH₃ | Cl |
| III-251 | H | CHO | COOCH₃ | H | CH₃ | Cl |
| III-252 | OH | CHO | COOCH₃ | H | CH₃ | Cl |
| III-253 | H | COOH | COOCH₃ | H | CH₃ | Cl |
| III-254 | OH | COOH | COOCH₃ | H | CH₃ | Cl |
| III-255 | H | CONHCH₂COOH | COOCH₃ | H | CH₃ | Cl |
| III-256 | OH | CONHCH₂COOH | COOCH₃ | H | CH₃ | Cl |
| III-257 | H | H | COOH | H | CH₃ | Cl |
| III-258 | OH | H | COOH | H | CH₃ | Cl |
| III-259 | H | CHO | COOH | H | CH₃ | Cl |
| III-260 | OH | CHO | COOH | H | CH₃ | Cl |
| III-261 | H | COOH | COOH | H | CH₃ | Cl |
| III-262 | OH | COOH | COOH | H | CH₃ | Cl |
| III-263 | H | CONHCH₂COOH | COOH | H | CH₃ | Cl |
| III-264 | OH | CONHCH₂COOH | COOH | H | CH₃ | Cl |
| III-265 | H | H | H | OH | CH₃ | Cl |
| III-266 | OH | H | H | OH | CH₃ | Cl |
| III-267 | H | CHO | H | OH | CH₃ | Cl |
| III-268 | OH | CHO | H | OH | CH₃ | Cl |
| III-269 | H | COOH | H | OH | CH₃ | Cl |
| III-270 | OH | COOH | H | OH | CH₃ | Cl |
| III-271 | H | CONHCH₂COOH | H | OH | CH₃ | Cl |

**[Table 18]**

| Compound No. | R¹ | R² | R³ | R⁴ | R⁶ | R⁷ |
|---|---|---|---|---|---|---|
| III-272 | OH | CONHCH₂COOH | H | OH | CH₃ | Cl |
| III-273 | H | H | COOCH₃ | OH | CH₃ | Cl |
| III-274 | OH | H | COOCH₃ | OH | CH₃ | Cl |
| III-275 | H | CHO | COOCH₃ | OH | CH₃ | Cl |
| III-276 | OH | CHO | COOCH₃ | OH | CH₃ | Cl |
| III-277 | H | COOH | COOCH₃ | OH | CH₃ | Cl |
| III-278 | OH | COOH | COOCH₃ | OH | CH₃ | Cl |
| III-279 | H | CONHCH₂COOH | COOCH₃ | OH | CH₃ | Cl |
| III-280 | OH | CONHCH₂COOH | COOCH₃ | OH | CH₃ | Cl |
| III-281 | H | H | COOH | OH | CH₃ | Cl |
| III-282 | OH | H | COOH | OH | CH₃ | Cl |
| III-283 | H | CHO | COOH | OH | CH₃ | Cl |
| III-284 | OH | CHO | COOH | OH | CH₃ | Cl |
| III-285 | H | COOH | COOH | OH | CH₃ | Cl |
| III-286 | OH | COOH | COOH | OH | CH₃ | Cl |
| III-287 | H | CONHCH₂COOH | COOH | OH | CH₃ | Cl |
| III-288 | OH | CONHCH₂COOH | COOH | OH | CH₃ | Cl |
| III-289 | H | H | H | H | Cl | Cl |
| III-290 | OH | H | H | H | Cl | Cl |
| III-291 | H | CHO | H | H | Cl | Cl |
| III-292 | OH | CHO | H | H | Cl | Cl |
| III-293 | H | COOH | H | H | Cl | Cl |
| III-294 | OH | COOH | H | H | Cl | Cl |
| III-295 | H | CONHCH₂COOH | H | H | Cl | Cl |
| III-296 | OH | CONHCH₂COOH | H | H | Cl | Cl |
| III-297 | H | H | COOCH₃ | H | Cl | Cl |
| III-298 | OH | H | COOCH₃ | H | Cl | Cl |
| III-299 | H | CHO | COOCH₃ | H | Cl | Cl |
| III-300 | OH | CHO | COOCH₃ | H | Cl | Cl |
| III-301 | H | COOH | COOCH₃ | H | Cl | Cl |
| III-302 | OH | COOH | COOCH₃ | H | Cl | Cl |
| III-303 | H | CONHCH₂COOH | COOCH₃ | H | Cl | Cl |
| III-304 | OH | CONHCH₂COOH | COOCH₃ | H | Cl | Cl |
| III-305 | H | H | COOH | H | Cl | Cl |

**[Table 19]**

| Compound No. | R¹ | R² | R³ | R⁴ | R⁶ | R⁷ |
|---|---|---|---|---|---|---|
| III-306 | OH | H | COOH | H | Cl | Cl |
| III-307 | H | CHO | COOH | H | Cl | Cl |
| III-308 | OH | CHO | COOH | H | Cl | Cl |
| III-309 | H | COOH | COOH | H | Cl | Cl |
| III-310 | OH | COOH | COOH | H | Cl | Cl |
| III-311 | H | CONHCH₂COOH | COOH | H | Cl | Cl |
| III-312 | OH | CONHCH₂COOH | COOH | H | Cl | Cl |
| III-313 | H | H | H | OH | Cl | Cl |
| III-314 | OH | H | H | OH | Cl | Cl |
| III-315 | H | CHO | H | OH | Cl | Cl |
| III-316 | OH | CHO | H | OH | Cl | Cl |
| III-317 | H | COOH | H | OH | Cl | Cl |
| III-318 | OH | COOH | H | OH | Cl | Cl |
| III-319 | H | CONHCH₂COOH | H | OH | Cl | Cl |
| III-320 | OH | CONHCH₂COOH | H | OH | Cl | Cl |
| III-321 | H | H | COOCH₃ | OH | Cl | Cl |
| III-322 | OH | H | COOCH₃ | OH | Cl | Cl |
| III-323 | H | CHO | COOCH₃ | OH | Cl | Cl |
| III-324 | OH | CHO | COOCH₃ | OH | Cl | Cl |
| III-325 | H | COOH | COOCH₃ | OH | Cl | Cl |
| III-326 | OH | COOH | COOCH₃ | OH | Cl | Cl |
| III-327 | H | CONHCH₂COOH | COOCH₃ | OH | Cl | Cl |
| III-328 | OH | CONHCH₂COOH | COOCH₃ | OH | Cl | Cl |
| III-329 | H | H | COOH | OH | Cl | Cl |
| III-330 | OH | H | COOH | OH | Cl | Cl |
| III-331 | H | CHO | COOH | OH | Cl | Cl |
| III-332 | OH | CHO | COOH | OH | Cl | Cl |
| III-333 | H | COOH | COOH | OH | Cl | Cl |
| III-334 | OH | COOH | COOH | OH | Cl | Cl |
| III-335 | H | CONHCH₂COOH | COOH | OH | Cl | Cl |
| III-336 | OH | CONHCH₂COOH | COOH | OH | Cl | Cl |
| III-337 | H | H | H | H | OCH₃ | Cl |
| III-338 | OH | H | H | H | OCH₃ | Cl |
| III-339 | H | CHO | H | H | OCH₃ | Cl |

**[Table 20]**

| Compound No. | R¹ | R² | R³ | R⁴ | R⁶ | R⁷ |
|---|---|---|---|---|---|---|
| III-340 | OH | CHO | H | H | OCH₃ | Cl |
| III-341 | H | COOH | H | H | OCH₃ | Cl |
| III-342 | OH | COOH | H | H | OCH₃ | Cl |
| III-343 | H | CONHCH₂COOH | H | H | OCH₃ | Cl |
| III-344 | OH | CONHCH₂COOH | H | H | OCH₃ | Cl |
| III-345 | H | H | COOCH₃ | H | OCH₃ | Cl |
| III-346 | OH | H | COOCH₃ | H | OCH₃ | Cl |
| III-347 | H | CHO | COOCH₃ | H | OCH₃ | Cl |
| III-348 | OH | CHO | COOCH₃ | H | OCH₃ | Cl |
| III-349 | H | COOH | COOCH₃ | H | OCH₃ | Cl |
| III-350 | OH | COOH | COOCH₃ | H | OCH₃ | Cl |
| III-351 | H | CONHCH₂COOH | COOCH₃ | H | OCH₃ | Cl |
| III-352 | OH | CONHCH₂COOH | COOCH₃ | H | OCH₃ | Cl |
| III-353 | H | H | COOH | H | OCH₃ | Cl |
| III-354 | OH | H | COOH | H | OCH₃ | Cl |
| III-355 | H | CHO | COOH | H | OCH₃ | Cl |
| III-356 | OH | CHO | COOH | H | OCH₃ | Cl |
| III-357 | H | COOH | COOH | H | OCH₃ | Cl |
| III-358 | OH | COOH | COOH | H | OCH₃ | Cl |
| III-359 | H | CONHCH₂COOH | COOH | H | OCH₃ | Cl |
| III-360 | OH | CONHCH₂COOH | COOH | H | OCH₃ | Cl |
| III-361 | H | H | H | OH | OCH₃ | Cl |
| III-362 | OH | H | H | OH | OCH₃ | Cl |
| III-363 | H | CHO | H | OH | OCH₃ | Cl |
| III-364 | OH | CHO | H | OH | OCH₃ | Cl |
| III-365 | H | COOH | H | OH | OCH₃ | Cl |
| III-366 | OH | COOH | H | OH | OCH₃ | Cl |
| III-367 | H | CONHCH₂COOH | H | OH | OCH₃ | Cl |
| III-368 | OH | CONHCH₂COOH | H | OH | OCH₃ | Cl |
| III-369 | H | H | COOCH₃ | OH | OCH₃ | Cl |
| III-370 | OH | H | COOCH₃ | OH | OCH₃ | Cl |
| III-371 | H | CHO | COOCH₃ | OH | OCH₃ | Cl |
| III-372 | OH | CHO | COOCH₃ | OH | OCH₃ | Cl |
| III-373 | H | COOH | COOCH₃ | OH | OCH₃ | Cl |

**[Table 21]**

| Compound No. | R¹ | R² | R³ | R⁴ | R⁶ | R⁷ |
|---|---|---|---|---|---|---|
| III-374 | OH | COOH | COOCH₃ | OH | OCH₃ | Cl |
| III-375 | H | CONHCH₂COOH | COOCH₃ | OH | OCH₃ | Cl |
| III-376 | OH | CONHCH₂COOH | COOCH₃ | OH | OCH₃ | Cl |
| III-377 | H | H | COOH | OH | OCH₃ | Cl |
| III-378 | OH | H | COOH | OH | OCH₃ | Cl |
| III-379 | H | CHO | COOH | OH | OCH₃ | Cl |
| III-380 | OH | CHO | COOH | OH | OCH₃ | Cl |
| III-381 | H | COOH | COOH | OH | OCH₃ | Cl |
| III-382 | OH | COOH | COOH | OH | OCH₃ | Cl |
| III-383 | H | CONHCH₂COOH | COOH | OH | OCH₃ | Cl |
| III-384 | OH | CONHCH₂COOH | COOH | OH | OCH₃ | Cl |
| III-385 | H | H | H | H | H | OCH₃ |
| III-386 | OH | H | H | H | H | OCH₃ |
| III-387 | H | CHO | H | H | H | OCH₃ |
| III-388 | OH | CHO | H | H | H | OCH₃ |
| III-389 | H | COOH | H | H | H | OCH₃ |
| III-390 | OH | COOH | H | H | H | OCH₃ |
| III-391 | H | CONHCH₂COOH | H | H | H | OCH₃ |
| III-392 | OH | CONHCH₂COOH | H | H | H | OCH₃ |
| III-393 | H | H | COOCH₃ | H | H | OCH₃ |
| III-394 | OH | H | COOCH₃ | H | H | OCH₃ |
| III-395 | H | CHO | COOCH₃ | H | H | OCH₃ |
| III-396 | OH | CHO | COOCH₃ | H | H | OCH₃ |
| III-397 | H | COOH | COOCH₃ | H | H | OCH₃ |
| III-398 | OH | COOH | COOCH₃ | H | H | OCH₃ |
| III-399 | H | CONHCH₂COOH | COOCH₃ | H | H | OCH₃ |
| III-400 | OH | CONHCH₂COOH | COOCH₃ | H | H | OCH₃ |
| III-401 | H | H | COOH | H | H | OCH₃ |
| III-402 | OH | H | COOH | H | H | OCH₃ |
| III-403 | H | CHO | COOH | H | H | OCH₃ |
| III-404 | OH | CHO | COOH | H | H | OCH₃ |
| III-405 | H | COOH | COOH | H | H | OCH₃ |
| III-406 | OH | COOH | COOH | H | H | OCH₃ |
| III-407 | H | CONHCH₂COOH | COOH | H | H | OCH₃ |

**[Table 22]**

| Compound No. | R¹ | R² | R³ | R⁴ | R⁶ | R⁷ |
|---|---|---|---|---|---|---|
| III-408 | OH | CONHCH₂COOH | COOH | H | H | OCH₃ |
| III-409 | H | H | H | OH | H | OCH₃ |
| III-410 | OH | H | H | OH | H | OCH₃ |
| III-411 | H | CHO | H | OH | H | OCH₃ |
| III-412 | OH | CHO | H | OH | H | OCH₃ |
| III-413 | H | COOH | H | OH | H | OCH₃ |
| III-414 | OH | COOH | H | OH | H | OCH₃ |
| III-415 | H | CONHCH₂COOH | H | OH | H | OCH₃ |
| III-416 | OH | CONHCH₂COOH | H | OH | H | OCH₃ |
| III-417 | H | H | COOCH₃ | OH | H | OCH₃ |
| III-418 | OH | H | COOCH₃ | OH | H | OCH₃ |
| III-419 | H | CHO | COOCH₃ | OH | H | OCH₃ |
| III-420 | OH | CHO | COOCH₃ | OH | H | OCH₃ |
| III-421 | H | COOH | COOCH₃ | OH | H | OCH₃ |
| III-422 | OH | COOH | COOCH₃ | OH | H | OCH₃ |
| III-423 | H | CONHCH₂COOH | COOCH₃ | OH | H | OCH₃ |
| III-424 | OH | CONHCH₂COOH | COOCH₃ | OH | H | OCH₃ |
| III-425 | H | H | COOH | OH | H | OCH₃ |
| III-426 | OH | H | COOH | OH | H | OCH₃ |
| III-427 | H | CHO | COOH | OH | H | OCH₃ |
| III-428 | OH | CHO | COOH | OH | H | OCH₃ |
| III-429 | H | COOH | COOH | OH | H | OCH₃ |
| III-430 | OH | COOH | COOH | OH | H | OCH₃ |
| III-431 | H | CONHCH₂COOH | COOH | OH | H | OCH₃ |
| III-432 | OH | CONHCH₂COOH | COOH | OH | H | OCH₃ |
| III-433 | H | H | H | H | CH₃ | OCH₃ |
| III-434 | OH | H | H | H | CH₃ | OCH₃ |
| III-435 | H | CHO | H | H | CH₃ | OCH₃ |
| III-436 | OH | CHO | H | H | CH₃ | OCH₃ |
| III-437 | H | COOH | H | H | CH₃ | OCH₃ |
| III-438 | OH | COOH | H | H | CH₃ | OCH₃ |
| III-439 | H | CONHCH₂COOH | H | H | CH₃ | OCH₃ |
| III-440 | OH | CONHCH₂COOH | H | H | CH₃ | OCH₃ |
| III-441 | H | H | COOCH₃ | H | CH₃ | OCH₃ |

**[Table 23]**

| Compound No. | R¹ | R² | R³ | R⁴ | R⁶ | R⁷ |
|---|---|---|---|---|---|---|
| III-442 | OH | H | COOCH₃ | H | CH₃ | OCH₃ |
| III-443 | H | CHO | COOCH₃ | H | CH₃ | OCH₃ |
| III-444 | OH | CHO | COOCH₃ | H | CH₃ | OCH₃ |
| III-445 | H | COOH | COOCH₃ | H | CH₃ | OCH₃ |
| III-446 | OH | COOH | COOCH₃ | H | CH₃ | OCH₃ |
| III-447 | H | CONHCH₂COOH | COOCH₃ | H | CH₃ | OCH₃ |
| III-448 | OH | CONHCH₂COOH | COOCH₃ | H | CH₃ | OCH₃ |
| III-449 | H | H | COOH | H | CH₃ | OCH₃ |
| III-450 | OH | H | COOH | H | CH₃ | OCH₃ |
| III-451 | H | CHO | COOH | H | CH₃ | OCH₃ |
| III-452 | OH | CHO | COOH | H | CH₃ | OCH₃ |
| III-453 | H | COOH | COOH | H | CH₃ | OCH₃ |
| III-454 | OH | COOH | COOH | H | CH₃ | OCH₃ |
| III-455 | H | CONHCH₂COOH | COOH | H | CH₃ | OCH₃ |
| III-456 | OH | CONHCH₂COOH | COOH | H | CH₃ | OCH₃ |
| III-457 | H | H | H | OH | CH₃ | OCH₃ |
| III-458 | OH | H | H | OH | CH₃ | OCH₃ |
| III-459 | H | CHO | H | OH | CH₃ | OCH₃ |
| III-460 | OH | CHO | H | OH | CH₃ | OCH₃ |
| III-461 | H | COOH | H | OH | CH₃ | OCH₃ |
| III-462 | OH | COOH | H | OH | CH₃ | OCH₃ |
| III-463 | H | CONHCH₂COOH | H | OH | CH₃ | OCH₃ |
| III-464 | OH | CONHCH₂COOH | H | OH | CH₃ | OCH₃ |
| III-465 | H | H | COOCH₃ | OH | CH₃ | OCH₃ |
| III-466 | OH | H | COOCH₃ | OH | CH₃ | OCH₃ |
| III-467 | H | CHO | COOCH₃ | OH | CH₃ | OCH₃ |
| III-468 | OH | CHO | COOCH₃ | OH | CH₃ | OCH₃ |
| III-469 | H | COOH | COOCH₃ | OH | CH₃ | OCH₃ |
| III-470 | OH | COOH | COOCH₃ | OH | CH₃ | OCH₃ |
| III-471 | H | CONHCH₂COOH | COOCH₃ | OH | CH₃ | OCH₃ |
| III-472 | OH | CONHCH₂COOH | COOCH₃ | OH | CH₃ | OCH₃ |
| III-473 | H | H | COOH | OH | CH₃ | OCH₃ |
| III-474 | OH | H | COOH | OH | CH₃ | OCH₃ |
| III-475 | H | CHO | COOH | OH | CH₃ | OCH₃ |

**[Table 24]**

| Compound No. | R¹ | R² | R³ | R⁴ | R⁶ | R⁷ |
|---|---|---|---|---|---|---|
| III-476 | OH | CHO | COOH | OH | CH₃ | OCH₃ |
| III-477 | H | COOH | COOH | OH | CH₃ | OCH₃ |
| III-478 | OH | COOH | COOH | OH | CH₃ | OCH₃ |
| III-479 | H | CONHCH₂COOH | COOH | OH | CH₃ | OCH₃ |
| III-480 | OH | CONHCH₂COOH | COOH | OH | CH₃ | OCH₃ |
| III-481 | H | H | H | H | Cl | OCH₃ |
| III-482 | OH | H | H | H | Cl | OCH₃ |
| III-483 | H | CHO | H | H | Cl | OCH₃ |
| III-484 | OH | CHO | H | H | Cl | OCH₃ |
| III-485 | H | COOH | H | H | Cl | OCH₃ |
| III-486 | OH | COOH | H | H | Cl | OCH₃ |
| III-487 | H | CONHCH₂COOH | H | H | Cl | OCH₃ |
| III-488 | OH | CONHCH₂COOH | H | H | Cl | OCH₃ |
| III-489 | H | H | COOCH₃ | H | Cl | OCH₃ |
| III-490 | OH | H | COOCH₃ | H | Cl | OCH₃ |
| III-491 | H | CHO | COOCH₃ | H | Cl | OCH₃ |
| III-492 | OH | CHO | COOCH₃ | H | Cl | OCH₃ |
| III-493 | H | COOH | COOCH₃ | H | Cl | OCH₃ |
| III-494 | OH | COOH | COOCH₃ | H | Cl | OCH₃ |
| III-495 | H | CONHCH₂COOH | COOCH₃ | H | Cl | OCH₃ |
| III-496 | OH | CONHCH₂COOH | COOCH₃ | H | Cl | OCH₃ |
| III-497 | H | H | COOH | H | Cl | OCH₃ |
| III-498 | OH | H | COOH | H | Cl | OCH₃ |
| III-499 | H | CHO | COOH | H | Cl | OCH₃ |
| III-500 | OH | CHO | COOH | H | Cl | OCH₃ |
| III-501 | H | COOH | COOH | H | Cl | OCH₃ |
| III-502 | OH | COOH | COOH | H | CI | OCH₃ |
| III-503 | H | CONHCH₂COOH | COOH | H | Cl | OCH₃ |
| III-504 | OH | CONHCH₂COOH | COOH | H | Cl | OCH₃ |
| III-505 | H | H | H | OH | Cl | OCH₃ |
| III-506 | OH | H | H | OH | Cl | OCH₃ |
| III-507 | H | CHO | H | OH | Cl | OCH₃ |
| III-508 | OH | CHO | H | OH | Cl | OCH₃ |
| III-509 | H | COOH | H | OH | Cl | OCH₃ |

**[Table 25]**

| Compound No. | R¹ | R² | R³ | R⁴ | R⁶ | R⁷ |
|---|---|---|---|---|---|---|
| III-510 | OH | COOH | H | OH | CI | OCH₃ |
| III-511 | H | CONHCH₂COOH | H | OH | Cl | OCH₃ |
| III-512 | OH | CONHCH₂COOH | H | OH | Cl | OCH₃ |
| III-513 | H | H | COOCH₃ | OH | Cl | OCH₃ |
| III-514 | OH | H | COOCH₃ | OH | Cl | OCH₃ |
| III-515 | H | CHO | COOCH₃ | OH | Cl | OCH₃ |
| III-516 | OH | CHO | COOCH₃ | OH | Cl | OCH₃ |
| III-517 | H | COOH | COOCH₃ | OH | Cl | OCH₃ |
| III-518 | OH | COOH | COOCH₃ | OH | Cl | OCH₃ |
| III-519 | H | CONHCH₂COOH | COOCH₃ | OH | Cl | OCH₃ |
| III-520 | OH | CONHCH₂COOH | COOCH₃ | OH | Cl | OCH₃ |
| III-521 | H | H | COOH | OH | Cl | OCH₃ |
| III-522 | OH | H | COOH | OH | Cl | OCH₃ |
| III-523 | H | CHO | COOH | OH | Cl | OCH₃ |
| III-524 | OH | CHO | COOH | OH | Cl | OCH₃ |
| III-525 | H | COOH | COOH | OH | Cl | OCH₃ |
| III-526 | OH | COOH | COOH | OH | Cl | OCH₃ |
| III-527 | H | CONHCH₂COOH | COOH | OH | Cl | OCH₃ |
| III-528 | OH | CONHCH₂COOH | COOH | OH | Cl | OCH₃ |
| III-529 | H | H | H | H | OCH₃ | OCH₃ |
| III-530 | OH | H | H | H | OCH₃ | OCH₃ |
| III-531 | H | CHO | H | H | OCH₃ | OCH₃ |
| III-532 | OH | CHO | H | H | OCH₃ | OCH₃ |
| III-533 | H | COOH | H | H | OCH₃ | OCH₃ |
| III-534 | OH | COOH | H | H | OCH₃ | OCH₃ |
| III-535 | H | CONHCH₂COOH | H | H | OCH₃ | OCH₃ |
| III-536 | OH | CONHCH₂COOH | H | H | OCH₃ | OCH₃ |
| III-537 | H | H | COOCH₃ | H | OCH₃ | OCH₃ |
| III-538 | OH | H | COOCH₃ | H | OCH₃ | OCH₃ |
| III-539 | H | CHO | COOCH₃ | H | OCH₃ | OCH₃ |
| III-540 | OH | CHO | COOCH₃ | H | OCH₃ | OCH₃ |
| III-541 | H | COOH | COOCH₃ | H | OCH₃ | OCH₃ |
| III-542 | OH | COOH | COOCH₃ | H | OCH₃ | OCH₃ |
| III-543 | H | CONHCH₂COOH | COOCH₃ | H | OCH₃ | OCH₃ |

**[Table26]**

| Compound No. | R¹ | R² | R³ | R⁴ | R⁶ | R⁷ |
|---|---|---|---|---|---|---|
| III-544 | OH | CONHCH₂COOH | COOCH₃ | H | OCH₃ | OCH₃ |
| III-545 | H | H | COOH | H | OCH₃ | OCH₃ |
| III-546 | OH | H | COOH | H | OCH₃ | OCH₃ |
| III-547 | H | CHO | COOH | H | OCH₃ | OCH₃ |
| III-548 | OH | CHO | COOH | H | OCH₃ | OCH₃ |
| III-549 | H | COOH | COOH | H | OCH₃ | OCH₃ |
| III-550 | OH | COOH | COOH | H | OCH₃ | OCH₃ |
| III-551 | H | CONHCH₂COOH | COOH | H | OCH₃ | OCH₃ |
| III-552 | OH | CONHCH₂COOH | COOH | H | OCH₃ | OCH₃ |
| III-553 | H | H | H | OH | OCH₃ | OCH₃ |
| III-554 | OH | H | H | OH | OCH₃ | OCH₃ |
| III-555 | H | CHO | H | OH | OCH₃ | OCH₃ |
| III-556 | OH | CHO | H | OH | OCH₃ | OCH₃ |
| III-557 | H | COOH | H | OH | OCH₃ | OCH₃ |
| III-558 | OH | COOH | H | OH | OCH₃ | OCH₃ |
| III-559 | H | CONHCH₂COOH | H | OH | OCH₃ | OCH₃ |
| III-560 | OH | CONHCH₂COOH | H | OH | OCH₃ | OCH₃ |
| III-561 | H | H | COOCH₃ | OH | OCH₃ | OCH₃ |
| III-562 | OH | H | COOCH₃ | OH | OCH₃ | OCH₃ |
| III-563 | H | CHO | COOCH₃ | OH | OCH₃ | OCH₃ |
| III-564 | OH | CHO | COOCH₃ | OH | OCH₃ | OCH₃ |
| III-565 | H | COOH | COOCH₃ | OH | OCH₃ | OCH₃ |
| III-566 | OH | COOH | COOCH₃ | OH | OCH₃ | OCH₃ |
| III-567 | H | CONHCH₂COOH | COOCH₃ | OH | OCH₃ | OCH₃ |
| III-568 | OH | CONHCH₂COOH | COOCH₃ | OH | OCH₃ | OCH₃ |
| III-569 | H | H | COOH | OH | OCH₃ | OCH₃ |
| III-570 | OH | H | COOH | OH | OCH₃ | OCH₃ |
| III-571 | H | CHO | COOH | OH | OCH₃ | OCH₃ |
| III-572 | OH | CHO | COOH | OH | OCH₃ | OCH₃ |
| III-573 | H | COOH | COOH | OH | OCH₃ | OCH₃ |
| III-574 | OH | COOH | COOH | OH | OCH₃ | OCH₃ |
| III-575 | H | CONHCH₂COOH | COOH | OH | OCH₃ | OCH₃ |
| III-576 | OH | CONHCH₂COOH | COOH | OH | OCH₃ | OCH₃ |
| III-577 | H | C(O)NHCH(CH₂OH)COOH | CH₃ | CH₃ | CH₃ | CH₃ |

**[Table 27]**

| Compound No. | R¹ | R² | R³ | R⁴ | R⁶ | R⁷ |
|---|---|---|---|---|---|---|
| III-578 | CH₃ | C(O)NHCH(CH₂OH)COOH | CH₂CH₃ | CH₃ | CH₃ | CH₃ |
| III-579 | H | C(O)NHCH(CH₂OH)COOH | H | CH₃ | CH₃ | CH₃ |
| III-580 | CH₃ | C(O)NHCH(CH₂OH)COOH | H | CH₃ | CH₃ | CH₃ |
| III-581 | H | C(O)NHCH(CH₂OH)COOH | CH₃ | H | CH₃ | CH₃ |
| III-582 | CH₃ | C(O)NHCH(CH₂OH)COOH | CH₂CH₃ | H | CH₃ | CH₃ |
| III-583 | H | C(O)NHCH(CH₂OH)COOH | H | H | CH₃ | CH₃ |
| III-584 | CH₃ | C(O)NHCH(CH₂OH)COOH | H | H | CH₃ | CH₃ |
| III-585 | H | C(O)NHCH(CH₂OH)COOH | CH₃ | CH₃ | H | CH₃ |
| III-586 | CH₃ | C(O)NHCH(CH₂OH)COOH | CH₂CH₃ | CH₃ | H | CH₃ |
| III-587 | H | C(O)NHCH(CH₂OH)COOH | H | CH₃ | H | CH₃ |
| III-588 | CH₃ | C(O)NHCH(CH₂OH)COOH | H | CH₃ | H | CH₃ |
| III-589 | H | C(O)NHCH(CH₂OH)COOH | CH₃ | H | H | CH₃ |
| III-590 | CH₃ | C(O)NHCH(CH₂OH)COOH | CH₂CH₃ | H | H | CH₃ |
| III-591 | H | C(O)NHCH(CH₂OH)COOH | H | H | H | CH₃ |
| III-592 | CH₃ | C(O)NHCH(CH₂OH)COOH | H | H | H | CH₃ |
| III-593 | H | C(O)NHCH(CH₂OH)COOH | CH₃ | CH₃ | CH₃ | CH₃ |
| III-594 | CH₃ | C(O)NHCH(CH₂OH)COOH | CH₂CH₃ | CH₃ | CH₃ | CH₃ |
| III-595 | H | C(O)NHCH(CH₂OH)COOH | H | CH₃ | CH₃ | CH₃ |
| III-596 | CH₃ | C(O)NHCH(CH₂OH)COOH | H | CH₃ | CH₃ | CH₃ |
| III-597 | H | C(O)NHCH(CH₂OH)COOH | CH₃ | H | CH₃ | CH₃ |
| III-598 | CH₃ | C(O)NHCH(CH₂OH)COOH | CH₂CH₃ | H | CH₃ | CH₃ |
| III-599 | H | C(O)NHCH(CH₂OH)COOH | H | H | CH₃ | CH₃ |
| III-600 | CH₃ | C(O)NHCH(CH₂OH)COOH | H | H | CH₃ | CH₃ |
| III-601 | H | C(O)NHCH(CH₂OH)COOH | CH₃ | CH₃ | H | CH₃ |
| III-602 | CH₃ | C(O)NHCH(CH₂OH)COOH | CH₂CH₃ | CH₃ | H | CH₃ |
| III-603 | H | C(O)NHCH(CH₂OH)COOH | H | CH₃ | H | CH₃ |
| III-604 | CH₃ | C(O)NHCH(CH₂OH)COOH | H | CH₃ | H | CH₃ |
| III-605 | H | C(O)NHCH(CH₂OH)COOH | CH₃ | H | H | CH₃ |

**[Table 28]**

| Compound No. | R¹ | R² | R³ | R⁴ | R⁶ | R⁷ |
|---|---|---|---|---|---|---|
| III-606 | CH₃ | C(O)NHCH(CH₂OH)COOH | CH₂CH₃ | H | H | CH₃ |
| III-607 | H | C(O)NHCH(CH₂OH)COOH | H | H | H | CH₃ |
| III-608 | CH₃ | C(O)NHCH(CH₂OH)COOH | H | H | H | CH₃ |
| III-609 | H | CONHCH₂COOH | CH₃ | CH₃ | CH₃ | CH₃ |
| III-610 | CH₃ | CONHCH₂COOH | CH₂CH₃ | CH₃ | CH₃ | CH₃ |
| III-611 | H | CONHCH₂COOH | H | CH₃ | CH₃ | CH₃ |
| III-612 | CH₃ | CONHCH₂COOH | H | CH₃ | CH₃ | CH₃ |
| III-613 | H | CONHCH₂COOH | CH₃ | H | CH₃ | CH₃ |
| III-614 | CH₃ | CONHCH₂COOH | CH₂CH₃ | H | CH₃ | CH₃ |
| III-615 | H | CONHCH₂COOH | H | H | CH₃ | CH₃ |
| III-616 | CH₃ | CONHCH₂COOH | H | H | CH₃ | CH₃ |
| III-617 | H | CONHCH₂COOH | CH₃ | CH₃ | H | CH₃ |
| III-618 | CH₃ | CONHCH₂COOH | CH₂CH₃ | CH₃ | H | CH₃ |
| III-619 | H | CONHCH₂COOH | H | CH₃ | H | CH₃ |
| III-620 | CH₃ | CONHCH₂COOH | H | CH₃ | H | CH₃ |
| III-621 | H | CONHCH₂COOH | CH₃ | H | H | CH₃ |
| III-622 | CH₃ | CONHCH₂COOH | CH₂CH₃ | H | H | CH₃ |
| III-623 | H | CONHCH₂COOH | H | H | H | CH₃ |
| III-624 | CH₃ | CONHCH₂COOH | H | H | H | CH₃ |
| III-625 | H | CONHCH₂COOH | CH₃ | CH₃ | CH₃ | CH₃ |
| III-626 | CH₃ | CONHCH₂COOH | CH₂CH₃ | CH₃ | CH₃ | CH₃ |
| III-627 | H | CONHCH₂COOH | H | CH₃ | CH₃ | CH₃ |
| III-628 | CH₃ | CONHCH₂COOH | H | CH₃ | CH₃ | CH₃ |
| III-629 | H | CONHCH₂COOH | CH₃ | H | CH₃ | CH₃ |
| III-630 | CH₃ | CONHCH₂COOH | CH₂CH₃ | H | CH₃ | CH₃ |
| III-631 | H | CONHCH₂COOH | H | H | CH₃ | CH₃ |
| III-632 | CH₃ | CONHCH₂COOH | H | H | CH₃ | CH₃ |
| III-633 | H | CONHCH₂COOH | CH₃ | CH₃ | H | CH₃ |
| III-634 | CH₃ | CONHCH₂COOH | CH₂CH₃ | CH₃ | H | CH₃ |
| III-635 | H | CONHCH₂COOH | H | CH₃ | H | CH₃ |
| III-636 | CH₃ | CONHCH₂COOH | H | CH₃ | H | CH₃ |
| III-637 | H | CONHCH₂COOH | CH₃ | H | H | CH₃ |
| III-638 | CH₃ | CONHCH₂COOH | CH₂CH₃ | H | H | CH₃ |
| III-639 | H | CONHCH₂COOH | H | H | H | CH₃ |
| III-640 | CH₃ | CONHCH₂COOH | H | H | H | CH₃ |

**[Table 29]**

| Compound No. | R¹ | R² | R³ | R⁴ | R⁶ | R⁷ |
|---|---|---|---|---|---|---|
| III-641 | H | CHO | CH₃ | CH₃ | CH₃ | CH₃ |
| III-642 | CH₃ | CHO | CH₂CH₃ | CH₃ | CH₃ | CH₃ |
| III-643 | H | CHO | H | CH₃ | CH₃ | CH₃ |
| III-644 | CH₃ | CHO | H | CH₃ | CH₃ | CH₃ |
| III-645 | H | CHO | CH₃ | H | CH₃ | CH₃ |
| III-646 | CH₃ | CHO | CH₂CH₃ | H | CH₃ | CH₃ |
| III-647 | H | CHO | H | H | CH₃ | CH₃ |
| III-648 | CH₃ | CHO | H | H | CH₃ | CH₃ |
| III-649 | H | CHO | CH₃ | CH₃ | H | CH₃ |
| III-650 | CH₃ | CHO | CH₂CH₃ | CH₃ | H | CH₃ |
| III-651 | H | CHO | H | CH₃ | H | CH₃ |
| III-652 | CH₃ | CHO | H | CH₃ | H | CH₃ |
| III-653 | H | CHO | CH₃ | H | H | CH₃ |
| III-654 | CH₃ | CHO | CH₂CH₃ | H | H | CH₃ |
| III-655 | H | CHO | H | H | H | CH₃ |
| III-656 | CH₃ | CHO | H | H | H | CH₃ |
| III-657 | H | CHO | CH₃ | CH₃ | CH₃ | CH₃ |
| III-658 | CH₃ | CHO | CH₂CH₃ | CH₃ | CH₃ | CH₃ |
| III-659 | H | CHO | H | CH₃ | CH₃ | CH₃ |
| III-660 | CH₃ | CHO | H | CH₃ | CH₃ | CH₃ |
| III-661 | H | CHO | CH₃ | H | CH₃ | CH₃ |
| III-662 | CH₃ | CHO | CH₂CH₃ | H | CH₃ | CH₃ |
| III-663 | H | CHO | H | H | CH₃ | CH₃ |
| III-664 | CH₃ | CHO | H | H | CH₃ | CH₃ |
| III-665 | H | CHO | CH₃ | CH₃ | H | CH₃ |
| III-666 | CH₃ | CHO | CH₂CH₃ | CH₃ | H | CH₃ |
| III-667 | H | CHO | H | CH₃ | H | CH₃ |
| III-668 | CH₃ | CHO | H | CH₃ | H | CH₃ |
| III-669 | H | CHO | CH₃ | H | H | CH₃ |
| III-670 | CH₃ | CHO | CH₂CH₃ | H | H | CH₃ |
| III-671 | H | CHO | H | H | H | CH₃ |
| III-672 | CH₃ | CHO | H | H | H | CH₃ |
| III-673 | H | CONHCH₂COOC(CH₃)₃ | CH₃ | CH₃ | CH₃ | CH₃ |
| III-674 | CH₃ | CONHCH₂COOC(CH₃)₃ | CH₂CH₃ | CH₃ | CH₃ | CH₃ |
| III-675 | H | CONHCH₂COOC(CH₃)₃ | H | CH₃ | CH₃ | CH₃ |
| III-676 | CH₃ | CONHCH₂COOC(CH₃)₃ | H | CH₃ | CH₃ | CH₃ |
| III-677 | H | CONHCH₂COOC(CH₃)₃ | CH₃ | H | CH₃ | CH₃ |

**[Table 30]**

| Compound No. | R¹ | R² | R³ | R⁴ | R⁶ | R⁷ |
|---|---|---|---|---|---|---|
| III-678 | CH₃ | CONHCH₂COOC(CH₃)₃ | CH₂CH₃ | H | CH₃ | CH₃ |
| III-679 | H | CONHCH₂COOC(CH₃)₃ | H | H | CH₃ | CH₃ |
| III-680 | CH₃ | CONHCH₂COOC(CH₃)₃ | H | H | CH₃ | CH₃ |
| III-681 | H | CONHCH₂COOC(CH₃)₃ | CH₃ | CH₃ | H | CH₃ |
| III-682 | CH₃ | CONHCH₂COOC(CH₃)₃ | CH₂CH₃ | CH₃ | H | CH₃ |
| III-683 | H | CONHCH₂COOC(CH₃)₃ | H | CH₃ | H | CH₃ |
| III-684 | CH₃ | CONHCH₂COOC(CH₃)₃ | H | CH₃ | H | CH₃ |
| III-685 | H | CONHCH₂COOC(CH₃)₃ | CH₃ | H | H | CH₃ |
| III-686 | CH₃ | CONHCH₂COOC(CH₃)₃ | CH₂CH₃ | H | H | CH₃ |
| III-687 | H | CONHCH₂COOC(CH₃)₃ | H | H | H | CH₃ |
| III-688 | CH₃ | CONHCH₂COOC(CH₃)₃ | H | H | H | CH₃ |
| III-689 | H | CONHCH₂COOC(CH₃)₃ | CH₃ | CH₃ | CH₃ | CH₃ |
| III-690 | CH₃ | CONHCH₂COOC(CH₃)₃ | CH₂CH₃ | CH₃ | CH₃ | CH₃ |
| III-691 | H | CONHCH₂COOC(CH₃)₃ | H | CH₃ | CH₃ | CH₃ |
| III-692 | CH₃ | CONHCH₂COOC(CH₃)₃ | H | CH₃ | CH₃ | CH₃ |
| III-693 | H | CONHCH₂COOC(CH₃)₃ | CH₃ | H | CH₃ | CH₃ |
| III-694 | CH₃ | CONHCH₂COOC(CH₃)₃ | CH₂CH₃ | H | CH₃ | CH₃ |
| III-695 | H | CONHCH₂COOC(CH₃)₃ | H | H | CH₃ | CH₃ |
| III-696 | CH₃ | CONHCH₂COOC(CH₃)₃ | H | H | CH₃ | CH₃ |
| III-697 | H | CONHCH₂COOC(CH₃)₃ | CH₃ | CH₃ | H | CH₃ |
| III-698 | CH₃ | CONHCH₂COOC(CH₃)₃ | CH₂CH₃ | CH₃ | H | CH₃ |
| III-699 | H | CONHCH₂COOC(CH₃)₃ | H | CH₃ | H | CH₃ |
| III-700 | CH₃ | CONHCH₂COOC(CH₃)₃ | H | CH₃ | H | CH₃ |
| III-701 | H | CONHCH₂COOC(CH₃)₃ | CH₃ | H | H | CH₃ |
| III-702 | CH₃ | CONHCH₂COOC(CH₃)₃ | CH₂CH₃ | H | H | CH₃ |
| III-703 | H | CONHCH₂COOC(CH₃)₃ | H | H | H | CH₃ |
| III-704 | CH₃ | CONHCH₂COOC(CH₃)₃ | H | H | H | CH₃ |
| III-705 | H | C(O)NHCH (CO₂tBu)CH₂OtBu | CH₃ | CH₃ | CH₃ | CH₃ |

**[Table 31]**

| Compound No. | R¹ | R² | R³ | R⁴ | R⁶ | R⁷ |
|---|---|---|---|---|---|---|
| III-706 | CH₃ | C(O)NHCH (CO₂tBu)CH₂OtBu | CH₂CH₃ | CH₃ | CH₃ | CH₃ |
| III-707 | H | C(O)NHCH (CO₂tBu)CH₂OtBu | H | CH₃ | CH₃ | CH₃ |
| III-708 | CH₃ | C(O)NHCH (CO₂tBu)CH₂OtBu | H | CH₃ | CH₃ | CH₃ |
| III-709 | H | C(O)NHCH (CO₂tBu)CH₂OtBu | CH₃ | H | CH₃ | CH₃ |
| III-710 | CH₃ | C(O)NHCH (CO₂tBu)CH₂OtBu | CH₂CH₃ | H | CH₃ | CH₃ |
| III-711 | H | C(O)NHCH (CO₂tBu)CH₂OtBu | H | H | CH₃ | CH₃ |
| III-712 | CH₃ | C(O)NHCH (CO₂tBu)CH₂OtBu | H | H | CH₃ | CH₃ |
| III-713 | H | C(O)NHCH (CO₂tBu)CH₂OtBu | CH₃ | CH₃ | H | CH₃ |
| III-714 | CH₃ | C(O)NHCH (CO₂tBu)CH₂OtBu | CH₂CH₃ | CH₃ | H | CH₃ |
| III-715 | H | C(O)NHCH (CO₂tBu)CH₂OtBu | H | CH₃ | H | CH₃ |
| III-716 | CH₃ | C(O)NHCH (CO₇tBu)CH₂OtBu | H | CH₃ | H | CH₃ |
| III-717 | H | C(O)NHCH (CO₂tBu)CH₂OtBu | CH₃ | H | H | CH₃ |
| III-718 | CH₃ | C(O)NHCH (CO₂tBu)CH₂OtBu | CH₂CH₃ | H | H | CH₃ |
| III-719 | H | C(O)NHCH (CO₂tBu)CH₂OtBu | H | H | H | CH₃ |
| III-720 | CH₃ | C(O)NHCH (CO₂tBu)CH₂OtBu | H | H | H | CH₃ |
| III-721 | H | C(O)NHCH (CO₂tBu)CH₂OtBu | CH₃ | CH₃ | CH₃ | CH₃ |
| III-722 | CH₃ | C(O)NHCH (CO₂tBu)CH₂OtBu | CH₂CH₃ | CH₃ | CH₃ | CH₃ |
| III-723 | H | C(O)NHCH (CO₂tBu)CH₂OtBu | H | CH₃ | CH₃ | CH₃ |
| III-724 | CH₃ | C(O)NHCH (CO₂tBu)CH₂OtBu | H | CH₃ | CH₃ | CH₃ |
| III-725 | H | C(O)NHCH (CO₂tBu)CH₂OtBu | CH₃ | H | CH₃ | CH₃ |
| III-726 | CH₃ | C(O)NHCH (CO₂tBu)CH₂OtBu | CH₂CH₃ | H | CH₃ | CH₃ |
| III-727 | H | C(O)NHCH (CO₂tBu)CH₂OtBu | H | H | CH₃ | CH₃ |
| III-728 | CH₃ | C(O)NHCH (CO₂tBu)CH₂OtBu | H | H | CH₃ | CH₃ |
| III-729 | H | C(O)NHCH (CO₂tBu)CH₂OtBu | CH₃ | CH₃ | H | CH₃ |
| III-730 | CH₃ | C(O)NHCH (CO₂tBu)CH₂OtBu | CH₂CH₃ | CH₃ | H | CH₃ |

**[Table 32]**

| Compound No. | R¹ | R² | R³ | R⁴ | R⁶ | R⁷ |
|---|---|---|---|---|---|---|
| III-731 | H | C(O)NHCH (CO₂tBu)CH₂OtBu | H | CH₃ | H | CH₃ |
| III-732 | CH₃ | C(O)NHCH (CO₂tBu)CH₂OtBu | H | CH₃ | H | CH₃ |
| III-733 | H | C(O)NHCH (CO₂tBu)CH₂OtBu | CH₃ | H | H | CH₃ |
| III-734 | CH₃ | C(O)NHCH (CO₂tBu)CH₂OtBu | CH₂CH₃ | H | H | CH₃ |
| III-735 | H | C(O)NHCH (CO₂tBu)CH₂OtBu | H | H | H | CH₃ |
| III-736 | CH₃ | C(O)NHCH (CO₂tBu)CH₂OtBu | H | H | H | CH₃ |
| III-737 | H | C(O)NHCH(CO₂tBu)CH₂Ph | CH₃ | CH₃ | CH₃ | CH₃ |
| III-738 | CH₃ | C(O)(CO₂tBu)CH₂Ph | CH₂CH₃ | CH₃ | CH₃ | CH₃ |
| III-739 | H | C(O)NHCH(CO₂tBu)CH₂Ph | H | CH₃ | CH₃ | CH₃ |
| III-740 | CH₃ | C(O)NHCH(CO₂tBu)CH₂Ph | H | CH₃ | CH₃ | CH₃ |
| III-741 | H | C(O)NHCH(CO₂tBu)CH₂Ph | CH₃ | H | CH₃ | CH₃ |
| III-742 | CH₃ | C(O)NHCH(CO₂tBu)CH₂Ph | CH₂CH₃ | H | CH₃ | CH₃ |
| III-743 | H | C(O)NHCH(CO₂tBu)CH₂Ph | H | H | CH₃ | CH₃ |
| III-744 | CH₃ | C(O)NHCH(CO₂tBu)CH₂Ph | H | H | CH₃ | CH₃ |
| III-745 | H | C(O)NHCH(CO₂tBu)CH₂Ph | CH₃ | CH₃ | H | CH₃ |
| III-746 | CH₃ | C(O)NHCH(CO₂tBu)CH₂Ph | CH₂CH₃ | CH₃ | H | CH₃ |
| III-747 | H | C(O)NHCH(CO₂tBu)CH₂Ph | H | CH₃ | H | CH₃ |
| III-748 | CH₃ | C(O)NHCH(CO₂tBu)CH₂Ph | H | CH₃ | H | CH₃ |
| III-749 | H | C(O)NHCH(CO₂tBu)CH₂Ph | CH₃ | H | H | CH₃ |
| III-750 | CH₃ | C(O)NHCH(CO₂tBu)CH₂Ph | CH₂CH₃ | H | H | CH₃ |
| III-751 | H | C(O)NHCH(CO₂tBu)CH₂Ph | H | H | H | CH₃ |
| III-752 | CH₃ | C(O)NHCH(CO₂tBu)CH₂Ph | H | H | H | CH₃ |
| III-753 | H | C(O)NHCH(CO₂tBu)CH₂Ph | CH₃ | CH₃ | CH₃ | CH₃ |
| III-754 | CH₃ | C(O)NHCH(CO₂tBu)CH₂Ph | CH₂CH₃ | CH₃ | CH₃ | CH₃ |
| III-755 | H | C(O)NHCH(CO₂tBu)CH₂Ph | H | CH₃ | CH₃ | CH₃ |
| III-756 | CH₃ | C(O)NHCH(CO₂tBu)CH₂Ph | H | CH₃ | CH₃ | CH₃ |

**[Table 33]**

| Compound No. | R¹ | R² | R³ | R⁴ | R⁶ | R⁷ |
|---|---|---|---|---|---|---|
| III-757 | H | C(O)NHCH (CO₂tBu)CH₂Ph | CH₃ | H | CH₃ | CH₃ |
| III-758 | CH₃ | C(O)NHCH (CO₂tBu)CH₂Ph | CH₂CH₃ | H | CH₃ | CH₃ |
| III-759 | H | C(O)NHCH (CO₂tBu)CH₂Ph | H | H | CH₃ | CH₃ |
| III-760 | CH₃ | C(O)NHCH (CO₂tBu)CH₂Ph | H | H | CH₃ | CH₃ |
| III-761 | H | C(O)NHCH (CO₂tBu)CH₂Ph | CH₃ | CH₃ | H | CH₃ |
| III-762 | CH₃ | C(O)NHCH (CO₂tBu)CH₂Ph | CH₂CH₃ | CH₃ | H | CH₃ |
| III-763 | H | C(O)NHCH (CO₂tBu)CH₂Ph | H | CH₃ | H | CH₃ |
| III-764 | CH₃ | C(O)NHCH (CO₂tBu)CH₂Ph | H | CH₃ | H | CH₃ |
| III-765 | H | C(O)NHCH (CO₂tBu)CH₂Ph | CH₃ | H | H | CH₃ |
| III-766 | CH₃ | C(O)NHCH (CO₂tBu)CH₂Ph | CH₂CH₃ | H | H | CH₃ |
| III-767 | H | C(O)NHCH (CO₂tBu)CH₂Ph | H | H | H | CH₃ |
| III-768 | CH₃ | C(O)NHCH (CO₂tBu)CH₂Ph | H | H | H | CH₃ |

As used herein, the pyruvate dehydrogenase inhibitor is not particularly limited as long as it is an agent to use for inhibiting pyruvate dehydrogenase. Preferably, the pyruvate dehydrogenase inhibitor of the present invention may be provided as a pharmaceutical composition. For example, the pyruvate dehydrogenase inhibitor of the present invention may be a pharmaceutical composition for inhibiting pyruvate dehydrogenase or administering to a subject as an action mechanism. Such pharmaceutical compositions can be used for treatment or prophylaxis of diseases or disorders that pyruvate dehydrogenase relates to or contributes to its development or aggravation. Thus, in the other words, the pyruvate dehydrogenase inhibitor of the present invention may be a pharmaceutical composition for treating or preventing diseases or disorders that pyruvate dehydrogenase relates to or contributes to its development or aggravation. The diseases or disorders that pyruvate dehydrogenase relates to or contributes to its development or aggravation include, for example, influenza aggravation after infection, anorexia, mitochondrial diseases, diseases or disorders accompanied by decreasing ATP production, diabetes or cancer. As used herein, "influenza aggravation" is used as distinct from influenza infection. Thus, the influenza aggravation does not include an infection itself by influenza virus, and means symptoms, diseases or disorders caused secondly by influenza virus infection. Such symptoms, diseases or disorders caused secondly by influenza virus infection may include, for example, multiple organ failure (MOF: Multiple organ failure), influenza encephalopathy (IAE: Influenza-associated encephalopathy), weight loss and anorexia. Thus, treatment or prophylaxis of influenza aggravation in the present specification include treatment or prophylaxis of multiple organ failure (MOF: Multiple organ failure), influenza encephalopathy (IAE: Influenza-associated encephalopathy), weight loss and anorexia. Here, the mitochondrial diseases mean diseases or disorders based on having a mutation in mitochondrial ATP synthase group, which include, for example, pyruvate dehydrogenase deficiency, MELAS, and the like. The diseases or disorders accompanied by decreasing ATP production include, for example, diseases or disorders based on a mutation of carnitine palmitoyl transferase.

The present invention relates to a pharmaceutical composition comprising a compound represented by the general formula (I) as an active ingredient. Types of the pharmaceutical composition are not particularly limited, a formulation may include tablets, capsules, granules, powders, syrups, suspensions, suppositories, ointments, creams, gels, patches, inhalants, injections, and the like. These formulations can be prepared by a conventional method. The liquid formulations may be a formulation which is dissolved or suspended in water or other suitable solvents when used. Tablets and granules may be coated by a known method. In the case of injection, the compound of the present invention are dissolved in water, or, if necessary, in saline or glucose solution, optionally added buffers and preservatives. Any formulations for oral or parental administration are provided. It may be prepared as pharmaceutical compositions for the oral administration such as, for example, granules, fine granules, powders, hard capsules, soft capsules, syrups, emulsions, suspensions or solutions may be prepared, or for the parental administration such as intravenous administration, intramuscular administration, or injections such as for subcutaneous administration, drip infusions, transdermal absorbents, transmucosal absorbents, nasal drops, inhalants or suppositories. The injections or drip infusions may be prepared in the form of powders such as lyophilized form, then may be used by dissolving in an appropriate aqueous medium such as a saline when used.

### EFFECT OF THE INVENTION

The compound of the present invention represented by the general formula (I) shows a novel enzyme inhibitory activity and an efficacy of animal models, and it is useful for treatment or prophylaxis of diseases or disorders that pyruvate dehydrogenase kinase relates to or contributes to its development or aggravation. Specifically, the compound of the present invention represented by the general formula (I) is the first compound inhibits PDK4 in nM order, and exhibits a strong activity even in vivo at a dose of 100-fold or more lower than the dichloroacetic acid is an existing drug, it is possible to give treating or preventing agent of diseases or disorders that pyruvate dehydrogenase kinase relates to or contributes to its development or aggravation. When the compound of the present invention represented by the general formula (I) is administered to influenza infected mice model, the effect to protect mice against weight loss and death was confirmed, thus the compound of the present invention is effective for prevention and treatment of aggravation after influenza infection.
In particular, influenza infected mice model which are administered the compound of the present invention represented by the general formula (I) is observed recovering food and water consumption to a level near the uninfected mice and improving ATP levels form biochemical analysis, thus it is effective for treatment or prevention of diseases or disorders depending on ATP level reduction. The compound of the present invention represented by the general formula (I), in addition to prevent aggravation of influenza infection, shows the effect in diseases which are expected by inhibiting PDK4, thus it is effective to treat or prevent of such diseases. The compound of the present invention represented by the general formula (I) shows the effect as a cosmetic by cell metabolic improvement with mitochondrial function activation, thus it is also useful as a cosmetic.

### BRIEF DESCRIPTION OF THE DRAWINGS

[Figure 1] A graph showing the effect of the compound of the invention in influenza infected mice model. In the figure, the vertical axis represents the survival rate (%), the horizontal axis represents the number of days elapsed after influenza infection (day).

### MODE FOR CARRYING OUT THE INVENTION

The compound of the present invention can be synthesized by the following method.

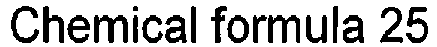

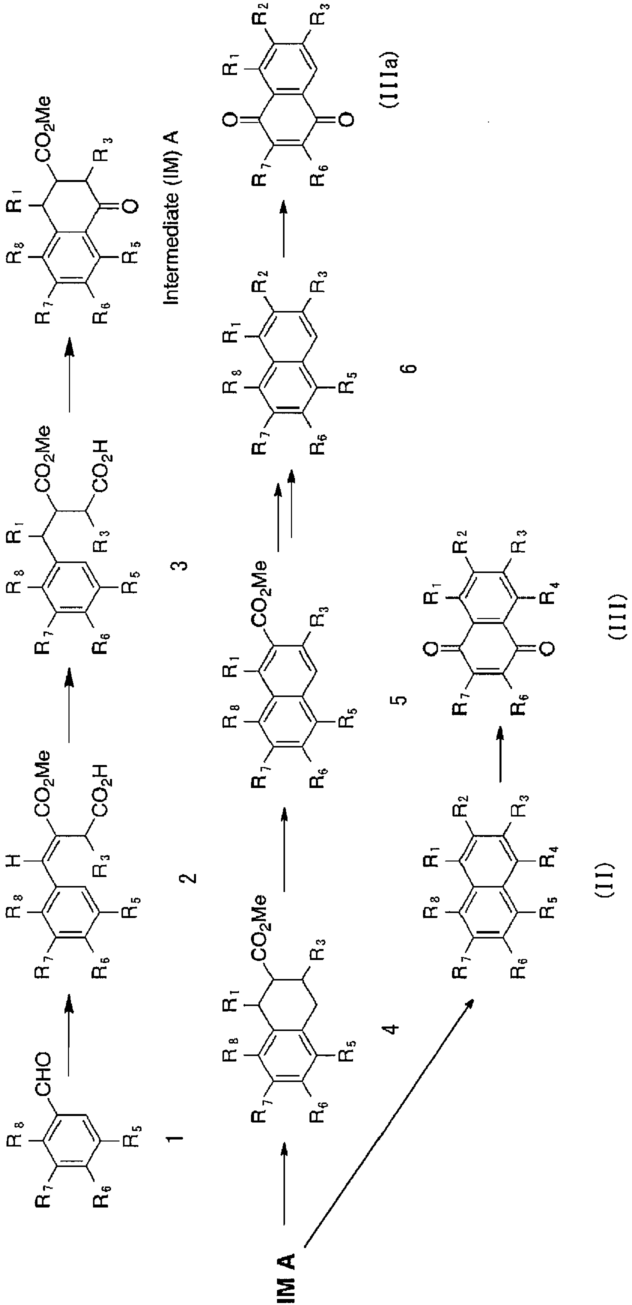

Under N₂ atmosphere, starting material aldehyde 1 and CH₃COCH (R³) CH₂COOCH₃ in t-butanol are added to a heated solution of dissolved potassium t-butoxide in t-butanol and reacted under heating to reflux. After the starting material 1 is consumed all, the reaction solution is cooled and evaporated excess solvent under reduced pressure. After the obtained residue is added to acidify the addition of 2M hydrochloric acid and extracted with diethyl ether. The 2M aqueous sodium hydroxide solution is added to ether extracts, after which the product is moved to the aqueous layer, the 4M hydrochloric acid is added to the aqueous solution, give an acid solution again. The aqueous solution is extracted with diethyl ether, dried, concentrated, and p flash chromatography purification to obtain the compound 2.

After adding 5% palladium / carbon to a solution of the compound 2 in methanol at room temperature, the solution is reacted under an atmosphere of hydrogen gas. After completion of the reaction, the reactant is filtered through Celite to remove the palladium reagent, and the filtrate is concentrated. The resulting crude product is purified by flash chromatography purification to obtain the compound 3.

The compound 3 is dissolved in polyphosphoric acid at room temperature then stirred and heated to 80 °C. After completion of the reaction, the reaction mixture is cooled to room temperature, the reaction mixture is poured into cold water with vigorously stirring to decompose the excess of polyphosphoric acid. The resulting aqueous solution is extracted with chloroform, dried and concentrated, and purified by performing flash chromatography purification to obtain intermediate (IM) A.

At room temperature, the intermediate (IM) A is dissolved in a triethylsilane, then trifluoroacetic acid is added to the solution to react. After completion of the reaction, triethylsilane and trifluoroacetic acid are evaporated under reduced pressure, then purified by flash chromatography purification to obtain the compound 4.

Under N₂ atmosphere, to the solution of the compound 4 dissolved in benzene at room temperature, 2,3-dichloro 5,6-dicyano-1,4-benzoquinone is added and heated to 80 °C, and reacted with stirring. After completion of the reaction, the mixture is cooled to room temperature, then a saturated aqueous sodium hydrogen carbonate solution is added to stop the reaction. The resulting solution is separated, dried and concentrated, and purified by performing flash chromatography purification to obtain the compound 5.

The compound 5 is dissolved in THF·H₂O at room temperature, 1 M aqueous sodium hydroxide is added and stirred. After completion of the reaction, the reaction mixture is acidified by adding a 1 M hydrochloric acid at room temperature. The resulting solution is extracted with chloroform, dried and concentrated, then purified by flash chromatography purification to obtain the compound 5, which is carboxylic acid derivative 6.

The solution of the compound 5 or the compound 6 in acetonitrile is added a cerium nitrate ammonium aqueous solution at room temperature. After completion of the reaction, the reaction mixture is diluted by adding water at room temperature, the resulting solution is extracted with chloroform, dried and concentrated, then purified by flash chromatography purification to obtain 1,4-naphthoquinone compound (IIIa).

Under N₂ atmosphere, to a solution of a lithium diisopropylamine in tetrahydrofuran the intermediate (IM) A is added slowly at -10 °C, after stirring for 10 min, iodine is added to the solution and further stirred. After completion of the reaction, sodium thiosulfate aqueous solution is added to the reaction mixture to reduce the excess of iodine. The resulting solution is extracted with chloroform, dried and concentrated, then purified by flash chromatography purification to obtain naphthol derivative (II) (R⁴ =OH).

Further, to a solution of the present compound in pyridine, the anhydride (RCO) ₂ is added and stirred at room temperature and after completion of the reaction, the reaction mixture is diluted by adding water at room temperature, the resulting solution is extracted with chloroform, dried, concentrated, then purified by flash chromatography purification to obtain acyloxy derivative (II) (R₄ =OCOR).

To a solution of acyl derivative (II) (R₄ =OCOR) in acetonitrile, a cerium nitrate ammonium aqueous solution is added at room temperature and stirred. After completion of the reaction, the reaction mixture is diluted by adding water at room temperature, the resulting solution is extracted with chloroform, dried and concentrated, then purified by flash chromatography purification to obtain 1,4-naphthoquinone-acyloxy derivative (III) (R⁴ =OCOR).

Further, to a solution of the present compound in methanol, aqueous sodium hydrogen carbonate solution is added stirred at is diluted by adding water to room temperature, the resulting solution is extracted with chloroform, dried, concentrated, then purified by flash chromatography purification to obtain 1,4-naphthoquinone derivatives (III) (R⁴ =OH).

The compound of the present invention can also be synthesized by the following method.

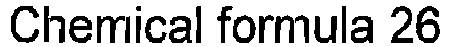

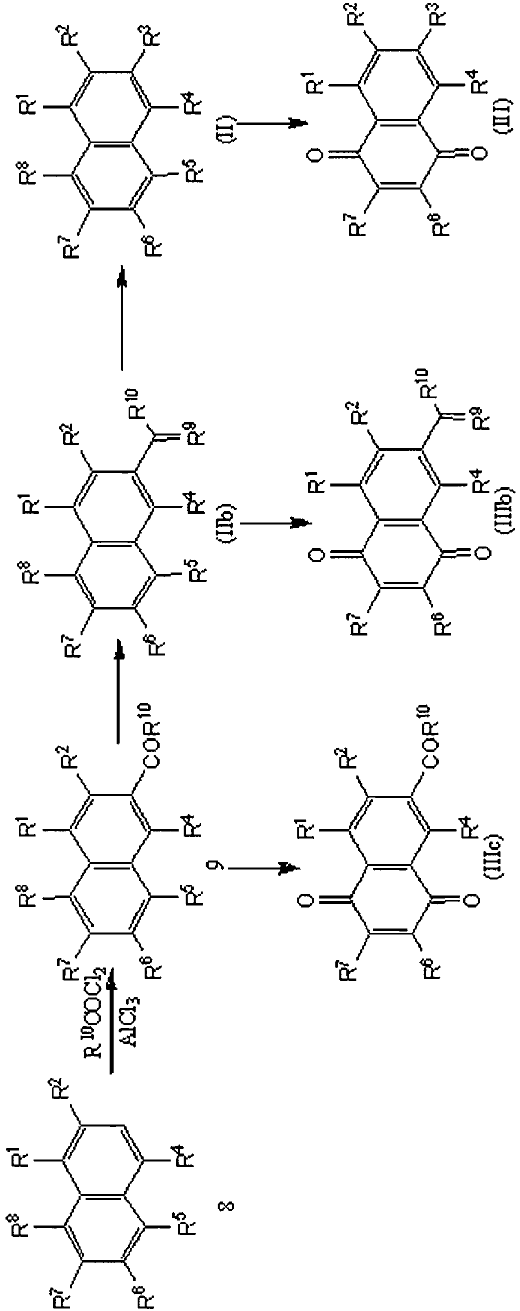

Under N₂ atmosphere, to a solution of the compound 8 in dichloromethane is added aluminium chloride at 0 °C. After stirring for 10 min, a fatty acid chloride (R¹⁰COCl) is added dropwise to the solution, after raising the temperature to room temperature, and further stirred. After completion of the reaction, water is added to the reaction mixture, the resulting mixture is extracted with chloroform, dried and concentrated, then purified by flash chromatography purification to obtain the compound 9.

Under O₂ atmosphere, to a solution of the compound 9 in dimethylformamide is added a powdered potassium hydroxide at room temperature, then the mixture is heated to 68 °C with stirring. After completion of the reaction, the reaction mixture is poured into cold water to dilute, and the aqueous solution is washed with ether, acidified by adding 4M hydrochloric acid, extracted with chloroform, dried and concentrated, then purified by flash chromatography purification to obtain the compound (IIb) (R⁹ =O, R10 =OH).

Further the compound (IIb) (R⁹ =O, R¹⁰=OH) is dissolved in benzene-methanol at 0 °C. After adding trimethylsilyl diazomethane and stirring, the ester derivative (IIb) (R⁹=O, R¹⁰ =OMe) may be obtained by concentration and flash chromatography. Further under N₂ atmosphere, the compound (lib) (R⁹ =O, R¹⁰ =OH) is dissolved in dichloromethane at room temperature and added a primary or secondary amine compound (R (R') NH), diisopropylethylamine, and hexafluorophosphoric acid (benzene triazole-1-yloxy) tri-pyridinophosphonium (PyBOP). After stirring, the reaction is quenched with 10% aqueous methanol solution, the mixture is extracted with chloroform, dried, concentrated, then purified by flash chromatography purification to obtain the amide compound (IIb) (R⁹ =O, R¹⁰ = NR (R')).

Under N₂ atmosphere, to a solution of the ester compound (IIb) (R⁹ =O, R¹⁰ =OMe) in tetrahydrofuran is added dropwise a diisobutyl aluminum hydride at -78°C with stirring. After completion of the reaction, water is added to inactivate excess reagent, and the reaction mixture is raised to room temperature, saturated Rochelle salt solution is added and stirred. The mixture solution is extracted with chloroform, dried and concentrated, then purified by flash chromatography purification to obtain the alcohol compound (II) (R³ = CH₂OH).
Further, under N₂ atmosphere, the alcohol compound (II) (R³ = CH₂OH) is dissolved in dichloromethane at room temperature, an activated manganese dioxide powder is added, and after stirring, the aldehyde compound (II) (R³ = CHO) may be obtained by filtration and concentration.

To a solution of the compound (II) in acetonitrile, a cerium nitrate ammonium aqueous solution is added at room temperature and stirred. After completion of the reaction, the reaction mixture is diluted by adding water at room temperature, the resulting solution is extracted with chloroform, dried, concentrated, then purified by flash chromatography purification to obtain 1,4-naphthoquinone derivatives (III) of performing flash chromatography purification.
Further, at room temperature, to a solution of 1,4-naphthoquinone aldehyde derivative (III) (R³ = CHO) in dichloromethane is added O- replacement hydroxylamine with stirring. After completion of the reaction, to the solution water is added, and the resulting mixture is extracted with chloroform, dried, concentrated, then purified by flash chromatography purification to obtain oxime derivative (III) (R³ = CHN-O-R).

Under N₂ atmosphere, to a solution of the compound IIb in acetonitrile, cerium nitrate ammonium aqueous solution is added and stirred. After completion of the reaction, the reaction mixture is diluted by adding water at room temperature, the resulting solution is extracted with chloroform, dried, concentrated, then purified by flash chromatography purification to obtain 1,4-naphthoquinone derivative (IIIb).

To a solution of the compound 9 in acetonitrile, a cerium nitrate ammonium aqueous solution is added at room temperature and stirred. After completion of the reaction, the reaction mixture is diluted by adding water at room temperature, the resulting solution is extracted with chloroform, dried, concentrated, then purified by flash chromatography purification to obtain 1,4-naphthoquinone derivative (IIIc).

The compound of the present invention represented by the general formula (I) has an inhibitory activity against PDK4, and it may be used as a pharmaceutical composition for diseases or disorders that PDK inhibitors (eg. PDK4 inhibitors) or PDK (particularly PDK4) relate to its development or aggravation, or a cosmetic composition. The diseases or disorders that PDK (particularly PDK4) relate to its development or aggravation mean the diseases or disorders that are developed or aggravated by promoting expression or increasing activity of PDK (particularly PDK4). Examples include influenza aggravation after infection, anorexia, mitochondrial diseases, diseases or disorders accompanied by decreasing ATP production, diabetes or cancer. The influenza aggravation after infection is diseases, disorders, or symptoms caused by influenza infection, and includes multiple organ failure (MOF: Multiple organ failure), influenza encephalopathy (IAE: Influenza-associated encephalopathy), multiple organ failure (MOF: Multiple organ failure), influenza encephalopathy (IAE: Influenza-associated encephalopathy), multiple organ failure (MOF: Multiple organ failure), influenza encephalopathy (IAE: Influenza-associated encephalopathy). From the present inventors research output, it is confirmed that if influenza infection works for increasing acute cytokine level and causes a fever, PDK4 expression is induced, the induced PDK4 phosphorylates PDH, and PDH activity decreases to cause ATP depletion by acute mitochondrial function reduction, then acute aggravation, multiple organ failure, or influenza encephalopathy is induced. Thus, the influenza aggravation after infection in the present invention includes every symptom, disease, or disorder triggered by influenza infection and PDK4 expression induction in the mechanism above. Since, from the present inventors' discovery, it becomes clear that PDK4 phosphorylates PDH, and PDH activity decreases to cause ATP depletion, the PDK4 inhibitors of the present invention may be used for treatment or prophylaxis of diseases or disorders based on mitochondrial function reduction, or diseases or disorders based on ATP reduction. Specifically, the PDK4 inhibitors of the present invention may be treating or preventing agents for mitochondrial diseases, or diseases or disorders accompanied by decreasing ATP production. The mitochondrial diseases mean diseases, disorders or symptoms based on having a mutation in mitochondrial ATP synthase group, which include, for example, pyruvate dehydrogenase deficiency, MELAS, and the like. The diseases or disorders accompanied by decreasing ATP production include diseases, disorders or symptoms based on a mutation of carnitine palmitoyl transferase.

Thus, in another embodiment, the present invention relates to a treating or preventing method of diseases or disorders that PDK (particularly PDK4) relates to its development or aggravation, which includes administering an effective amount of the compound represented by the general formula (I) to a patient who needs it. Alternatively, the present invention relates to a compound represented by the general formula (I) for treatment or prophylaxis of diseases or disorders that PDK (particularly PDK4) relates to its development or aggravation.

The pharmaceutical composition of the present invention may be prepared by a conventional method by using a pharmaceutically acceptable carrier. When preparing oral solid formulations, excipients are added to the active ingredient, then by a conventional method to give a solvent, granules, powders, capsules, and the like after optionally adding binders, disintegrants, lubricants, and the like. When preparing injections, pH adjusting agents, buffering agents, stabilizing agents, solubilizing agents, and the like are optionally added to the active ingredient to obtain subcutaneous or intravenous injection by a conventional method.

The pharmaceutical compositions of the present invention may be used in oral administration forms or parental administration forms such as injection or drip infusions. When the compound is administered to mammals, it may be administered orally as tablets, powders, granules or syrups, or parentally as injections or drip infusions. Amount of dosage may be decided according to degree of symptoms, age, weight, sex, administration route, dosage form, responsiveness to drugs, or type of disease, it may be, for example, 50 - 500mg daily for adults in once to several times divided doses.

### EXAMPLES

The present invention is hereinafter described more specifically by means of the following production examples and experimental examples, and is not limited in any way. All the documents referred herein are incorporated by reference in its entirety.

### (Production Example 1)

### 3-carbomethoxy-4-(2,5-dimethoxy-3-methyl-phenyl)-3-butenoic acid

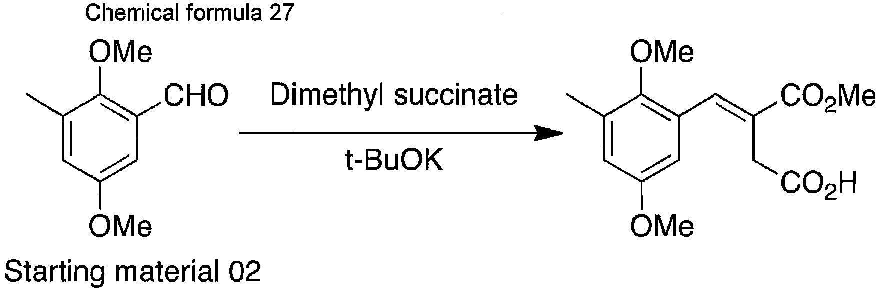

To a solution oft-BuOK (2.90mg, 25.8mmol) in t-BuOH (36.7mL), a solution of the starting material 02 (3.87mg, 21.5mmol) and dimethyl succinate (3.1 mL, 23.7mmol) in t-BuOH (35mL) was added dropwise under N₂ while being refluxed, then the reaction mixture was refluxed with stirring. After 40 minutes, the mixture was cooled at room temperature. Most of t-BuOH was removed under reduced pressure, then, thus obtained suspension was acidified by the addition of 2N aqueous HCl (30mL), and extracted with Et₂O (50mL x 3). The combined organic layers were extracted with 2N aqueous NaOH (40mL x 5) was, and the combined aqueous solution was acidified by the addition of 4N aqueous HCl (150mL). Then, the acid solution was extracted with Et₂O (100mL x 3), dried over Na₂SO₄, filtrated, and concentrated. Flash column chromatography (silica gel; CHCl₃ / MeOH = 40/1) gave the title compound (4.69g, 74% yield) as a colorless oil.

¹H-NMR (500MHz, CDCl₃) δ (ppm): 2.28 (s, 3H), 3.54 (s, 2H), 3.62 (s, 3H), 3.76 (s, 3H), 3.86 (s, 3H), 6.67 (d, J = 2.8Hz, 1 H), 6.75 (d, J = 2.8Hz, 1 H), 7.98 (s, 1 H) HRMS (FAB) m / z [M + H] ⁺ (294.1103 calcd for C₁₅H₁₈O₆)

### (Production Example 2) 3-carbomethoxy-4-(2,5-dimethoxy-3-methyl-phenyl) butenoic acid

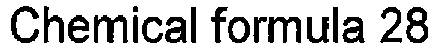

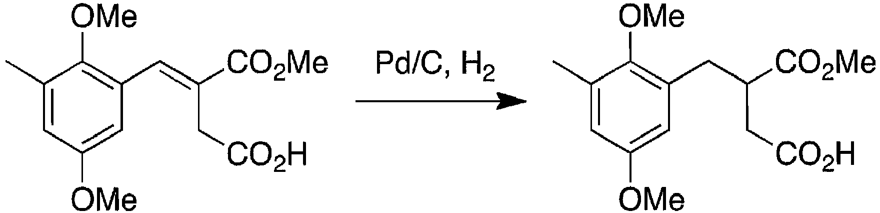

To a solution of the butenoic acid (436mg, 1.48mmol) in MeOH (15mL), 0.5% Pd / C (218mg) was added at room temperature. The mixture was stirred at room temperature under H₂. After stirring for 1 hour, the solution was filtrated through Celite (registered trademark) to remove the Pd / C, and the filtrate was concentrated. Flash column chromatography (silica gel; CHCl₃ / MeOH = 40/1) gave the title compound (420mg, 96% yield) as a colorless oil.

¹H-NMR (500MHz, CDCl₃) δ (ppm): 2.61 (s, 3H), 2.45 (dd, J =4.6, 17.2Hz, 1H), 2.69 (dd, J = 9.2, 17.2Hz, 1 H), 2.77 (dd, J = 8.9, 13.8Hz, 1 H), 3.02 (dd, J = 5.8, 13.8Hz, 1 H), 3.16 (m, 1 H), 3.66 (s, 3H), 3.68 (s, 3H), 3.73 (s, 3H), 6.48 (d, J = 2.9Hz, 1 H), 6.60 (d, J = 2.9Hz, 1 H)
¹³C-NMR (125MHz, CDCl₃) δ (ppm)

### (Production Example 3)

### 3-carbomethoxy-3,4-dihydro-2H-5,8-dimethoxy-6-methyl-1-oxo-naphthalene (IM01) and 3-carboxyl-3,4-dihydro-2H-5,8-dimethoxy-6-methyl-1-oxo-naphthalene (IM02)

Chemical formula 29

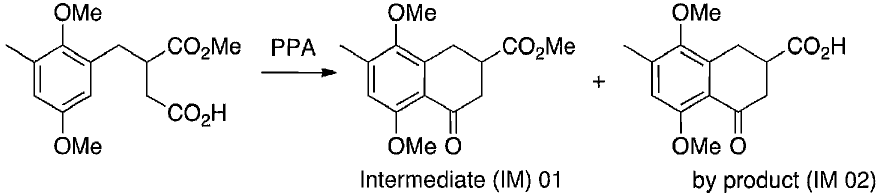

The half ester of butanoic acid (1.81g, 6.11mmol) was dissolved in polyphosphoric acid (30.5mL), and stirred at 80 °C for 40 minutes. Then, the reaction mixture was cooled to room temperature and quenched by pouring into ice-cold water (50mL) with vigorous stirring. The mixture was extracted with CHCl₃ (50mL x 3), and the combined organic layers were dried over Na₂SO₄, filtrated, and concentrated. Flash column chromatography (silica gel; ethyl hexane / acetate = 40/1) gave respectively IM01 (1.43g, 84% yield) as and IM02 (132mg, 8.2% yield) both as a colorless solid.

### (IM01)

¹H-NMR (500MHz, CDCl₃) δ (ppm): 2.34 (s, 3H), 2.77 (dd, J = 10.9,17.2Hz, 1H), 2.85 (ddd, J = 1 .7,4.6,17.2Hz, 1H), 2.97 (dd, J = 10.3,16.0,1H), 3.04 (m, 1H), 3.36 (ddd, J = 1.7,3.5,16.0Hz, 1H), 3.69 (s, 3H), 3.71 (s, 3H), 3.86 (s, 3H), 6.67 (s, 1H)
¹³C-NMR (125MHz, CDCl₃) δ (ppm):
17.2,26.5,39.5,42.3,52.3,56.3,60.4,112.8,120.2,136 .7,138.9,149.1,156.7,173.9,194.7 HRMS (FAB) m / z [M + H] ⁺ calcd for C₁₅H₁₈O₅ 278.1154;

### (IM02)

¹H-NMR (500MHz, CDCl3) δ (ppm): 2.34 (d, J = 5.2Hz, 3H), 2.80 (m, 1 H), 2.87 (m, 1 H), 3.02 (m, 1 H), 3.10 (m, 1 H), 3.40 (m, 1 H), 3.71 (s, 3H), 3.87 (s, 3H), 6.68 (d, J = 5.2Hz, 1 H)
¹³C-NMR (125MHz, CDCl₃) δ (ppm)

### (Production Example 4)

### 2-carbomethoxy-5,8-dimethoxy-7-methyl-1,2,3,4-tetrahydronaphthalene (IM03)

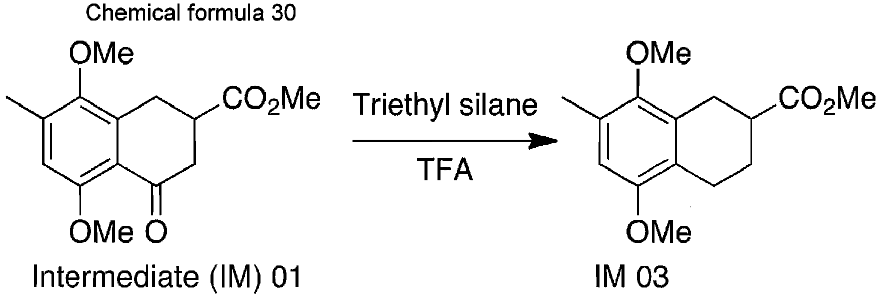

To a solution of IM01 (1.40g, 5.03mmol) in triethylsilane (2.5mL), TFA (7.6mL) was added at room temperature. The reaction mixture was stirred for 5 minutes and then concentrated. The residue was purified by flash column chromatography (silica gel; CHCl₃ / MeOH = 100/1) to give the IM03 (1.32g, 99% yield) as a colorless solid.

¹H-NMR (500MHz, CDCl₃) δ (ppm): 1.75 (ddd, J = 5.7,11.5,24.1 Hz, 1H), 2.22 (m, 1H), 2.30 (s , 3H), 2.55 (ddd, J = 6.3,11.5,17.5Hz, 1 H), 2.65 (m, 1H), 2.81 (dd, J = 10.9,16. 1Hz, 1H), 2.89 (ddd, J = 3.2,5.7,17.5Hz, 1 H), 3.17 (dd, J = 4.0,16.1 Hz, 1 H), 3.70 (s, 3H), 3.72 (s, 3H), 3.87 (s, 3H), 6.68 (s, 1 H)
¹³C-NMR (125MHz, CDCl₃) δ (ppm):
16.3,22.8,25.4,26.4,39.6,51.9,55.5,60.0,109.7,123.3,127.9,129.5,149.9,153.2,176.4 HRMS (EI) m / z 264.1355 [M] ⁺ (264.1362 calcd for C₁₅H₂₀O₄)

### (Production Example 5) 7-carbomethoxy-1,4-dimethoxy-2-methyl-naphthalene

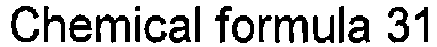

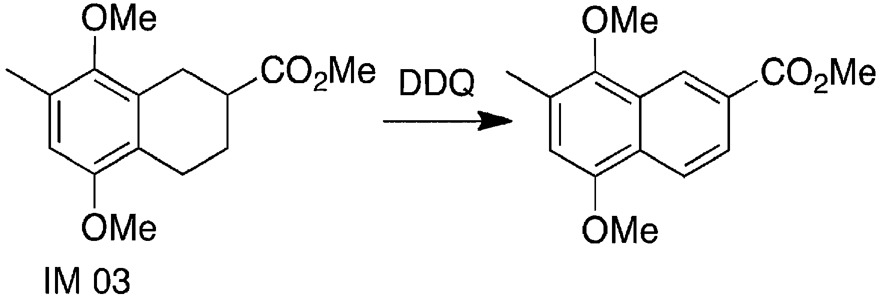

To a solution of IM03 (141 mg, 0.53mmol) in benzene (2.65mL), DDQ (243mg, 1.07mmol) was added under N₂ at room temperature. The reaction mixture was warmed to 70 °C, stirred for 1 hour, then cooled to room temperature. The reaction mixture was quenched with aqueous NaHCO₃ (2mL), and the mixture which was separated the two layers was obtained. The organic layer was washed with aqueous NaHCO₃ (5mL x 3), dried over Na₂SO₄, and concentrated. Flash column chromatography (silica gel; CHCl₃) gave the title compound (112mg, 81% yield) as a colorless solid.

¹H-NMR (500MHz, CDCl₃) δ (ppm): 2.46 (s, 3H), 3.89 (s, 3H), 3.97 (s, 3H), 3.98 (s, 3H), 6.71 (s, 3H), 8.00 (dd, J = 1.7Hz, 8.6Hz, 1H), 8.23 (d, J = 8.6Hz, 1H), 8.76 (d, J = 1.7Hz, 1 H)
HRMS (EI) m / z 260.1056 [M] ⁺ (260.1049 calcd for C₁₅H₁₆O₄)

### (Production Example 6) 7-carbomethoxy-2-methyl-1,4-naphthoquinone (KIS-077)

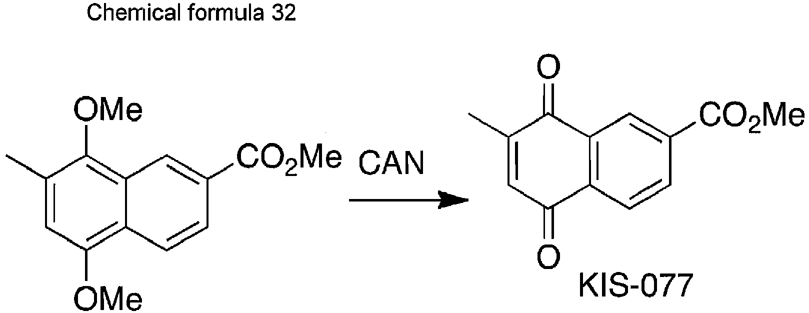

To a solution of the dimethoxynaphthalene ester (112mg, 0.43mmol) in MeCN (3.0mL), a solution of CAN (707.3mg, 1.29mmol) in H₂O (3.0mL) was added at room temperature, the reaction mixture was stirred for 10 minutes. The mixture was then diluted with H₂O (5mL), and extracted with CHCl₃ (10mL x 3). The combined organic layers were dried over Na₂SO₄, filtrated, and concentrated. Flash column chromatography (silica gel; hexane / ethyl acetate = 20/1) gave KIS-077 (92.8mg, 94% yield) as a pale yellow solid.

¹H-NMR (500MHz, CDCl₃) δ (ppm): 2.22 (d, J = 1.7Hz, 3H), 3.99 (s, 3H), 6.88 (dd, J = 1.7, 3.2Hz, 1H), 8.12 (d, J = 8.0Hz, 1H), 8.35 (dd, J = 1.7, 8.0Hz, 1 H), 8.72 (d, J = 1.7Hz, 1H)
HRMS (EI) m / z 230.0575 [M] ⁺ (230.0579 calcd for C₁₃H₁₀O₄)

### (Production Example 7) 7-carboxyl-1,4-dimethoxy-2-methylnaphthalene

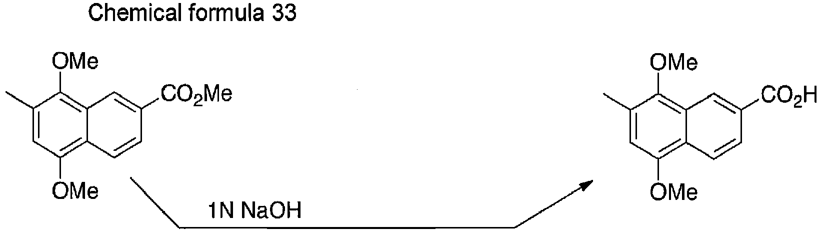

To a solution of the dimethoxynaphthalene ester (493mg, 1.98mmol) in THF (25.2mL) and H₂O (12.6mL), 1 N aqueous NaOH solution (7.0mL) was added. The reaction mixture was stirred at room temperature for 10 hours and then acidified by the addition of 1 N aqueous HCl (10mL). The thus obtained mixture was extracted with CHCl₃ (20mL × 3), and the combined organic layers were dried over Na₂SO₄, filtrated, and concentrated. Flash column chromatography (silica gel; CHCl₃ / MeOH = 40/1) gave the title compound (454.8mg, 97% yield) as a colorless solid.

¹H-NMR (500MHz, CDCl₃) δ (ppm): 2.47 (s, 3H), 3.90 (s, 3H), 3.99 (s, 3H), 6.74 (s, 1 H), 8.04 (dd, J = 1.7, 8.6Hz, 1 H), 8.26 (d, J = 8.6Hz, 1 H), 8.85 (d, J = 1.7Hz, 1H) ¹³C-NMR (125MHz, CDCl₃) δ (ppm)

### (Production Example 8) 7-carboxyl-2-methyl-1,4-naphthoquinone (KIS-078)

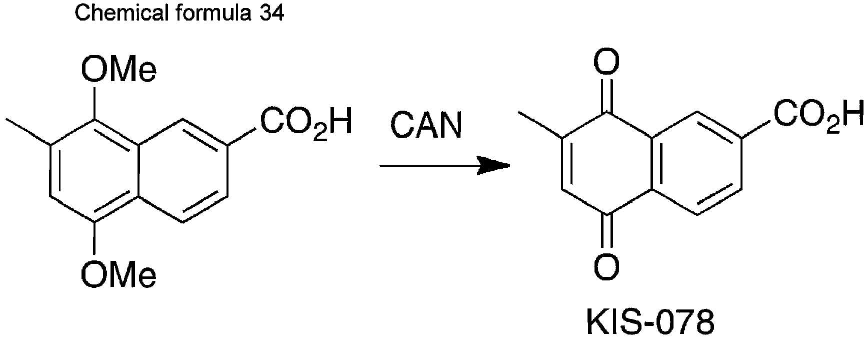

By the procedure of preparation of KIS-077, the carboxylic acid compound (100mg, 0.36mmol) was changed into a yellow solid of KIS-078 (67.7mg, 87% yield) (purification; silica gel; CHCl₃ / MeOH = 50/1).

¹H-NMR (500MHz, CDCl₃) δ (ppm): 2.25 (d, J = 1.7Hz, 3H), 6.93 (d, J = 1.7Hz, 3H), 8.18 (d, J = 8.0Hz, 1H), 8.44 (dd, J = 1.7, 8.0Hz, 1H), 8.83 (d, J = 1.7Hz, 1H) ¹³C-NMR (125MHz, CDCl₃) δ (ppm)

### (Production Example 9)

### 7-carbomethoxy-5-hydroxy-2,4-dimethoxy-2-methylnaphthalene

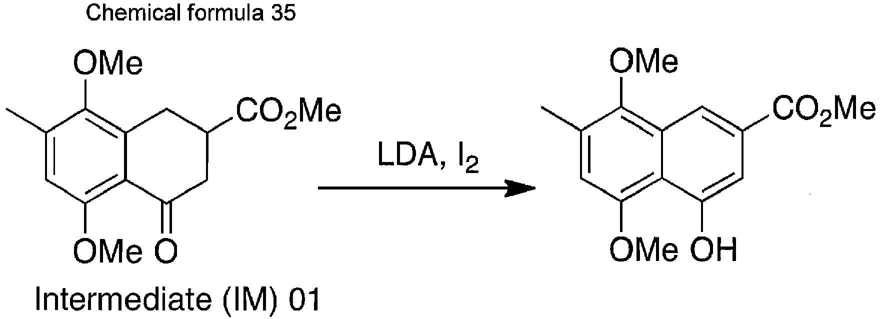

To solution of diisopropylamine (122µL, 0.87mmol) in THF (7.2mL), n-BuLi (1.64M) in hexane (524µL, 0.86mmol) was added dropwise under N₂ at -78 °C, the mixture was stirred at -78 °C for 15 minutes to prepare lithium diisopropylamine in THF solution, then warmed to -10 °C. A solution of IM01 (200mg, 0.72mmol) in THF (7.2mL) was added to the above LDA solution and the resulting mixture was stirred at -10 °C for 10 minutes. Then, I₂ was added (109mg, 0.86mmol) in a mixture solution and the mixture was further stirred for 1 hour, then quenched by addition of a solution of Na₂S2O₃ (1g) in H₂O (10mL). The quenched solution was extracted with CHCl₃ (15mL × 3), dried over Na₂SO₄, filtrated, and concentrated. Flash column chromatography (silica gel; hexane / ethyl acetate = 40/1) gave the title compound (169.7mg, 85% yield) as a colorless solid.

¹H-NMR (500MHz, CDCl₃) δ (ppm): 2.42 (s, 3H), 3.86 (s, 3H), 3.95 (s, 3H), 4.03 (s, 3H), 6.68 (s, 1 H), 7.40 (d, J = 1.7Hz, 1 H), 8.26 (d, J = 1.7Hz, 1 H), 9.36 (s, 1 H) ¹³C-NMR (125MHz, CDCl₃) δ (ppm):
16.3,52.4,56.5,61.6,109.3,109.4,115.8,116.7,126.9,129.5,130.4,149.1,151.9,155.2,167. 3
HRMS (EI) m / z [M] ⁺ (276.2845 calcd for C₁₅H₁₆O₅)

### (Production Example 10)

### 5-acetoxy-7-carbomethoxy-2,4-dimethoxy-2-methylnaphthalene

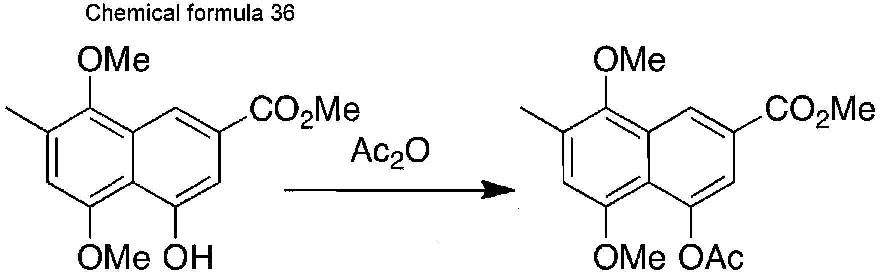

To a solution of the phenol compound (50mg, 0.18mmol) in pyridine (1.8mL), Ac₂O (19µL, 0.20mmol) and DMAP (2.2mg, 0.018mmol) were added under N₂ at room temperature, and the reaction mixture was stirred for 3 hours, then quenched with H₂O. The mixture was extracted with CHCl₃ (5mL × 3), dried over Na₂SO₄, filtrated, and concentrated. Flash column chromatography (silica gel; hexane / ethyl acetate = 20/1) gave the title compound (54.8mg, quantitative yield) as a colorless solid.

¹H-NMR (500MHz, CDCl₃) δ (ppm): 2.37 (s, 3H), 2.43 (s, 3H), 3.87 (s, 3H), 3.90 (s, 3H), 3.96 (s, 3H), 6.75 (s, 1H), 7.61 (d, J = 1.7Hz, 1H), 8.69 (d, J = 1.7Hz, 1H) ¹³C-NMR (125MHz, CDCl₃) δ (ppm):
16.4,21.0,52.5,56.5,61.8,111.9,118.3,120.6,123.3,127.8,128.0,130.6,147.1,148.5,151. 0,166.6,170.2
HRMS (EI) m / z [M] ⁺ (318.3212 calcd for C₁₇H₁₈O₆)

### (Production Example 11)

### 5-acetoxy-7-carbomethoxy-2-methyl-1,4-naphthoquinone

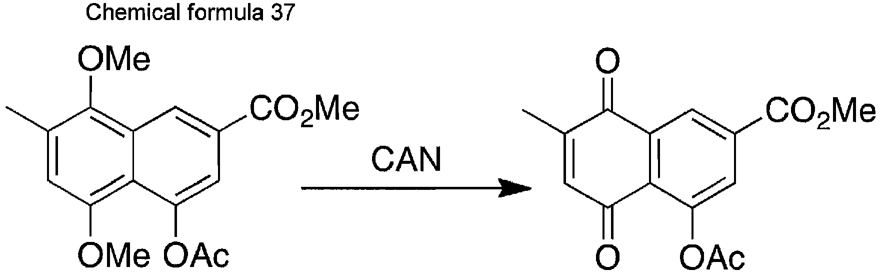

By the procedure of preparation of KIS-077, the acetate compound (51.9mg, 0.17mmol) was changed into a pale yellow solid of the title compound (41.9mg, 90% yield) (purification; silica gel; hexane / ethyl acetate = 20/1).

¹H-NMR (500MHz, CDCl₃) δ (ppm): 2.18 (d, J = 1.8Hz, 3H), 2.44 (s, 3H), 3.98 (s, 3H), 6.75 (d, J = 1.7Hz, 1H), 7.99 (d, J = 1.7Hz, 1H), 8.65 (d, J = 1.8Hz, 1H) ¹³C-NMR (125MHz, CDCl₃) δ (ppm):
16.3,21.1,53.1,126.0,126.3,130.2,134.2,125.8,137.2,147.6,149.6,164.6,169.3,183.1,18 4.1
HRMS (EI) m / z [M] ⁺ (288.2522 calcd for C₁₅H₁₂O₆)

### (Production Example 12)

### 7-carbomethoxy-5-hydroxy-2-methyl-1,4-naphthoquinone (KIS-079)

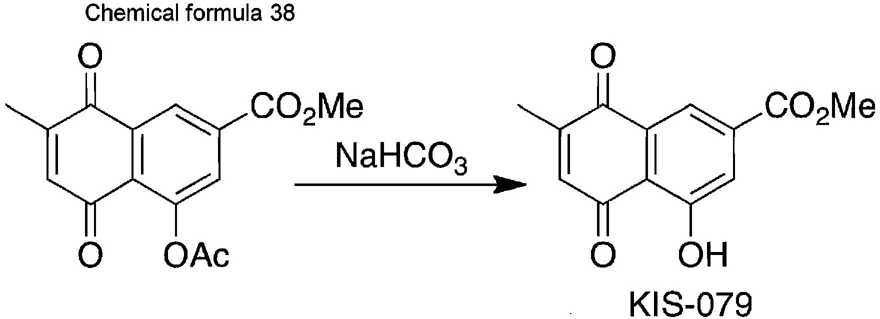

To a solution of the quinone acetate (40mg, 0.15mmol) in MeOH (7.5mL), NaHCO₃ aqueous solution (5mL) was added at room temperature. After stirring for 30 minutes, the solution was diluted by the addition of H₂O (5mL), and extracted with CHCl₃ (10mL × 3). The combined organic layers were dried over Na₂SO₄, filtrated, and concentrated. Flash column chromatography (silica gel; hexane / ethyl acetate = 40/1) gave KIS-079 (6.3mg, 17% yield) as a yellow solid.

¹H-NMR (500MHz, CDCl₃) δ (ppm): 2.22 (d, J = 1.7Hz, 3H), 3.97 (s, 3H), 6.86 (dd, J = 1.7,3 .2Hz, 1H), 7.90 (d, J = 1.8Hz, 1H), 8.24 (d, J = 1.8Hz, 1H), 11.9 (s, 1H) HRMS (EI) m / z 246.0526 [M] ⁺ (246.0528 calcd for C₁₃H₁₀O₅)

### (Production Example 13) 7-carbomethoxyl-5,6-dihydro--7H-2-methyl-5-oxo-1,4-naphthoquinone (KIS-103)

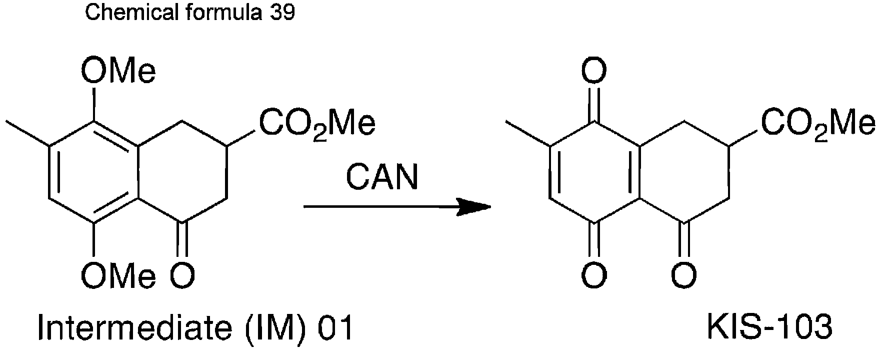

By the procedure of preparation of KIS-077, IM01 (250mg, 0.90mmol) was changed into a yellow solid of KIS-103 (102mg, 45% yield) (purification; silica gel; hexane / ethyl acetate = 40/1).

¹H-NMR (500MHz, CDCl₃) δ (ppm): 2.04 (d, J = 1.7Hz, 3H), 2.44 (dd, J = 11.5,15.5Hz, 1H), 2.48 (dd, J = 11.2,17.2Hz, 1H) 2.85 (ddd, J = 1.2,5.2,17.2Hz, 1H), 2.96 (m, 1H), 3.02 (ddd, J = 1.2,4.6,15.5Hz, 1H), 3.74 (s, 3H), 6.47 (d, J = 1.7Hz, 1H) ¹³C-NMR (125MHz, CDCl₃) δ (ppm):

### (Production Example 14) 7-carboxy-5-hydroxy-2-methyl-1,4-naphthoquinone (KIS-090)

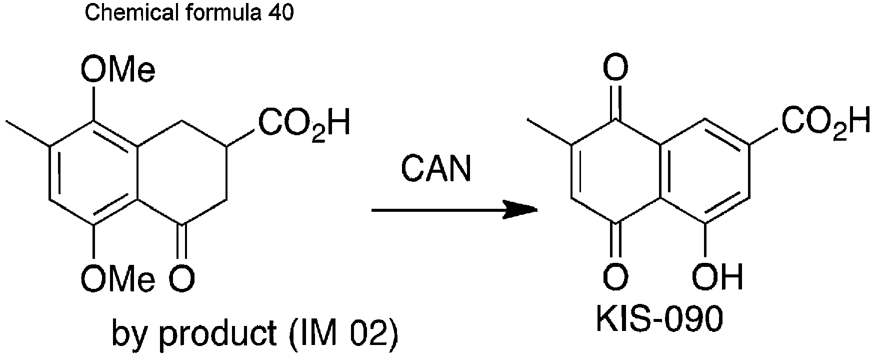

By the procedure of preparation of KIS-077, IM02 (30mg, 0.11mmol) was changed into a yellow solid of the title compound KIS-090 (9.5mg, 10% yield) (purification; silica gel; CHCl₃ / MeOH = 50/1).

¹H-NMR (500MHz, CDCl₃) δ (ppm): 2.19 (d, J = 1.8Hz, 3H), 6.81 (d, J = 1.8Hz, 1 H), 7.61 (d, J = 1.8Hz, 1 H), 7.63 (d, J = 1.8Hz, 1 H), 12.0 (s, 1 H)
¹³C-NMR (125MHz, CDCl₃) δ (ppm)
HRMS (EI) m / z [M] ⁺ (232.0372 calcd for C₁₂H₈O₅)

### (Production Example 15)

### 7-carbomethoxyl-5,6,7,8-tetrahydro-2-methyl-1,4-naphthoquinone (KIS-102)

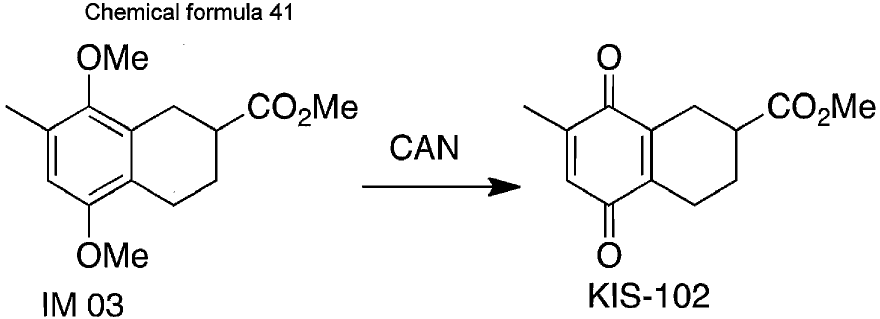

By the procedure of preparation of KIS-077, IM03 (100mg, 0.40mmol) was changed into a yellow solid of the title compound KIS-102 (46mg, 52% yield) (purification; silica gel; hexane / ethyl acetate = 40/1).

¹H-NMR (500MHz, CDCl₃) δ (ppm): 1.75 (m, 1H), 2.04 (d, J = 1.7Hz, 3H), 2.09 (m, 1H), 2.04 (m, 1H), 2.64 (m, 3H), 2.80 (m, 1H), 3.72 (s, 3H), 6.55 (dd, J = 1.7, 3.4Hz, 1H)
HRMS (EI) m / z [M] ⁺ (232.0372 calcd for C₁₂H₈O₅)

### (Production Example 16) 6-carboxyl-1,4-dimethoxy-2-methylnaphthalene (IM05)

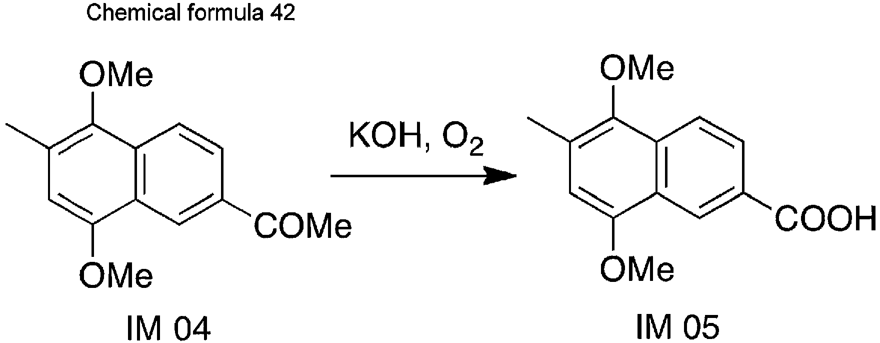

To a solution of IM04 (200mg, 0.82mmol) in DMF (20mL), powdered KOH (260mg, 4.60mmol) was added under O₂ at room temperature, the reaction mixture was warmed to 68 °C, stirred for 3 hours, then poured into the cooled water (20mL). The mixture was washed with Et₂O (20mL × 2), and the aqueous solution was acidified by the addition of 4N aqueous HCl (10mL). Then the mixture was extracted with CHCl₃ (20mL × 3), dried over Na₂SO₄, filtrated, and concentrated to give a pure IM05 (145mg, 72% yield) as a pale yellow solid.

¹H-NMR (500MHz, DMSO) δ (ppm): 2.50 (s, 3H), 3.79 (s, 3H), 3.98 (s, 3H), 6.91 (s, 1 H), 8.00 (dd, J = 1.8, 8.6Hz, 1 H), 8.03 (dd, J = 1.8 8.6Hz, 1 H), 8.74 (s, 1 H) ¹³C-NMR (125MHz, DMSO) δ (ppm):
16.2,55.8,61.0,108.5,121.6,123.4,124.6,126.0,126.5,129.2,129.9,146.0,151.6,167.4 HRMS (EI) m / z [M] ⁺ (246.0892 calcd for C₁₄H₁₄O₄)

### (Production Example 17) 6-carbomethoxy-1,4-dimethoxy-2-methylnaphthalene (IM06)

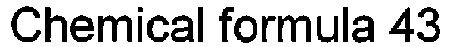

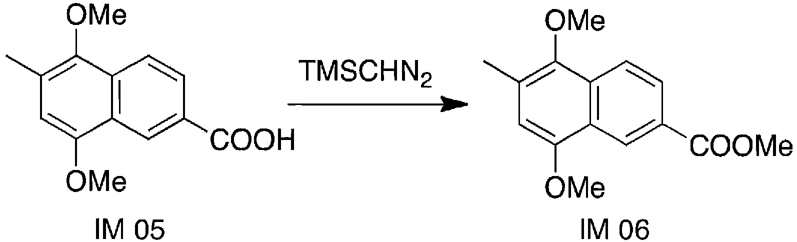

To a solution of IM05 (300mg, 1.22mmol) in benzene / MeOH (10 /1 v / v) (24.4mL), a solution of TMS- diazomethane in hexane (2.0M, 1.22mL, 2.44mmol) was added under N₂ at 0 °C, and the reaction mixture was stirred for 3 hours. After consuming all of the starting material, AcOH was added to the reaction mixture, and the thus obtained mixture was concentrated. Flash column chromatography (silica gel; hexane / ethyl acetate = 20/1) gave the IM06 (known compound) (289mg, 91% yield) as a pale orange solid.

### (Production Example 18) 6-hydroxymethyl-1,4-dimethoxy-2-methylnaphthalene (IM07)

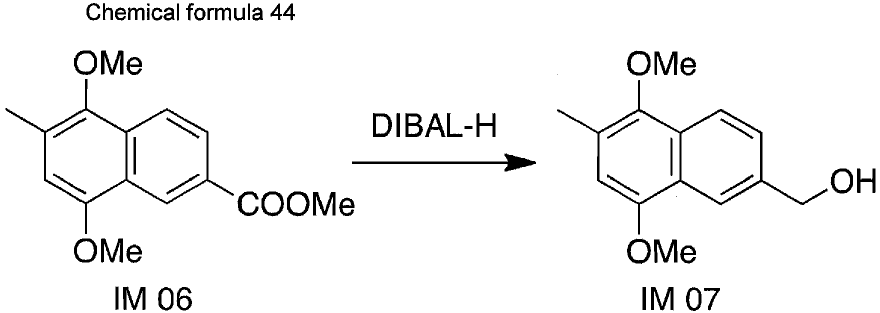

To s solution of IM06 (2.61g, 10mmol) in THF (100mL), a solution of diisobutylaluminum hydride in hexane (1.0M, 30mL, 30mmol) was added at -78 °C and the reaction mixture was stirred at -78 °C for 3 hours. Then, the reaction was quenched by dropwise addition of H₂O (5mL), was warmed slowly to room temperature with stirring. The mixture thus obtained was added a saturated Rochelle salt solution (150mL), stirred for further 1 hour, then the mixture was extracted with CHCl₃ (200mL × 2). The combined organic layers 1.0N aqueous HCl solution (100mL × 1), washed with saturated aqueous NaHCO₃ (100mL × 1) and brine (100mL × 1), dried over Na₂SO₄, filtrated, and concentrated. Flash column chromatography (silica gel; hexane / ethyl acetate = 5/1) gave the IM07 (1.94g, 83% yield) as a colorless solid.

¹H-NMR (500MHz, CDCl₃) δ (ppm): 1.71 (br, 1H), 2.44 (s, 3H), 3.85 (s, 3H), 3.97 (s, 3H), 4.84 (s, 2H), 6.61 (s, 1H), 7.53 (dd, J = 1.7,8.6Hz, 1H), 8.02 (d, J = 8.6Hz, 1H), 8.16 (d, J = 1.7Hz, 1 H)
¹³C-NMR (125MHz, CDCl₃) δ (ppm):
16.8,55.9,61.5,108.2,122.8,123.4,124.5,129.9,130.7,131.7,133.0,147.3,152.7,192.5 HRMS (EI) m / z [M] ⁺ (230.0943 calcd for C₁₄H₁₄O₃)

### (Production Example 19) 6-formyl-1,4-dimethoxy-2-methyl naphthalene

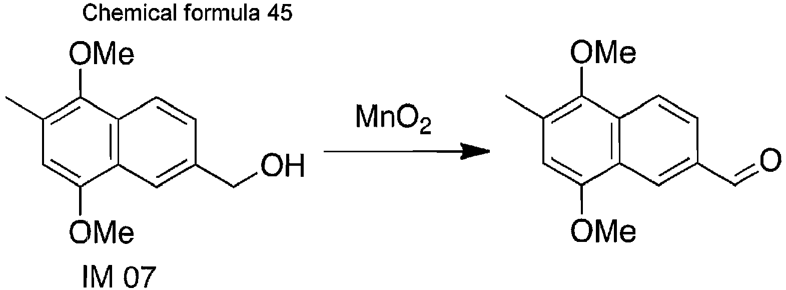

To a solution of IM07 (100mg, 0.43mmol) in CH₂Cl₂ (4.3mL), MnO₂ (374mg, 4.3mmol) was added at room temperature, the reaction mixture was sonicated for 7 hours. Then the reaction mixture was filtrated to remove MnO₂, and the filtrate was concentrated. Flash column chromatography (silica gel; CHCl₃) gave the title compound (76.4mg, 77% yield) as a pale yellow solid.

¹H-NMR (500MHz, CDCl₃) δ (ppm): 2.48 (s, 3H), 3.86 (s, 3H), 4.01 (s, 3H), 6.69 (s, 1H), 7 .97 (dd, J = 1.7, 8.6Hz, 1H), 8.10 (d, J = 8.6, 1H), 8.69 (d, J = 1.7Hz, 1H), 10. 12 (s, 1H)
¹³C-NMR (125MHz, CDCl₃) δ (ppm):
16.8,55.9,61.5,108.2,122.8,123.4,124.5,129.9,130.7,131.7,133.0,147.3,152.7,192.5 HRMS (EI) m / z [M] ⁺ (230.0943 calcd for C₁₄H₁₄O₃)

### (Production Example 20) 6-formyl-2-methyl-1,4-naphthoquinone (KIS-097)

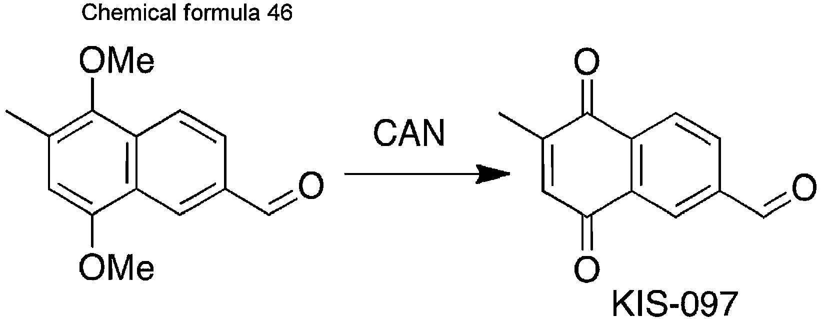

By the procedure of preparation of KIS-077, the aldehyde compound (100mg, 0.43mmol) was changed into a yellow solid of KIS-097 (74.2mg, 86% yield) (purification; silica gel; hexane / ethyl acetate =20/1).

¹H-NMR (500MHz, CDCl₃) δ (ppm): 2.24 (d, J = 1.7Hz, 3H), 6.95 (d, J = 1.7Hz, 1H), 8.24 (dd, J = 1.7, 8.0Hz, 1H), 8.27 (d, J = 8.0H, 1H), 8.56 (d, J =1.7Hz, 1H), 10.18 (s, 1H)
¹³C-NMR (125MHz, CDCl₃) δ (ppm):
16.7,127.6,128.4,133.0,125.6,126.2,139.7,148.9,183.9,185.0,190.9 (x2)
HRMS (EI) m / z 200.0473 [M] ⁺ (200.0473 calcd for C₁₂H₈O₃)

### (Production Example 21) 2-methyl-6-{[(2-propenyloxy) imino]methyl}-1,4-naphthoquinone (KIS-104)

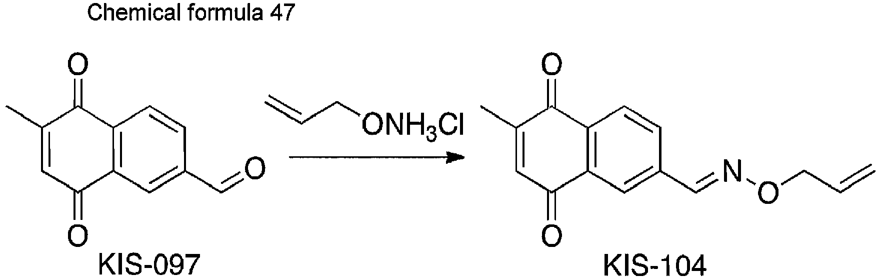

To a solution of KIS-097 (50mg, 0.25mmol) in CH₂Cl₂ (5mL), O-ally hydroxylamine·HCl (41.1mg, 0.375mmol) was added under N₂ at room temperature. The reaction mixture was stirred at room temperature for 3 hours, then quenched by addition of H₂O (5mL). The mixture was extracted with CHCl₃ (5mL × 3), and the combined organic layers were dried over Na₂SO₄, filtrated, and concentrated. Flash column chromatography (silica gel; CHCl₃) gave KIS-104 (73.6mg, 74% yield) as a pale yellow solid.

¹H-NMR (500MHz, CDCl₃) δ (ppm): 2.19 (d, J = 1.2Hz, 3H), 4.72 (ddd, J = 1.5,1.5,5.7Hz, 2H), 5.27 (ddd, J = 1.7,2.9,10.5Hz, 1H), 5.36 (ddd, J = 1.7,3.2,17.2Hz, 1H), 6.04 (m, 1H) 6.84 (d, J = 1.2Hz, 1H), 7.94, (dd, J = 1.7,8.0Hz, 1H), 8.06 (d, J = 8. 0Hz, 1H), 8.17 (ddd, J = 1.7,3.4,3.4Hz, 2H)
¹³C-NMR (125MHz, CDCl₃) δ (ppm):
16.6,75.9,118.6,125.0,127.2,131.2,132.5,132.7,133.7,135.8,137.6,147.1,148.6,184.6,1 85.1
HRMS (EI) m / z 255.0892 [M] ⁺ (255.2686 calcd for C₁₅H₁₃NO₃)

### (Production Example 22) 6-acetyl-2-methyl-1,4-naphthoquinone (KIS-091)

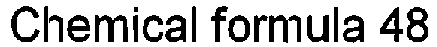

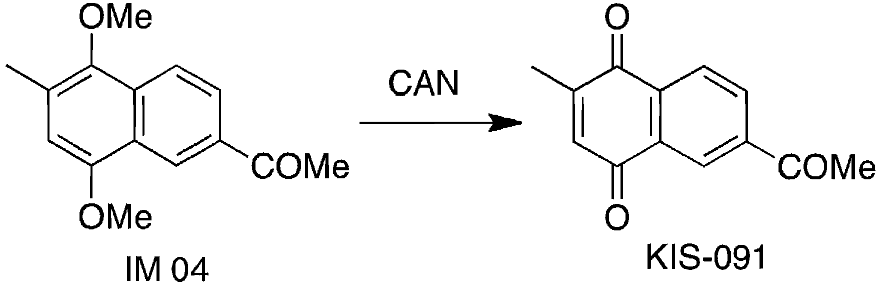

By the procedure of preparation of KIS-077, IM04 (100mg, 0.41 mmol) was changed into (purification; silica gel; hexane / ethyl acetate = 20/1) KIS-091 (known compound) (56.0mg, 64% yield) as a pale yellow solid.

### (Production Example 23) 6-ethyl-1,4-dimethoxy-2-methyl naphthalene and (±)-6-(1-hydroxyethyl)-1,4-dimethoxy-2-methylnaphthalene

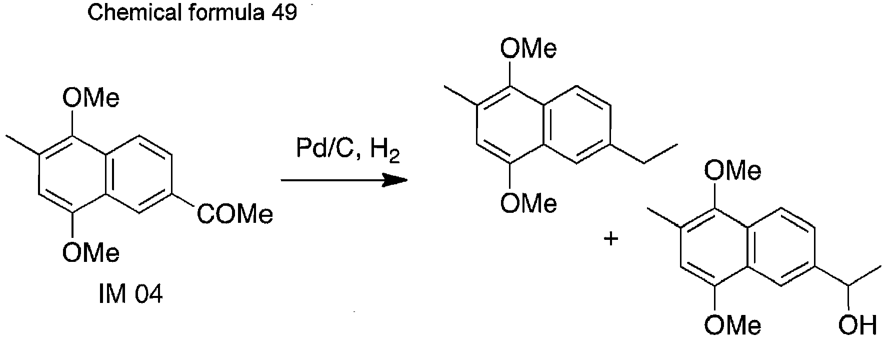

To a solution of IM04 (300mg, 1.23mmol) in MeOH (12.3mL), 0.5% Pd / C (150mg) was added at room temperature. The mixture was stirred under H₂ at room temperature. After stirring for 1 hour, the solution was filtrated through Celite (registered trademark) to remove the Pd / C, and the filtrate was concentrated. Flash column chromatography (silica gel; CHCl₃ / MeOH = 40/1), gave the title compound (ethyl) (140mg, 49% yield) as a colorless solid and further the title compound (hydroxyethyl) (152mg, 50% the yield) as a colorless solid.

¹H-NMR (500MHz, CDCl₃) δ (ppm): 1.55 (d, J = 6.3Hz, 3H), 2.42 (s, 3H), 3.83 (s, 3H), 3.94 (s, 3H), 5.05 (dd, J = 6.3 12.6Hz, 1 H), 6.06 (s, 1 H), 7.54 (dd, J = 1.7,8.6Hz, 1H), 8.00 (d, J = 8.6Hz, 1H), 8.15 (d, J = 1.7Hz, 1H)
¹³C-NMR (125MHz, CDCl₃) δ (ppm)
HRMS (EI) m / z [M] ⁺ (232.0372 calcd for C₁₂H₈O₅)

### (Production Example 24) 6-ethyl-2-methyl-1,4-naphthoquinone (KIS-094)

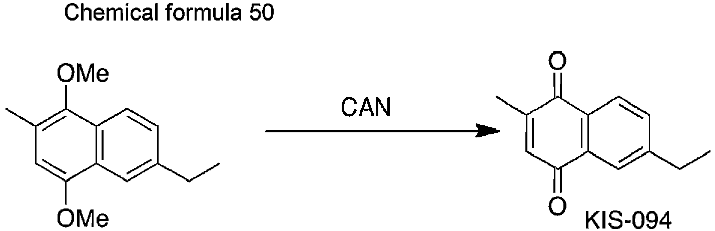

By the procedure of preparation of KIS-077, the dimethoxynaphthalene (100mg, 0.43mmol) was changed into (purification; silica gel; hexane / ethyl acetate = 20/1) KIS-094 (known compound) (75.0mg, 87 % yield) a pale yellow solid.

### (Production Example 25) (±)-2- methyl-6-(1-hydroxyethyl)-1,4-naphthoquinone (KIS-092)

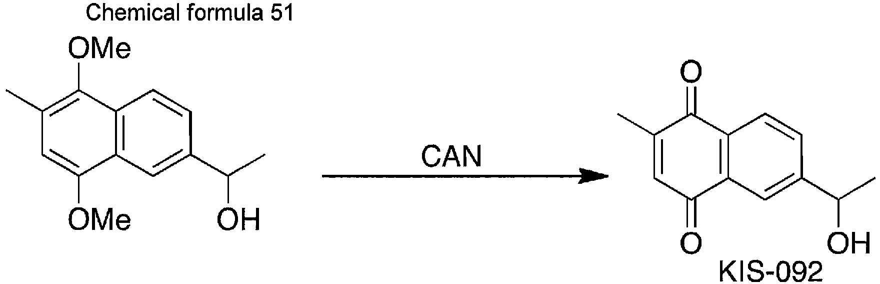

By the procedure of preparation of KIS-077, the dimethoxynaphthalene (85mg, 0.37mmol) was changed into (silica gel; hexane / ethyl acetate = 40/1) KIS-092 (57.6mg, 74% yield) as a pale yellow solid.

¹H-NMR (500MHz, CDCl₃) δ (ppm): 1.51 (d, J = 6.3Hz, 3H), 2.15 (d, J = 1.2Hz, 3H), 2.62 (br, 1H), 5.00 (dd, J = 6.3,12.9Hz, 1H), 6.77 (d, J = 1.2Hz, 1H), 7.71 (dd, J = 1.7,7. 5Hz, 1 H), 7.96 (d, J = 1.7Hz, 1 H), 8.00 (d, J = 7.5Hz, 1 H)
¹³C-NMR (125MHz, CDCl₃) δ (ppm):
16.6,25.4,69.8,123.1,127.1,130.6,131.3,132.4,135.7,148.4,152.3,185.2,185.4
HRMS (EI) m / z [M] ⁺ (232.0372 calcd for C₁₂H₈O₅)

### (Production Example 26) 6-carboxyl-2-methyl-1,4-naphthoquinone (KIS-093)

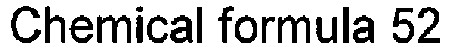

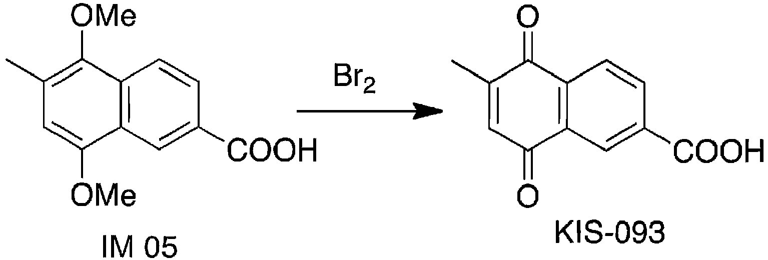

To a solution of IM05 (150mg, 0.61mmol) in 90% aqueous CH₃COOH (10mL), bromine (47µL, 1.82mmol) was added at room temperature, the mixture was stirred at room temperature. After stirring for 1 hour, the solution was diluted with H₂O (10mL), was collected insoluble solids by filtration. The collected solid was washed with MeOH (10mL × 3) and dried in vacuo to give KIS-093 (the known compound) (81 mg, 61% yield) as a pale yellow solid.

### (Production Example 27) 6-carbomethoxy-2-methyl-1,4-naphthoquinone (KIS-095)

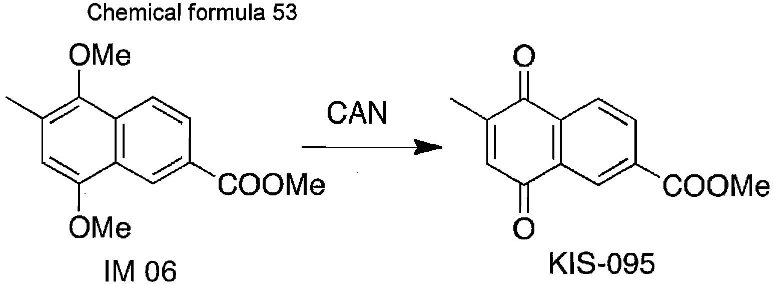

By the procedure of preparation of KIS-077, IM06 (91mg, 0.35mmol) was changed into (purification; silica gel; hexane / ethyl acetate = 10/1) KIS-095 (known compounds) (55.0mg, 68% yield) as a pale yellow solid.

### (Production Example 28) 6-hydroxymethyl-2-methyl-1,4-naphthoquinone (KIS-096)

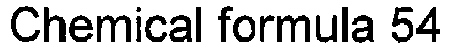

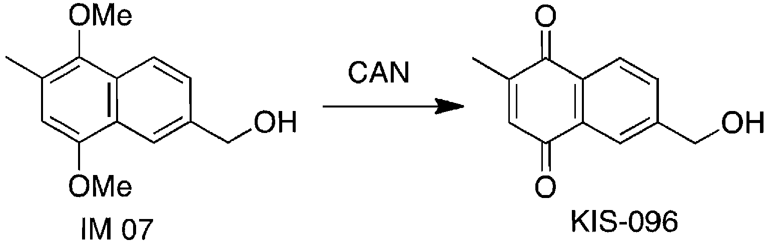

By the procedure of preparation of KIS-077, IM07 (100mg, 0.43mmol) was changed into (purification; silica gel; CHCl₃) KIS-096 (known compound) (69.5mg, 80% yield) as a pale yellow solid.

### (Production Example 29) Glycine,

### N-[(1,4-dimethoxy-2-methyl-6-naphthylenyl)carbonyl]-, tert-butyl ester

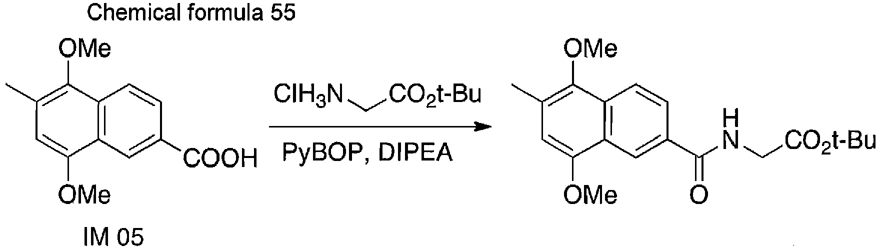

To a solution of IM05 (300mg, 1.22mmol) in CH₂Cl₂ (24.4mL), glycine tert- butyl ester hydrochloride (225mg, 1.34mmol), DIPEA (0.53mL, 3.05mmol) and PyBOP (760mg, 1.46mmol) were added at room temperature, and the reaction mixture was stirred at room temperature. After stirring for 10 minutes, the reaction mixture was cooled to 0 °C, then quenched by addition of MeOH (1 mL) and H₂O (10mL). Thus the resulting two-phase mixture was separated and the aqueous layer was extracted with CHCl₃ (15mL × 3). The combined organic layers were dried over Na₂SO₄, filtrated, and concentrated. Flash column chromatography (silica gel CHCl₃ / MeOH = 100/1) gave the title compound (231.6mg, 53%) as a pale yellow solid.

¹H-NMR (500MHz, CDCl₃) δ (ppm): 1.52 (s, 9H), 2.46 (s, 3H), 3.85 (s, 3H), 3.98 (s, 3H), 4.20 (d, J = 5.2Hz, 2H), 6.64 (s, 1H), 6.82 (br, 1H), 7.95 (dd, J = 1.8, 8.6Hz, 1H), 8.06 (d, J = 9.1 Hz, 1 H), 8.64 (d, J = 1.7Hz, 1 H)
¹³C-NMR (125MHz, CDCl₃) δ (ppm): 16.6,28.2 (x3),
42.8,55.7,61.5,82.6,107.7,121.8,122.3,124.5,124.9,128.5,129.9,130.2,147.0,152.3,167. 6,169.5
HRMS (FAB) m / z 359.1734 [M] ⁺ (359.1733 calcd for C₂₀H₂₅NO₅)

### (Production Example 30) Glycine,

### N-[(1,4-dihydro-1,4-dioxo-2-methyl-6-naphthylenyl)carbonyl] -, tert-butyl ester (KIS-098)

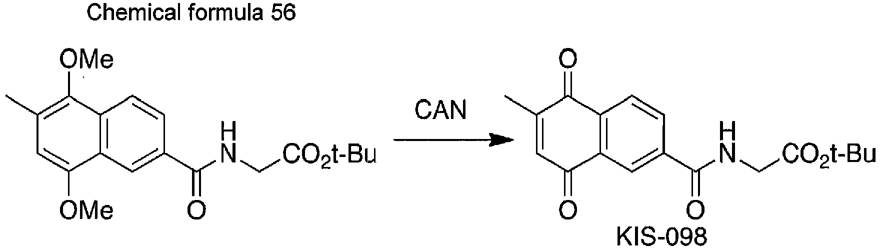

To a solution of the dimethoxynaphthalene (200mg, 0.55mmol) in MeCN (3.6ml), CAN (930.5mg, 1.69mmol) in H₂O (3.6mL) was added of at room temperature, and the reaction mixture was stirred for 10 minutes. The mixture was then diluted with H₂O (5mL), and extracted with CHCl₃ (10mL × 3). The combined organic layers were dried over Na₂SO₄, filtrated, and concentrated. Flash column chromatography (silica gel; acetate / hexane 1/15) gave KIS-098 (4.01g, 80% yield) as a pale yellow solid.

¹H-NMR (500MHz, CDCl₃) δ (ppm): 1.51 (s, 9H), 2.22 (d, J = 1.5Hz, 3H), 4.17 (d, J = 5.5Hz, 2H), 6.84 (br, 1 H), 6.90 (d, J = 2.0Hz, 1 H), 8.17 (d, J = 8.5Hz, 1 H), 8.22 (dd, J = 2.0,8.0Hz, 1 H), 8.40 (d, J = 1.5Hz, 1 H)
¹³C-NMR (125MHz, CDCl₃) δ (ppm): 16.6,28.2 (x3),
42.8,83.0,124.3,127.3,132.4,132.7,134.1,136.1,138.6,148.7,165.5,169.0,184.2,185.0 HRMS (FAB) m / z 330.1346 [M + H] ⁺ (330.1341 calcd for C₁₈H₂₀NO₅)

### (Production Example 31)

### N-[(1,4-dihydro-1,4-dioxo-2-methyl-6-naphthylenyl)carbonyl]glycine (KIS-099)

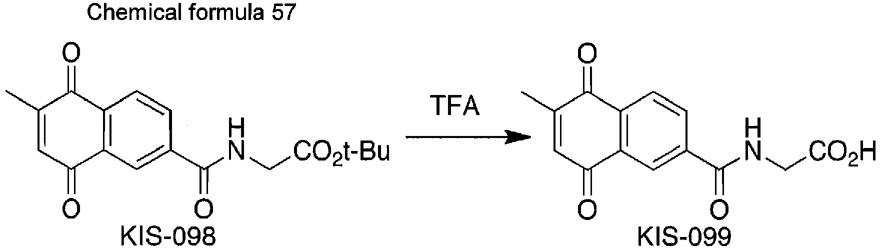

KIS-098 (100mg, 0.30mmol) was dissolved in 50% TFA in CH₂Cl₂ (30mL), and thus obtained solution was stirred at room temperature. After stirring for 30 minutes, the mixture was concentrated. Flash column chromatography (silica gel; CHCl₃ / MeOH = 20/1), gave KIS-099 (79.4mg, 97%) as a light brownish yellow solid.

¹H-NMR (500MHz, CDCl₃) δ (ppm): 1.54 (s, 9H), 2.25 (d, J = 1.7Hz, 3H), 4.20 (d, J = 5.2Hz, 2H), 6.84 (br, 1 H), 6.93 (d, J = 1.7Hz, 1 H), 8.20 (d, J = 8.6Hz, 1 H), 8.25 (dd, J = 1 .7, 8.6Hz, 1 H), 8.40 (d, J = 1.7Hz, 1 H)
¹³C-NMR (125MHz, CDCl₃) δ (ppm): 16.6,28.2 (x3),
42.8,83.0,124.3,127.3,132.4,132.7,134.1,136.1,138.6,148.7,165.5,169.0,184.2,185.0 HRMS (FAB) m / z 274.0722 [M + H] ⁺ (274.0715 calcd for C₁₄H₁₂NO₅)

### (Production Example 32)

### 2-N-[(1,4-dimethoxy-2-methyl-6-naphthylenyl)carbonyl]-3-tert-butoxy-tert-butyl ester

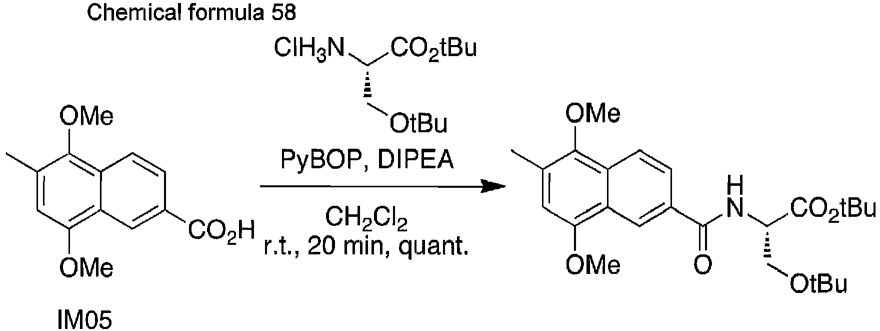

To a solution of IM05 (300mg, 1.22mmol) in CH₂Cl₂ (24.4mL), serine tert-butoxy -tert- butyl ester hydrochloride (340mg, 1.34mmol), DIPEA (0.53mL, 3.05mmol) and PyBOP (760mg, 1.46mmol) were added at room temperature, and the reaction mixture was stirred at room temperature. After stirring for 10 minutes, the reaction mixture was cooled to 0 °C, then quenched by addition of MeOH (1mL) and H₂O (10mL). Thus the resulting two-phase mixture was separated and the aqueous layer was extracted with CHCl₃ (15mL × 3). The combined organic layers were dried over Na₂SO₄, filtrated, and concentrated. Flash column chromatography (silica gel; hexane / EtOAc = 10/1) gave the title compound (543.1 mg, quantitative yield) as a colorless oil.

¹H-NMR (500MHz, CDCl₃) δ (ppm): 1.17 (s, 9H), 1.51 (s, 9H), 2.46 (s, 3H), 3.71 (dd, J = 2. 9,8.0Hz, 1 H), 3.85 (s, 3H), 3.90 (dd, J = 2.9,9.2Hz, 1 H), 3.98 (s, 3H), 4.88 (ddd, J = 8.0Hz, 1H), 6.64 (s, 1 H), 7.14 (d, J = 8.6Hz, 1 H), 7.94 (dd, J = 1.7,8.6Hz , 1H), 8.07 (d, J = 9.2Hz, 1 H), 8.67 (d, J = 1.7Hz, 1 H)
¹³C-NMR (125MHz, CDCl₃) δ (ppm):
HRMS (ESI) m / z 468.2354 [M + Na] ⁺ (468.2362 calcd for C₂₅H₃₅N₁Na₁O₆)

### (Production Example 33) 2-N-[(1,4-dihydro-1,4-dioxo-2-methyl-6-naphthylenyl) carbonyl] -3-tert- butoxy -tert- butyl ester

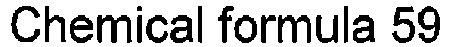

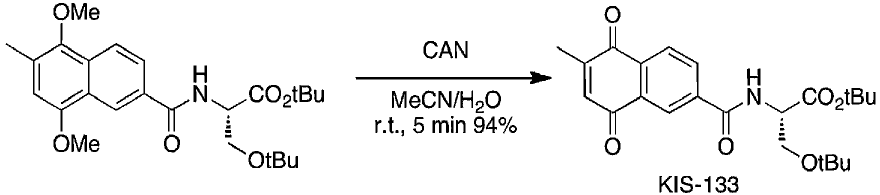

To a solution of the dimethoxynaphthalene (494mg, 1.11mmol) in MeCN (3.7ml), CAN (1.83g, 3.33mmol) in H₂O (3.7mL) was added at room temperature, and the reaction mixture is stirred for 5 minutes. The mixture was then diluted with H₂O (5mL), and extracted with CHCl₃ (10mL × 3). The combined organic layers were dried over Na₂SO₄, filtrated, and concentrated. Flash column chromatography (silica gel; hexane / EtOAc = 10/1) gave KIS-133 (435mg, 94% yield) as a pale yellow solid.

¹H-NMR (500MHz, CDCl₃) δ (ppm): 1.17 (s, 9H), 1.50 (s, 9H), 2.22 (d, J = 1.7Hz, 3H), 3.69 (dd, J = 2.9,8.9Hz, 1 H), 3.88 (dd, J = 2.9,8.6Hz, 1 H), 4.81 (ddd, J = 8.0,8.6, 8.9Hz, 1 H), 6.90 (d, J = 1.7Hz, 1 H), 7.10 (d, J = 8.0Hz), 8.19 (d, J = 8.0Hz, 1 H), 8.22 (dd, J = 1.7,8.0Hz, 1 H), 8.44 (d, J = 1.7Hz, 1 H)
¹³C-NMR (125MHz, CDCl₃) δ (ppm): 16.5,27.4 (x3), 28.1 (x3),
53.9,62.1,73.3,82.3,124.5,127.1,132.3,132.5,133.8,135.9,138.9,148.5,165.2,169.3,184. 1,184.9

### (Production Example 34) N-[(1,4-dihydro-1,4-dioxo-2-methyl-6-naphthylenyl)carbonyl] serine

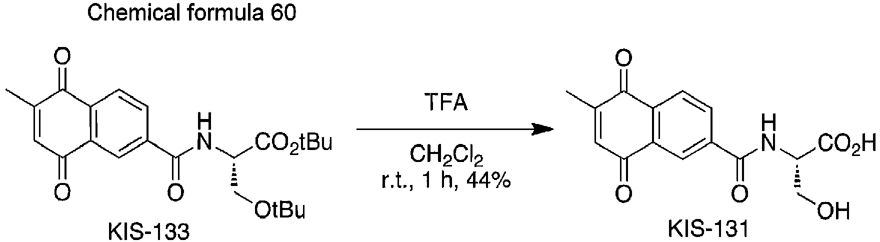

KIS-133 a (100mg, 0.24mmol) was dissolved in 50% TFA in CH₂Cl₂ (24mL), was thus obtained solution was stirred at room temperature. After stirring for 1 hour, the mixture was concentrated. Flash column chromatography (silica gel; CHCl₃ / MeOH = 10/1), gave KIS-131 (32mg, 44%) as a light brownish yellow solid.

¹H-NMR (500MHz, DMSO) δ (ppm): 2.14 (d, J = 1.7Hz, 3H), 3.82 (d, J = 5.2Hz), 4.52 (dt, J = 5.2,8.0Hz, 1H), 7.06 (d, J = 1.7,1H), 8.10 (d, J = 8.0Hz, 1H), 8.29 (dd, J = 1. 7,8.0Hz, 1H), 8.47 (d, J = 1.7Hz, 1H), 8.93 (d, J = 8.0Hz, 1H)
¹³C-NMR (125MHz, DMSO) δ (ppm):
16.0,55.9,61.0,124.6,126.4,131.8,132.6,133.5,135.5,148.4,165.0,171.6,184.2,184.6 HRMS (ESI) m / z 302.0666 [M-H]⁻ (302.0664 calcd for C₁₅H₁₂N₁O₆)

### (Production Example 35) N - [(1,4- dimethoxy-2-methyl-6-naphthylenyl) carbonyl] - benzyl -tert- butyl ester

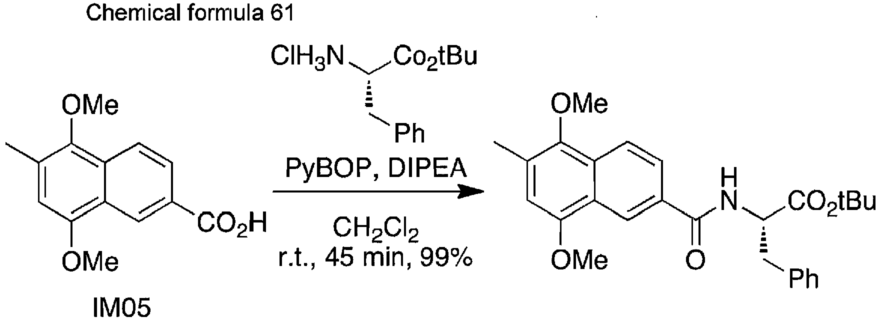

To a solution of IM05 (300mg, 1.22mmol) in CH₂Cl₂ (24.4mL), serine tert-butoxy-tert-butyl ester hydrochloride (345mg, 1.34mmol), DIPEA (0.53mL, 3.05mmol) and PyBOP (760mg, 1.46mmol) were added at room temperature, and the reaction mixture was stirred at room temperature. After stirring for 10 minutes, the reaction mixture was cooled to 0 °C, then quenched by addition of MeOH (1 mL) and H₂O (10mL). Thus the resulting two-phase mixture was separated and the aqueous layer was extracted with CHCl₃ (15mL × 3). The combined organic layers were dried over Na₂SO₄, filtrated, and concentrated. Flash column chromatography (silica gel; hexane / EtOAc = 10/1) gave the title compound (544.7mg, 99% yield) as a colorless solid.

¹H-NMR (500MHz, CDCl₃) δ (ppm): 1.46 (s, 9H), 2.46 (s, 3H), 3.27 (d, J = 5.7Hz, 2H), 3.85 (s, 3H), 3.98 (s, 3H), 5.03 (dt, J = 5.7,7.5Hz, 1H) 6.64 (s, 1H), 6.80 (d, J = 7.5Hz, 1H), 7.22-7.31 (complex m, 5H), 7.89 (dd, J = 1.7,8.6Hz, 1H), 8.06 (d, J = 8.6Hz, 1 H), 8.58 (d, J = 1.7Hz, 1 H)
¹³C-NMR (125MHz, CDCl₃) δ (ppm): 16.6, 28.7 (x3), 38.2, 54.1, 55.7, 61.4, 82.7, and 107.7,121.
9,122.3,124.5,124.7,127.1,128.4,128.5(x2),129.8(x2),130.1,1303,136.5,147.0, 152.3,167.0,171.0
HRMS (ESI) m / z 472.2093 [M + Na] ⁺ (472.2010 calcd for C₂₇H₃₁N₁Na₁O₅)

### (Production Example 36) N-[(1,4-dihydro-1,4-dioxo-2-methyl-6-naphthylenyl) carbonyl]-benzyl-tert-butyl ester

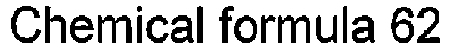

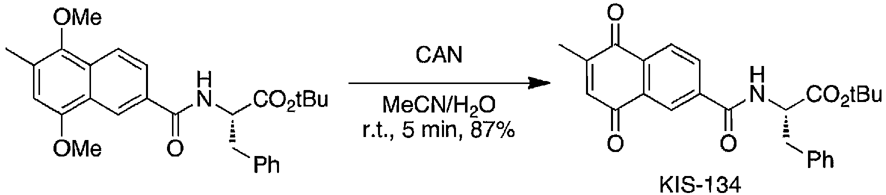

To a solution of the dimethoxynaphthalene (515mg, 1.14mmol) in MeCN (3.8ml), CAN (1.88g, 3.43mmol) in H₂O (3.8mL) was added at room temperature, and the reaction mixture was stirred for 5 minutes. The mixture was then diluted with H₂O (5mL), and extracted with CHCl₃ (10mL × 3). The combined organic layers were dried over Na₂SO₄, filtrated, and concentrated. Flash column chromatography (silica gel; hexane / EtOAc = 10/1) gave KIS-134 a (415mg, 87% yield) as a yellow solid.

¹H-NMR (500MHz, CDCl₃) δ (ppm):
¹³C-NMR (125MHz, CDCl₃) δ (ppm): 16.6,28.1(x3),
38.1,48.7,54.3,83.1,125.4,127.3,128.7(x2),129.7(x2),132.5(x2),134.0,136.1(x2), 138.9,148.6,165.0,170.6,184.1,185.0
HRMS (ESI) m / z 442.1628 [M + Na] ⁺ (442.1630 calcd for C₂₅H₂₅N₁Na₁O₅)

### (Production Example 37)

### N-[(1,4-dihydro-1,4-dioxo-2-methyl-6-naphthylenyl)carbonyl]phenylalanine

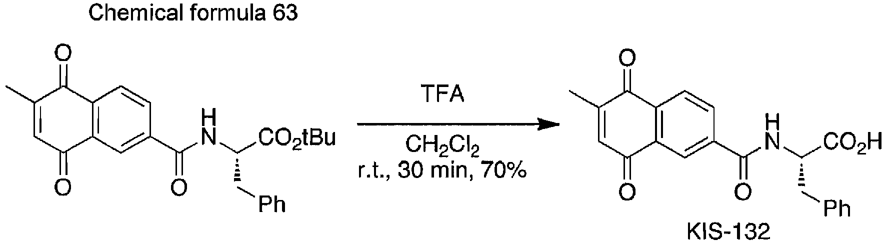

KIS-134 a (100mg, 0.24mmol) was dissolved in 50% TFA in CH₂Cl₂ (24mL), was thus obtained solution was stirred at room temperature. After stirring for 30 minutes, the mixture was concentrated. Flash column chromatography (silica gel; hexane / EtOAc = 10/1) gave KIS-132 (61.3mg, 70%) as a pale yellow solid.

¹H-NMR (500MHz, CDCl₃) δ (ppm): 2.22 (d, J = 1.1 Hz, 3H), 3.26 (dd, J = 5.7,14.2Hz, 1H), 3.39 (dd, J = 5.7,14.2Hz, 1H), 5.15 (ddd, J = 5.7,5.7,7.4Hz, 1H) 6.88 (d, J = 7.4Hz, 1H), 6.90 (d, J = 1.1Hz, 1H), 7.21-7.33 (complex m, 5H), 8.15 (dd, J = 1.7,7.4Hz, 1H), 8.17 (d, J = 7.4Hz, 1H), 8.33 (d, J = 1.7Hz, 1H)
¹³C-NMR (125MHz, CDCl₃) δ (ppm): 16.6,37.6,54.0,124.6,127.2,127.4,128.8(x2), 129.4(x2),132.2,133.0,134.0,135.9,136.0,138.2,148.9,166.0,174.7,184.5,184.8

### (Test Example 1) PDK4 enzyme inhibitory activity

Measurement of PDK4 / PDK2 inhibitory activity by off-chip mobility shift assay:
The compound solution (5µL), 4 × substrate / ATP / metal mixture (5µL) and 2 × recombinant human PDK4 (5µL) were added to 384 well micro plate. After reacting at room temperature for 5 hours, the reaction stop solution (60µL) was added to above solution. The substrate peptide (S) and phosphorylated peptide (P) was quantified using LabChip3000, then the inhibitory activity was calculated from the ratio (P / P + S).

The results are shown in the table below.

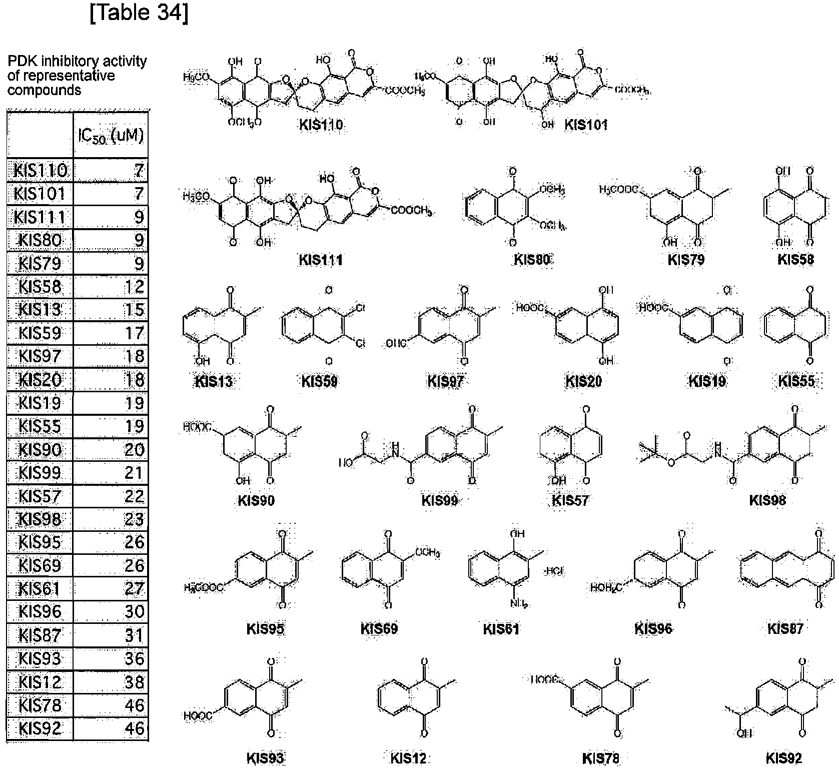

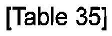

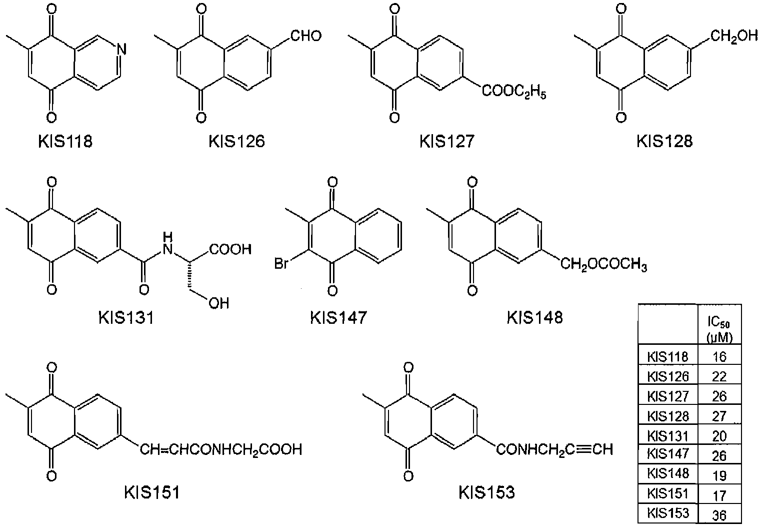

### (Test Example 2) The effect of the compounds of the present invention with influenza infection mouse model

### 1. Mouse, influenza virus, and reagents

Five weeks old C57BL/6J mice (Japan SLC) were purchased, and at 6 weeks old (16.4-18.1g) intramuscularly injected with anesthesia (mixture of KETALAR^{®} 62.5mg / kg and SELACTAR^{®} 12.5µg / kg), then trans-nasally infected with 10PFU / 20µl / mouse of Influenza A/Puerto Rico 8/34 strain (influenza A/PR8/8/34 strain). To an uninfected group (control), physiologic saline (Otsuka Pharmaceutical) used for dilution of the virus was trans-nasally administered with 20µl / mouse. The compounds of the present invention were intraperitoneally administered 0.93-0.8mg / kg / day dose, mixing with physiological saline so that a concentration of DMSO as a solvent is 5%, twice in a day from the next day of the infection. Ten mice (5 mice per a cage) for each group were used. Groups were the following three, uninfected group (control), infected mice group administered KIS compounds, and infected mice administered physiological saline 5% DMSO.

The results are shown in Figure 1. From these results, the compounds of the present invention have been shown to prolong the survival of influenza infected mice.

## Claims

1. A pyruvate dehydrogenase kinase inhibitor comprising a compound represented by the following general formula (I) or ester derivatives thereof, or pharmacologically acceptable salts thereof as an active ingredient, 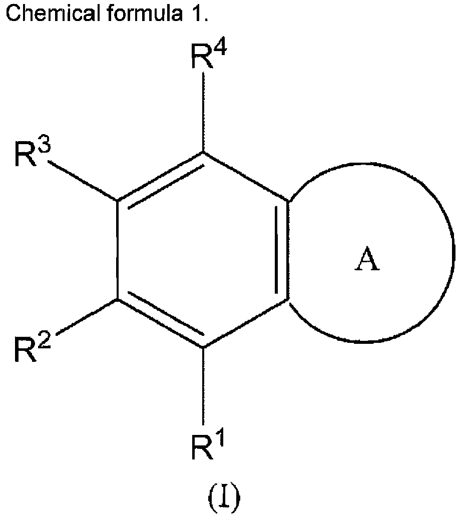 [wherein ring A represents a 6-membered aromatic hydrocarbon ring optionally substituted with 2-4 substituents,
wherein the substituents of the ring A, which are the same or different, represent a group selected from a hydroxyl group, an oxo group, an optionally substituted amino group, a halogen atom, an optionally substituted C1-6 alkyl group, an optionally substituted C2-40 alkenyl group, an optionally substituted C1-6 alkoxy group, an optionally substituted C2-6 alkenyloxy group, an optionally substituted C1-6 alkoxycarbonyl group, and an optionally substituted C2-7 alkanoyloxy group, or two substituents of the ring A may be bonded to form an optionally substituted dihydropyran (the dihydropyran may be condensed with tetrahydrofuran optionally substituted with an oxo group),
R¹ and R⁴, which are the same or different, represent a hydrogen atom, a hydroxyl group, an optionally substituted C1-6 alkyl group, an optionally substituted C2-6 alkenyl group, an optionally substituted C6-10 aryl group, an optionally substituted C1-6 alkoxy group, or an optionally substituted C2-7 alkanoyloxy group,
R² and R³, which are the same or different, represent a hydrogen atom, a carboxyl group, an optionally substituted C1-6 alkyl group, an optionally substituted C6-10 aryl group, or a group represented by -C (=R⁹)-R¹⁰,
wherein R⁹ represents an oxygen atom, a sulfur atom, a =N-R¹¹ group (wherein R¹¹ represents a hydroxyl group, an optionally substituted C1-6 alkyl group, an optionally substituted C6-10 aryl group, an optionally substituted C1-6 alkoxy group, an optionally substituted C2-6 alkenyloxy group, an optionally substituted C6-10 aryloxy group), or a =CH-R¹² group (wherein R¹² represents a formyl group, a carboxyl group, an optionally substituted C1-6 alkyl group, an optionally substituted C6-10 aryl group, an optionally substituted C1-6 alkoxycarbonyl group, an aminocarbonyl group, or an optionally substituted C1-6 alkylaminocarbonyl group),
R¹⁰ represents a hydrogen atom, an amino group, an optionally substituted C1-6 alkyl group, an optionally substituted C6-10 aryl group, an optionally substituted C1-6 alkoxy group, an optionally substituted C6-C10 aryloxy group, an optionally substituted C1-6 alkylamino group, or an optionally substituted C1-6 alkoxycarbonyl C1-6 alkylamino group, or
one of R² and R³ represents a group represented by the following general formula (wherein the definition of ring A, and groups represented by R¹, R² and R⁴ is,
respectively, the same as the definition of ring A, R¹, R² and R⁴ in the general formula (I)): and 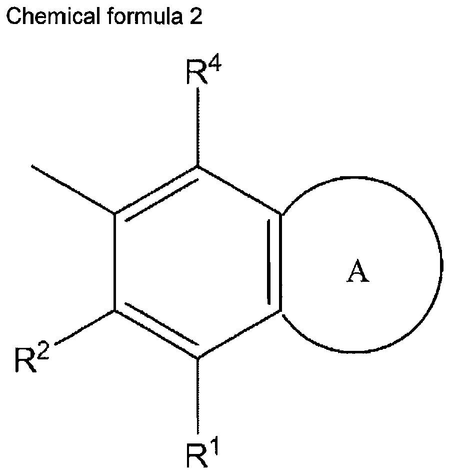 the other represents a hydrogen atom, a carboxyl group, an optionally substituted C1-6 alkyl group, a C6-10 aryl group, or a group represented by -C (=R⁹)-R¹⁰,
R² and R³ are taken together with the carbon atom to which they are attached to form a benzene ring or tetrahydrofuran, wherein the tetrahydrofuran may be spiro-linked with an optionally substituted tricyclic condensed heterocyclic ring,
wherein, in the above, the substituent in case being optionally substituted is a group selected from the following: a hydroxyl group, a carboxyl group, an amino group, a halogen atom, a C1-6 alkyl group optionally substituted with a hydroxyl group, a C2-6 alkenyl group, a C2-7 alkanoyl group, a C1-6 alkoxy group, a C1-6 alkoxycarbonyl group, a C6-10 aryl group, a C6-10 aryl C1-6 alkyl group].

2. The pyruvate dehydrogenase kinase inhibitor according to claim 1 comprising a compound represented by the following general formula (II) or (III) or ester derivatives thereof, or pharmacologically acceptable salts thereof as an active ingredient, 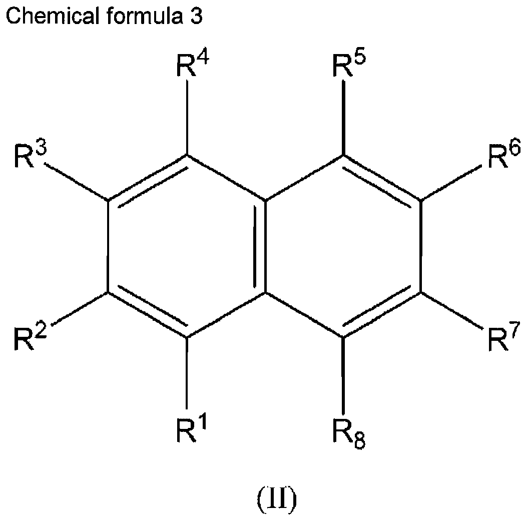 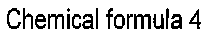 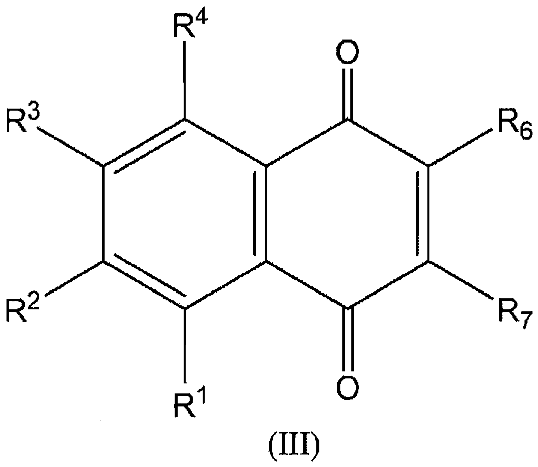 [wherein R¹ and R⁴, which are the same or different, represent a hydrogen atom, a hydroxyl group, an optionally substituted C1-6 alkyl group, an optionally substituted C2-6 alkenyl group, an optionally substituted C6-10 aryl group, an optionally substituted C1-6 alkoxy group, or an optionally substituted C2-7 alkanoyloxy group,
R² and R³, which are the same or different, represent a hydrogen atom, a carboxyl group, an optionally substituted C1-6 alkyl, a C6-10 aryl group, or a group represented by -C (=R⁹)-R¹⁰,
wherein R⁹ represents an oxygen atom, a sulfur atom, a =N-R¹¹ group (wherein R¹¹ represents a hydroxyl group, a C1-6 alkyl group, a C6-10 aryl group, a C1-6 alkoxy group, a C2-6 alkenyloxy group, a C6-10 aryloxy group), or a =CH-R¹² group (wherein R¹² represents a formyl group, a carboxyl group, a C1-6 alkyl group, a C6-10 aryl group, a C1-6 alkoxycarbonyl group, an aminocarbonyl group, or a C1-6 alkylaminocarbonyl group),
R¹⁰ represents a hydrogen atom, an amino group, a C1-6 alkyl group, a C6-10 aryl group, a C1-6 alkoxy group, an optionally substituted C1-6 alkylamino group, or an optionally substituted C1-6 alkoxycarbonyl C1-6 alkylamino group, or
one of R² and R³ represents a group represented by the following general formula (wherein the definition of ring A, and groups represented by R¹, R² and R⁴ is, respectively, the same as the definition of ring A, R¹, R² and R⁴ in the general formula (I)): or 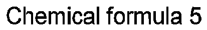 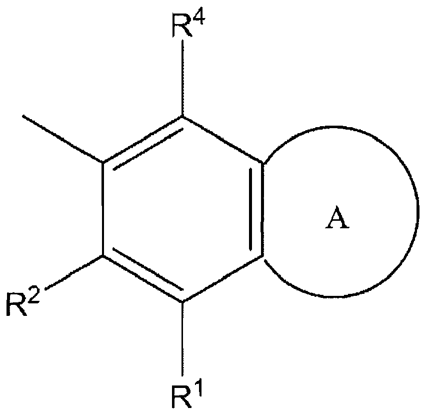 R² and R³ are taken together with the carbon atom to which they are attached to form a benzene ring or tetrahydrofuran, wherein the tetrahydrofuran may be spiro-linked with an optionally substituted tricyclic condensed heterocyclic ring,
R⁵ and R⁸, which are the same or different, represent a hydroxyl group, an amino group, an optionally substituted C1-6 alkoxy group, an optionally substituted C2-40 alkenyloxy group, or a C2-7 alkanoyloxy group,
R⁶ and R⁷ represent a hydrogen atom, a hydroxyl group, an optionally substituted amino group, a halogen atom, an optionally substituted C1-6 alkyl group, an optionally substituted C2-40 alkenyl, or an optionally substituted C1-6 alkoxy group,
wherein, in the above, the substituent in case being optionally substituted is a group selected from the following: a hydroxyl group, a carboxyl group, an amino group, a halogen atom, a C1-6 alkyl group, a C2-6 alkenyl group, a C2-7 alkanoyl group, a C1-6 alkoxycarbonyl group,
wherein, in the above, the substituent in case being optionally substituted is a group selected from the following: a hydroxyl group, a carboxyl group, an amino group, a halogen atom, a C1-6 alkyl group optionally substituted with a hydroxyl group, a C2-6 alkenyl group, a C2-7 alkanoyl group, a C1-6 alkoxy group, a C1-6 alkoxycarbonyl group, a C6-10 aryl group, a C6-10 aryl C1-6 alkyl group].

3. A compound represented by the following general formula (II) or (III) or ester derivatives thereof, or pharmacologically acceptable salts thereof: 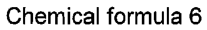 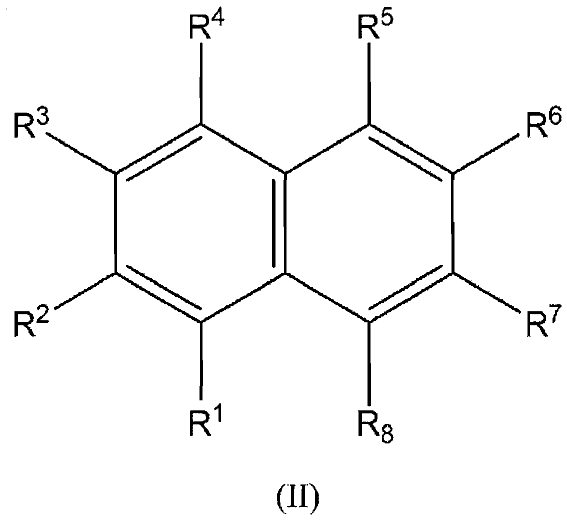 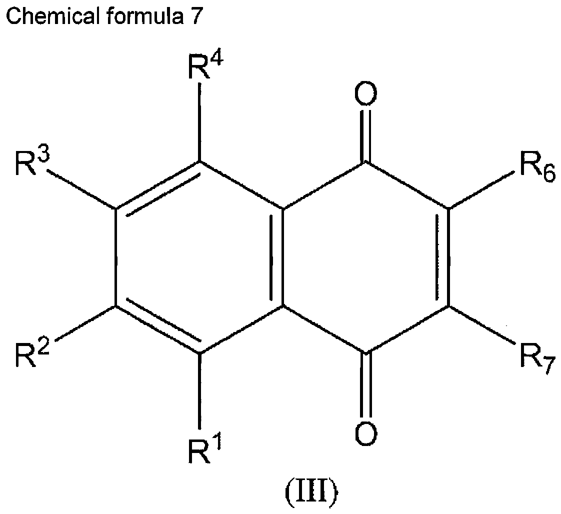 [wherein,
R¹ and R⁴, which are the same or different, represent a hydrogen atom, an optionally substituted C1-6 alkyl group, or an optionally substituted C6-10 aryl group,
one of R² and R³ represents a hydrogen atom, an optionally substituted C1-6 alkyl group, or an optionally substituted C6-10 aryl group,
the other represents a group represented by -C (=R⁹)-R¹⁰,
wherein, R⁹ represents an oxygen atom, a sulfur atom, a =N-R¹¹ group (wherein R¹¹ represents a C1-6 alkyl group, a C6-10 aryl group, a hydroxyl group, a C1-6 alkoxy group, a C2-20 alkenyloxy group, a C6-10 aryloxy group), or a =CH-R¹² group (wherein R¹² represents a C1-6 alkyl group, a C6-10 aryl group, a formyl group, a carboxyl group, a C1-6 alkoxycarbonyl group, an aminocarbonyl group, or a C1-6 alkylaminocarbonyl group),
R¹⁰ represents a hydrogen atom, an amino group, an optionally substituted C1-6 alkyl group, an optionally substituted C6-10 aryl group, an optionally substituted C1-6 alkoxy group, an optionally substituted C6-10 aryloxy group, an optionally substituted C1-6 alkylamino group, or an optionally substituted C1-6 alkoxycarbonyl C1-6 alkylamino group,
with the proviso that the group represented by -C (=R⁹)-R¹⁰ is not a carboxyl group, a methylcarbonyl group or a methoxycarbonyl group,
both R⁵ and R⁸ represent a C1-6 alkoxy group, and
R⁶ and R⁷ represent a hydrogen atom, an optionally substituted C1-6 alkyl group, or an optionally substituted amino group,
wherein, in the above, the substituent in case being optionally substituted is a group selected from the following: a hydroxyl group, a carboxyl group, an amino group, a halogen atom, a C1-6 alkyl group optionally substituted with a hydroxyl group, a C2-6 alkenyl group, a C2-7 alkanoyl group, a C1-6 alkoxy group, a C1-6 alkoxycarbonyl group, a C6-10 aryl group, a C6-10 aryl C1-6 alkyl group].

4. The compound according to claim 3 or ester derivatives thereof, or pharmacologically acceptable salts thereof,
wherein R¹ and R⁴, which are the same or different, represent a hydrogen atom, or an optionally substituted C1-6 alkyl group,
one of R² and R³ represents a hydrogen atom, or an optionally substituted C1-6 alkyl group,
the other represents a group represented by -C (=R⁹)-R¹⁰,
wherein R⁹ represents an oxygen atom, or a =N-R¹¹ group (wherein R¹¹ represents a C1-6 alkoxy group, a C2-6 alkenyloxy group, a C6-10 aryloxy group),
R¹⁰ represents a hydrogen atom, an optionally substituted C1-6 alkyl group, an optionally substituted C6-10 aryl group, an optionally substituted C1-6 alkoxy group, an optionally substituted C6-10 aryloxy group, an optionally substituted C1-6 alkylamino group, or an optionally substituted C1-6 alkoxycarbonyl C1-6 alkylamino group,
with the proviso that the group represented by -C (=R⁹)-R¹⁰ is not a carboxyl group, a methylcarbonyl group or a methoxycarbonyl group,
both R⁵ and R⁸ represent a C1-6 alkoxy group,
R⁶ and R⁷ represent a hydrogen atom, or an optionally substituted C1-6 alkyl group,
wherein, in the above, the substituent in case being optionally substituted is a group selected from the following: a hydroxyl group, a carboxyl group, an amino group, a halogen atom, a C1-6 alkyl group optionally substituted with a hydroxyl group, a C2-6 alkenyl group, a C2-7 alkanoyl group, a C1-6 alkoxy group, a C1-6 alkoxycarbonyl group, a C6-10 aryl group].

5. A pyruvate dehydrogenase kinase inhibitor comprising the compound according to claim 3 or 4 or ester derivatives thereof, or pharmacologically acceptable salts thereof as an active ingredient.

6. The pyruvate dehydrogenase kinase inhibitor according to claim 1, 2 or 5, wherein the inhibitor is pyruvate dehydrogenase kinase inhibitor 4.

7. A pharmaceutical composition for treatment or prophylaxis of diseases or disorders that pyruvate dehydrogenase kinase relates to its development or aggravation comprising the pyruvate dehydrogenase kinase inhibitor according to claim 1, 2 or 5 as an active ingredient.

8. The pharmaceutical composition according to claim 7, wherein the diseases or disorders that pyruvate dehydrogenase kinase relates to its development or aggravation is influenza aggravation after infection.

9. The pharmaceutical composition according to claim 7, wherein the diseases or disorders that pyruvate dehydrogenase kinase relates to its development or aggravation is anorexia.

10. The pharmaceutical composition according to claim 7, wherein the diseases or disorders that pyruvate dehydrogenase kinase relates to its development or aggravation is mitochondrial diseases, or diseases or disorders accompanied by decreasing ATP production.

11. The pharmaceutical composition according to claim 7, wherein the diseases or disorders that pyruvate dehydrogenase kinase relates to its development or aggravation is diabetes.

12. The pharmaceutical composition according to claim 7, wherein the diseases or disorders that pyruvate dehydrogenase kinase relates to its development or aggravation is cancer.

13. A cosmetic composition comprising the pyruvate dehydrogenase kinase inhibitor according to claim 1, 2 or 5.
